# EUROPEAN PATENT APPLICATION

(11) **EP 4 778 530 A2**
(43) Date of publication of application: **22.07.2026**
(21) Application number: 26178661.0
(22) Date of filing: 13.10.2021
(51) Int. Cl.: A61K 31/7105

(54) **IN VIVO TARGETING OF CD4+-T CELLS FOR MRNA THERAPEUTICS**

(30) Priority: 13.10.2020 US 202063091010 P
(62) Divisional of application: 21880990.3
(71) Applicant: The Trustees of The University of Pennsylvania, Philadelphia, PA 19104 (US)
(72) Inventor: The designation of the inventor has not yet been filed
(74) Representative: Callaghan, Dayle Anne

(57) **Abstract**

The present invention relates to compositions comprising a delivery vehicle conjugated to a targeting domain, wherein the delivery vehicle comprises at least one agent, and wherein the targeting domain specifically binds to an CD4⁺ T cell antigen. The invention also relates to methods of treating or preventing diseases and disorders, including cancers, infectious diseases, and immunological disorders, using the described compositions.

## Description

### CROSS REFERENCE TO RELATED APPLICATIONS

This application claims priority to U.S. Provisional Application No. 63/091,010, filed October 13, 2020, which is incorporated by reference herein in its entirety.

### STATEMENT REGARDING FEDERALLY SPONSORED RESEARCH OR

### DEVELOPMENT

This invention was made with government support under A1045008 awarded by National Institutes of Health. The government has certain rights in the invention.

### BACKGROUND OF THE INVENTION

Modulation of immune cells through activation, inhibition, or modification to alter their properties has become a popular and high-demand class of therapy, called immunotherapeutics. Today's immunotherapeutics largely rely on biological protein-based agents, which are expensive and challenging to manufacture (Pranchevicius et al., 2013, Bioengineered, 4:305-312; Liu et al., 2018, Precision Clinical Medicine, 1:65-74), or require ex vivo modification of immune cells (Schultz et al., 2018, Immunotherapy in Translational Cancer Research: Maugeri et al., 2019, Nature Communications, 10:4333). Some examples include antibodies or cytokines for modulating immune cell function, monoclonal antibodies for redirecting immune function, genetic editing of T cells for preventing viral infections, and chimeric antigen receptor (CAR) T cell therapy (Schlake et al., 2019, Cellular and Molecular Life Sciences, 76:301-328: Wraith, 2017, Frontiers in immunology 8, 1668-1668; Whilding et al., 2015, Mol Oncol. 9:1994-2018).

One of the most relevant applications of cancer immunotherapeutics are CAR T cell therapies. Currently, CAR T cells are generated *ex vivo*, which is costly as it requires extended cell culture in GMP cell processing facilities. Additionally, it is not a treatment option for patients with highly malignant cancers, very low T cell counts, or settings requiring large scale use (Schmidts et al., 2018, Frontiers in immunology, 9:2593-2593: Zhao et al., 2019, Front. Immunol, 10:2250; Junghans, 2017, Cancer Gene Therapy, 24:89-99). There is a vital need for development of *in vivo* T cell-targeted mRNA delivery systems for robust and rapid generation of CAR T cells. mRNA-based CAR T cell therapeutics could also provide a safer platform by reducing the risk of CAR T cell-induced toxicities, because of their transient nature, as well as avoiding the risk of genomic integration, when so desired (Foster et al., 2019, Molecular Therapy, 27:747-756; Foster et al., 2019, Hum Gene Ther, 30:168-178: Kowalski et al., 2019, Molecular Therapy, 27:710-728; Pardi et al., 2018, Nat Rev Drug Discov, 17:261-279). Moreover, mRNA-based therapeutics could offer gene editing tools for treating viral infections and cancer or correcting genetic defects, such as knocking out the C-C chemokine receptor 5 (CCR5) gene for preventing HIV infection of T cells (Didigu et al., 2014, Blood, 123:61-69; Liu et al., 2017, Cell Biosci, 7:47), or knocking out the programmed cell death-1 (PD-1) gene for engineering superior tumor infiltrating lymphocytes (TILs) (Bailet et al., 2019, Nature Biotechnology, 37:1425-1434). One of the key obstacles in development of mRNA-based immunotherapeutics is efficient *in vivo* delivery.

Thus there is a need in the art for an efficient, safe, and immune cell-specific mRNA-delivery system for the introduction and widescale use of current and the generation of a new class of robust mRNA-based immunodierapeutics. The present invention satisfies this unmet need.

### BRIEF DESCRIPTION OF THE DRAWINGS

The following detailed description of embodiments of the invention will be better understood when read in conjunction with the appended drawings. It should be understood that the invention is not limited to the precise arrangements and instrumentalities of the embodiments shown in the drawings.

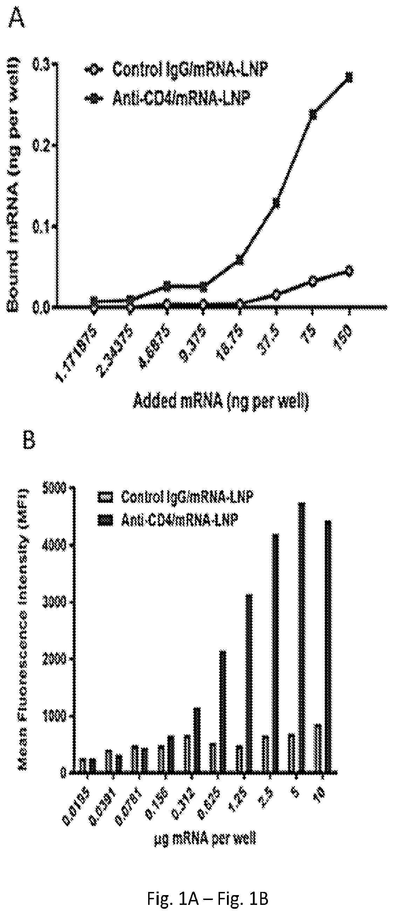

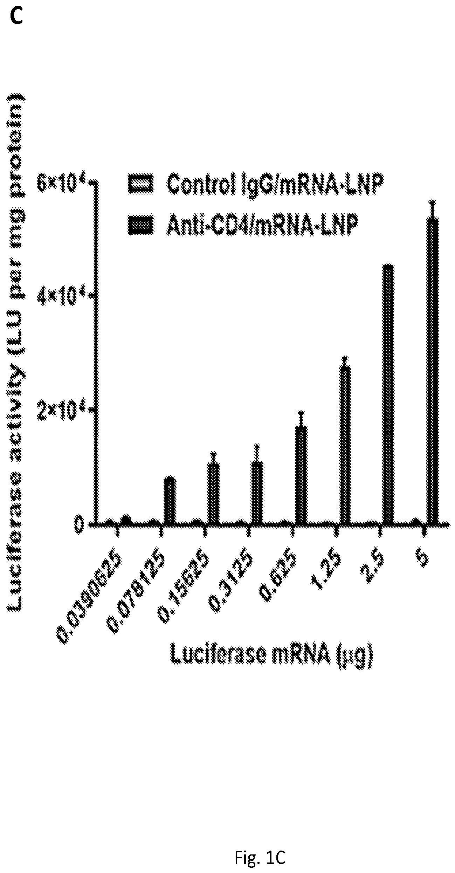

Figure 1A through Figure 1C depicts the binding and functional activity of CD4-targeted particles *in vitro.* Figure 1A depicts specific *in vitro* binding of anti-human CD4/¹²⁵I-labeled mRNA-LNP to human CD4⁺ T cells after a 1 hour incubation at room temperature (RT). Figure 1B depicts the binding of anti-CD4/mRNA-LNP and control IgG/mRNA-LNP to human CD4+ T cells, with increasing mRNA-LNP doses, and their corresponding mean fluorescence intensity (MFI). Figure 1C depicts the Luc activity measured in human CD4+ T cells treated with anti-human CD4/mRNA-LNP or control IgG/mRNA-LNP.

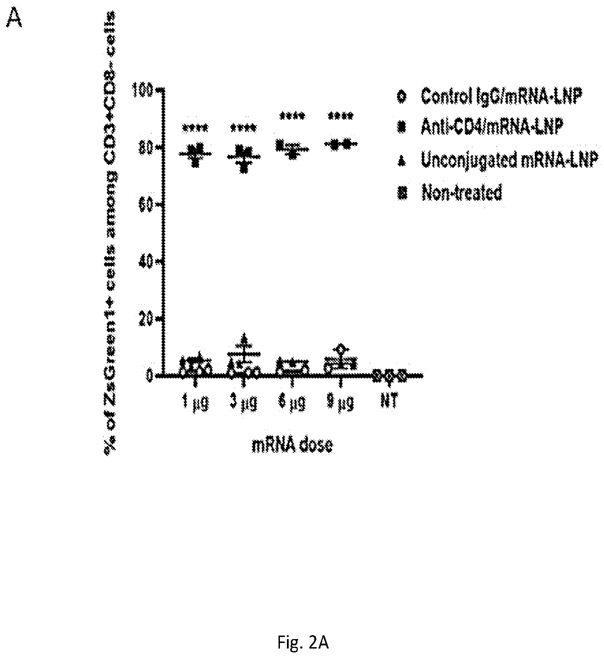

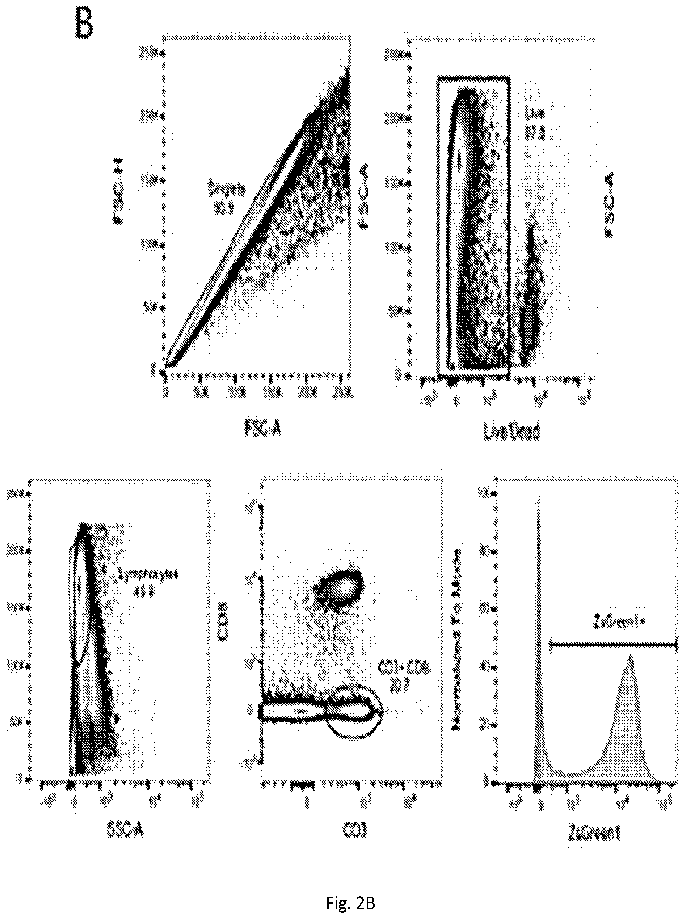

Figure 2A and Figure 2B depict the results of example experiments demonstrating Cre mRNA-mediated genetic recombination in vitro. Figure 2A depicts Cre mRNA-induced genetic recombination and consequent reporter gene expression presented as % of ZsGreen1⁺ cells among CD3⁺CD8⁻ cells. Splenocytes were harvested from Ai6 mice and incubated with Cre mRNA-LNP at doses of 1, 3, 6 or 9 µg per 2 million cells. %ZsGreen1⁺ cells upon anti-CD4/mRNA-LNP administration was compared to control IgG/mRNALNP and unconjugated mRNA-LNP administration (****P < 0.0001, two-way ANOVA with Bonferroni correction). Figure 2B depicts the gating strategy to identify ZsGreen1 positive cells among CD3⁺CD8⁻ cells.

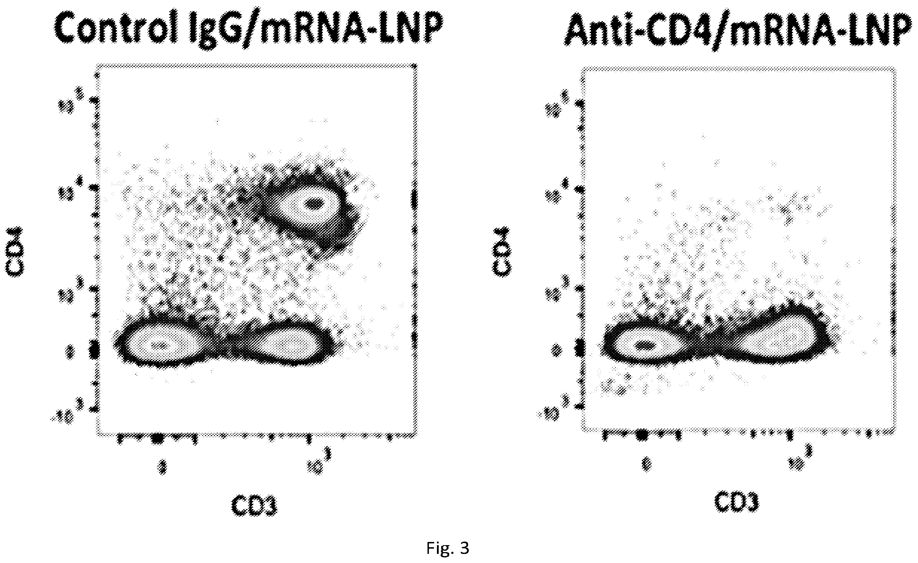

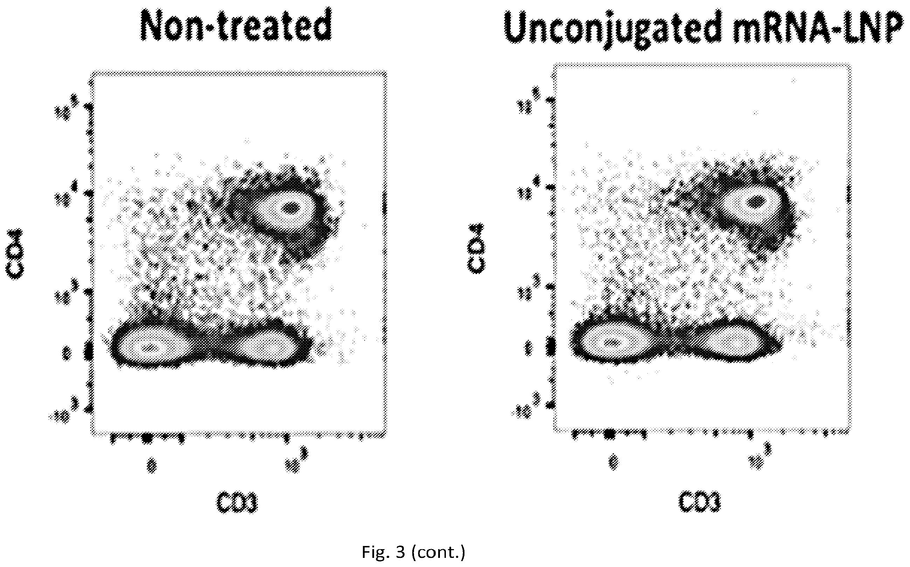

Figure 3 depicts the results of example experiments demonstrating the flow cytometric analysis of mRNA-LNP-treated CD3/CD4 cell populations. Splenocytes were harvested from Ai6 mice and incubated with Cre mRNA-LNP at a dose of 3 µg per 2 million cells per well in 6-well plates overnight. Transient disappearance of CD4 staining is observed upon administration of anti-CD4/mRNA-LNP, while non-targeted LNP treated cells are unaffected.

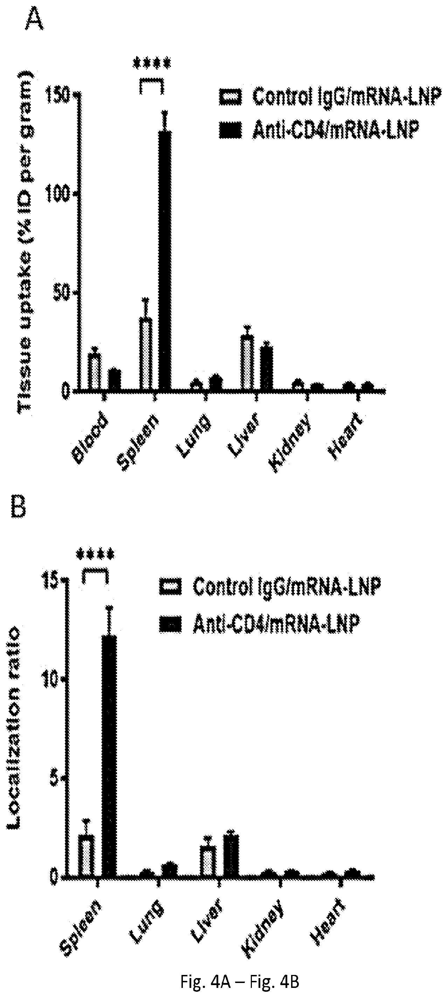

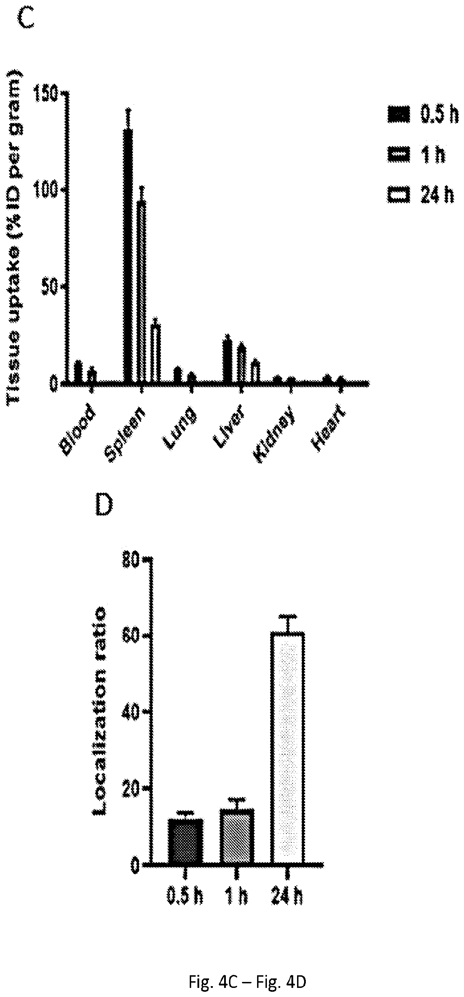

Figure 4A through Figure 4D depicts the results of example experiments demonstrating targeting of mRNA-LNP to CD4⁺ T cells *in vivo.* Figure 4A depicts the biodistribution of ¹²⁵I-labeled anti-CD4/ and control IgG/poly(C) mRNA-LNP in mice at 0.5 hours. Tissue uptake is indicated as mean ± SEM (****P < 0.0001). Figure 4B depicts the localization ratio, calculated as the ratio of %ID/g of a given organ to that in the blood of mice treated with either ¹²⁵I-labeled anti CD4/ or control IgG/mRNA-LNP at 30 minutes post-injection. Mean ± SEM is shown. In vivo mRNA-LNP-binding as quantitative measurement of the percentage of radiolabeled anti-CD4/mRNA-LNP in selected organs (Figure 4C) and localization ratios in spleens (Figure 4D), after intravenous injection of mRNA-LNP. Group size is 3 animals. Statistical analysis was performed by two-way ANOVA with Bonferroni correction (****P < 0.0001).

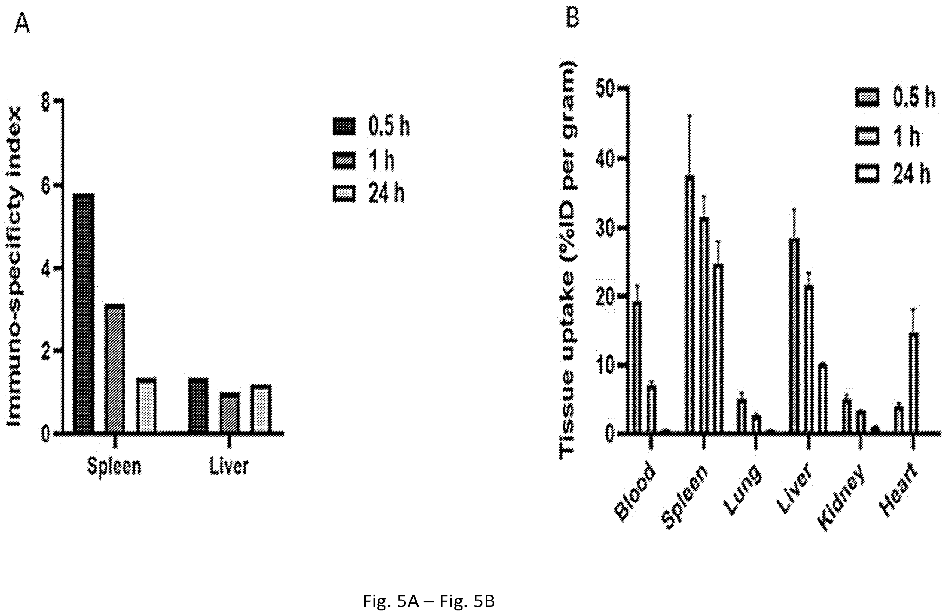

Figure 5A and Figure 5B depict the results of example experiments demonstrating targeting of Poly(C) RNA-LNP to CD4 in vivo. Figure 5A depicts the *in vivo* kinetics of anti-CD4/mRNA-LNP-binding in spleen and liver as immuno-specificity index. Immunospecificity index was calculated as the ratio of %ID/g of selected organs in mice treated with anti- CD4/ vs. control IgG/mRNA-LNP, normalized to blood levels. Figure 5B depicts the *in vivo* kinetics of control IgG/mRNA-LNP-binding as quantitative measurement of the percentage of radiolabeled mRNALNP in selected organs after intravenous injection of mRNA-LNP. Group size is 3 animals.

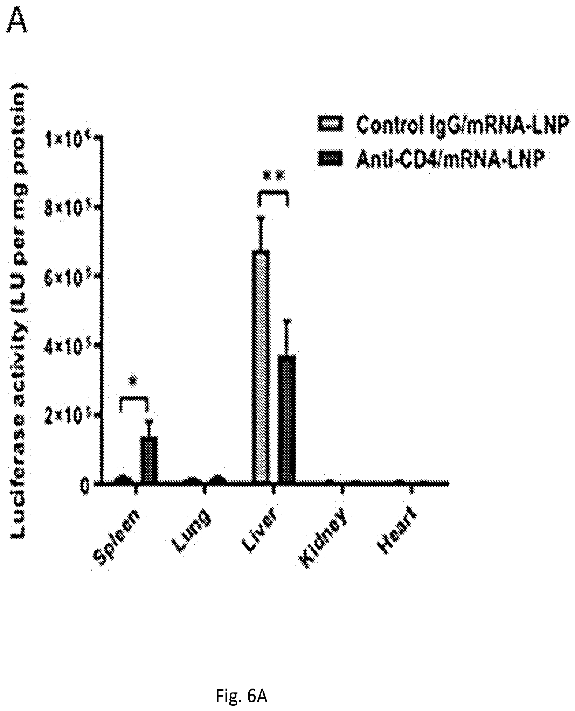

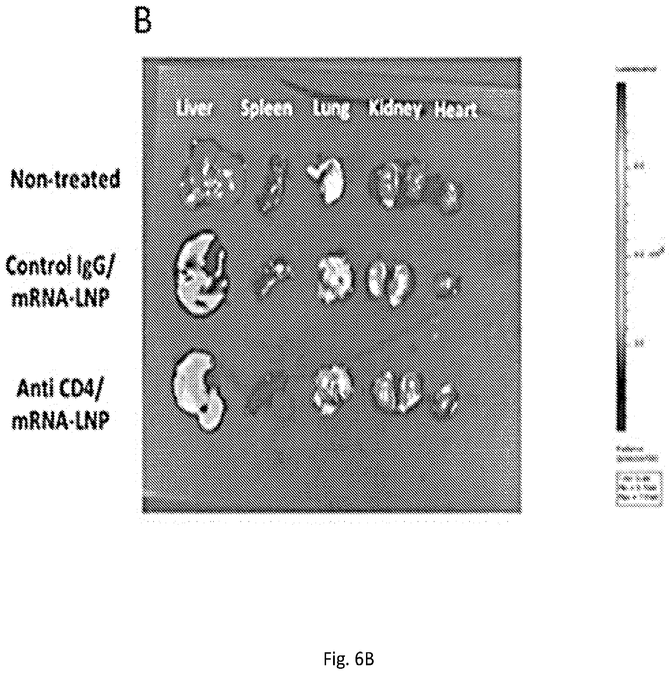

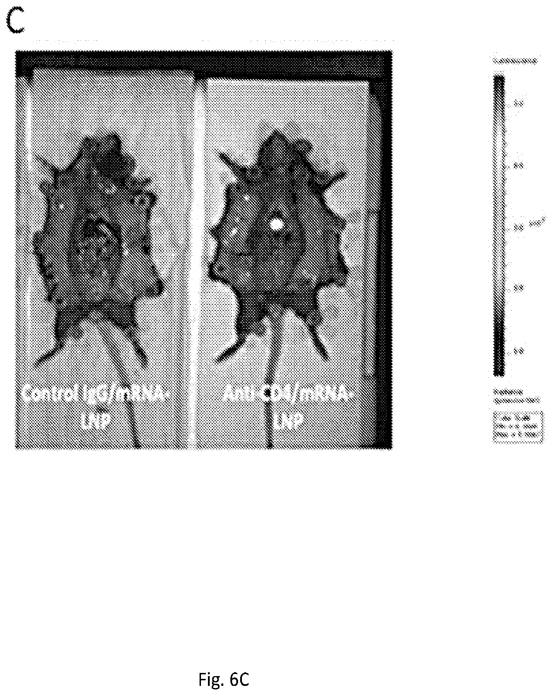

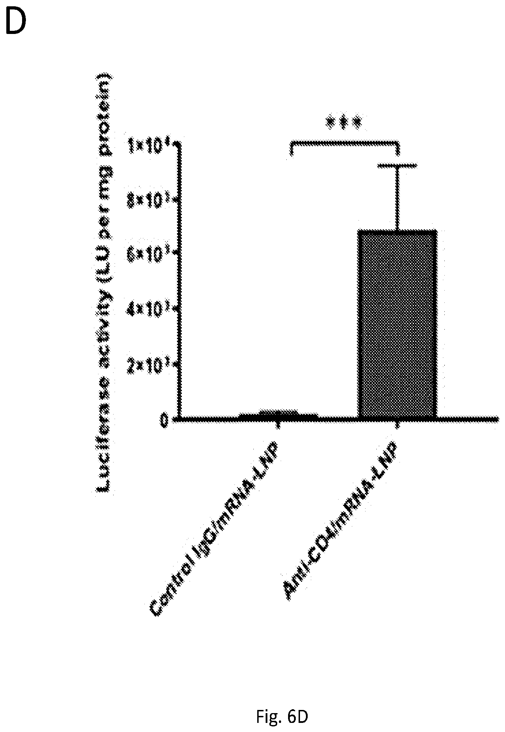

Figure 6A through Figure 6D depict the results of example experiments demonstrating biodistribution of targeted mRNA-LNP expression in vivo. Mice were IV injected with 8 µg of mRNA-LNP. Organ distribution of Luc mRNA expression 5 hours after administration of anti-CD4/ and control IgG/Luc mRNA-LNP was evaluated by measuring Luc activity in lysed tissues (Figure 6A) and by luminescence imaging (Figure 6B and Figure 6C). Figure 6A depicts a quantitative expression of Luc as light unit (LU)/mg protein. A representative sample set of dissected mouse organs (Figure 6B) and whole carcasses after organ removal (showing luminescing lymph nodes) (Figure 6C) were analyzed 5 minutes after the administration of D-luciferin. Figure 6D depicts a quantitative expression of Luc as LU/mg protein values in CD3+ cell preparation obtained from the spleens of mice injected with the mRNA-LNP. For Figure 6A and Figure 6D, the error bars indicate SEM. Group size is 3 animals. Statistical analysis was performed by two-way ANOVA with Bonferroni correction, (*P < 0.05, **P< 0.01, and ***P< 0.001).

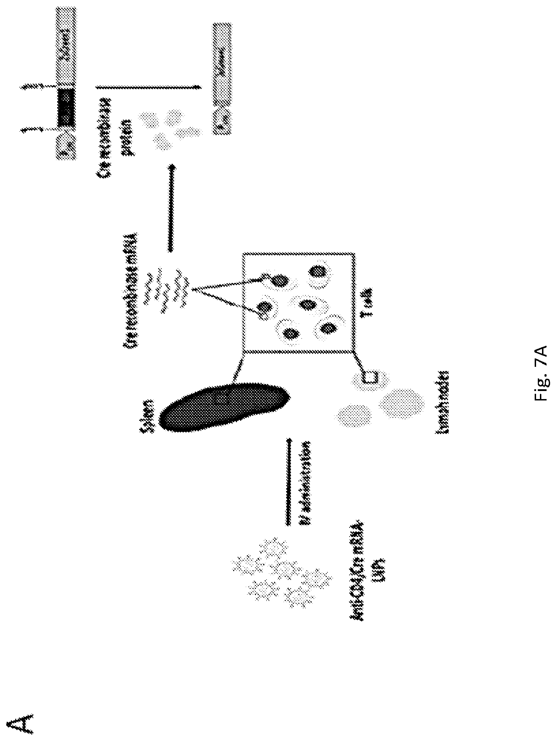

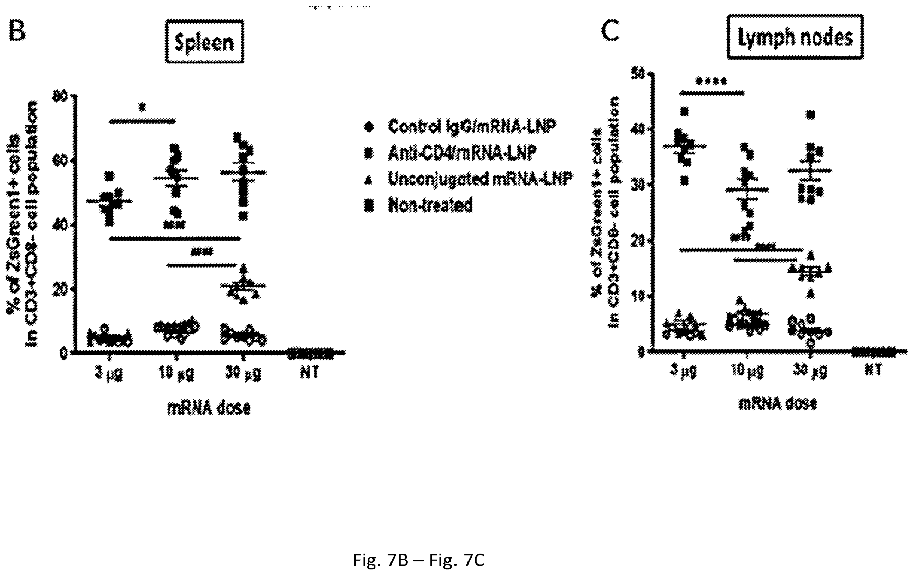

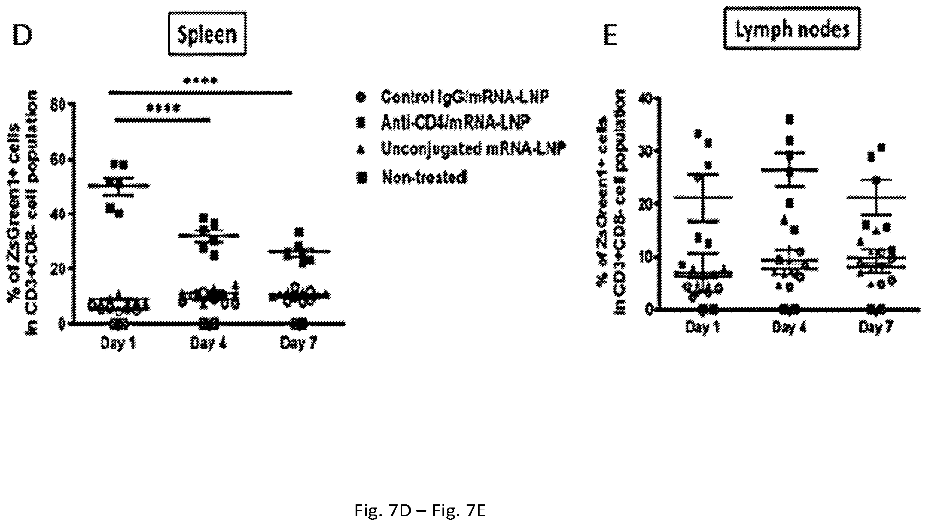

Figure 7A through Figure 7E depicts the results of example experiments demonstrating Cre-mediated genetic recombination upon in vivo administration of CD4- targeted Cre mRNA-LNP. Figure 7A depicts a schematic diagram depicting targeted delivery of anti- CD4/mRNA-LNP for selective genetic recombination in CD4⁺ T cells, and the principle of the Ai6 reporter allele: Cre-mediated excision of a loxP-flanked STOP cassette allows robust expression of ZsGreen1, a fluorescent protein. Ai6 mice received Cre mRNA-LNP at doses of 3, 10, and 30 µg via IV administration. Spleens and lymph nodes were harvested at 24 hours post treatment and % of ZsGreen1⁺ cells in the CD3+CD8- cell population were determined in splenic (Figure 7B) and lymph node (Figure 7C) single cell suspensions using flow cytometry. Changes in the number of ZsGreen1-expressing CD4⁺ T cells in spleens (Figure 7D) and lymph nodes (Figure 7E) over time were monitored after IV injection of 10 µg of mRNA-LNP. Group size is 8 or 9 (Figure 7B and Figure 7C) or 6 (Figure 7D and Figure 7E) animals in a total of three independent experiments. Each symbol represents one animal and horizontal lines show the mean with SEM. Statistical analysis was performed by two-way ANOVA with Bonferroni correction. %ZsGreen1⁺ cells after injection of different doses of anti-CD4/mRNA-LNP [*P < 0.05, ****P< 0.0001] and unconjugated mRNA-LNP [####P< 0.0001] were compared.

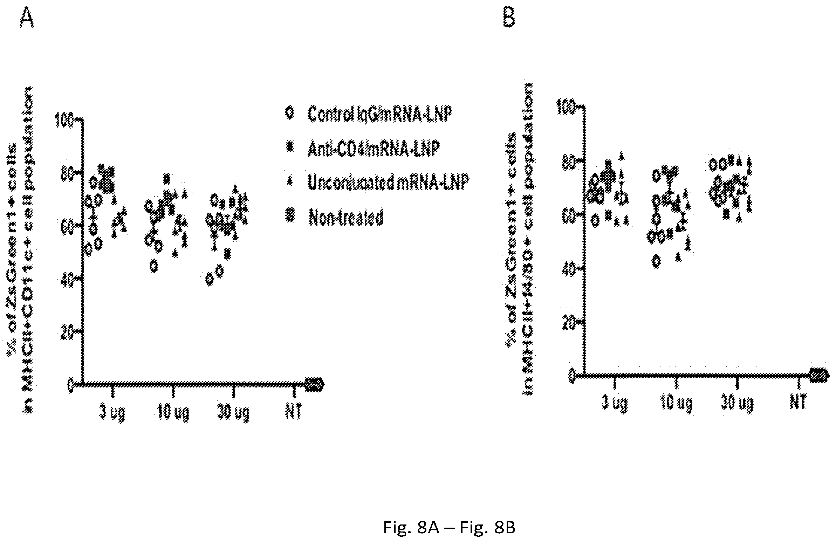

Figure 8A and Figure 8B depict the results of example experiments demonstrating Cre-mediated genetic recombination in non-T cells upon in vivo administration of Cre mRNA-LNP. Ai6 mice received Cre mRNA-LNP at doses of 3, 10, and 30 µg via IV administration. At 24 hours post treatment, %ZsGreen1⁺ cells in the dendritic cells (MHCII⁺CD11c⁺) (Figure 8A) and macrophages (MHCII⁺F4/80⁺) (Figure 8B) of spleen were determined in splenic single cell suspensions using flow cytometry. Group size is 8-9 animals in a total of three independent experiments. Each symbol represents one animal and horizontal lines show the mean with SEM.

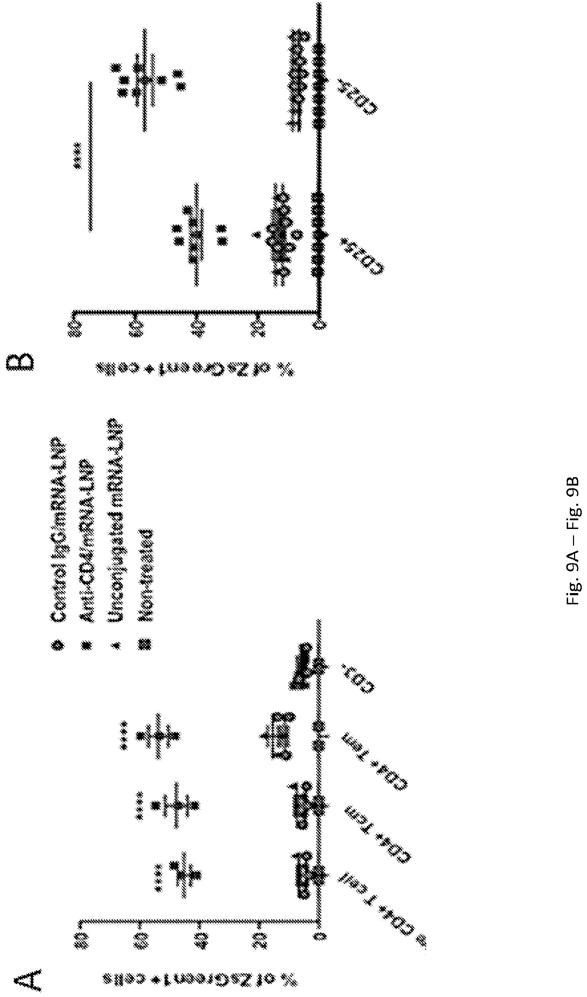

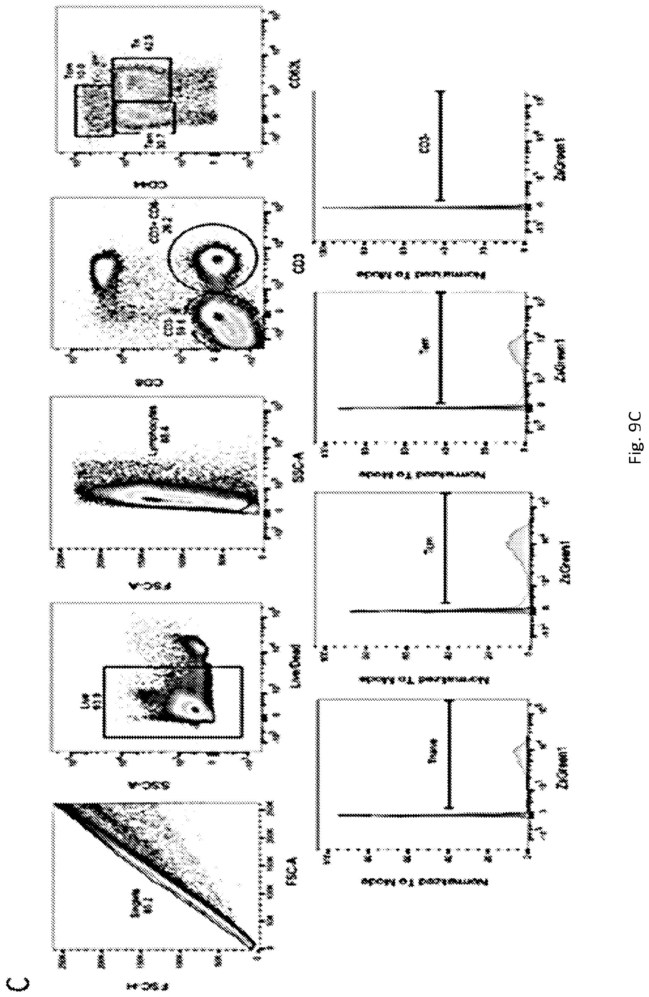

Figure 9A through Figure 9C depicts the results of example experiments demonstrating *in vivo* uptake of Cre mRNA-LNP by different T cell subtypes. Spleens were harvested at 24 hours post-treatment with 10 µg of Cre mRNA-LNP, and % of ZsGreen1⁺ cells in CD4⁺ T cell subpopulations (Figure 9A) and vs. CD25 marker (Figure 9B) were determined using flow cytometry. Naive CD4⁺ T cells are considered as CD44⁻CD62L⁻, central memory T cells as CD44⁺CD62L⁺, and effector memory T cells as CD44⁺CD62L⁻. Group size is 3-11 animals. Each symbol represents one animal and horizontal lines show the mean with SEM. Statistical analysis was performed by two-way ANOVA with Bonferroni correction comparing T cell subtypes. Figure 9C depicts the gating strategy to identify ZsGreen1 positive cells among different CD4⁺ T cell subtypes.

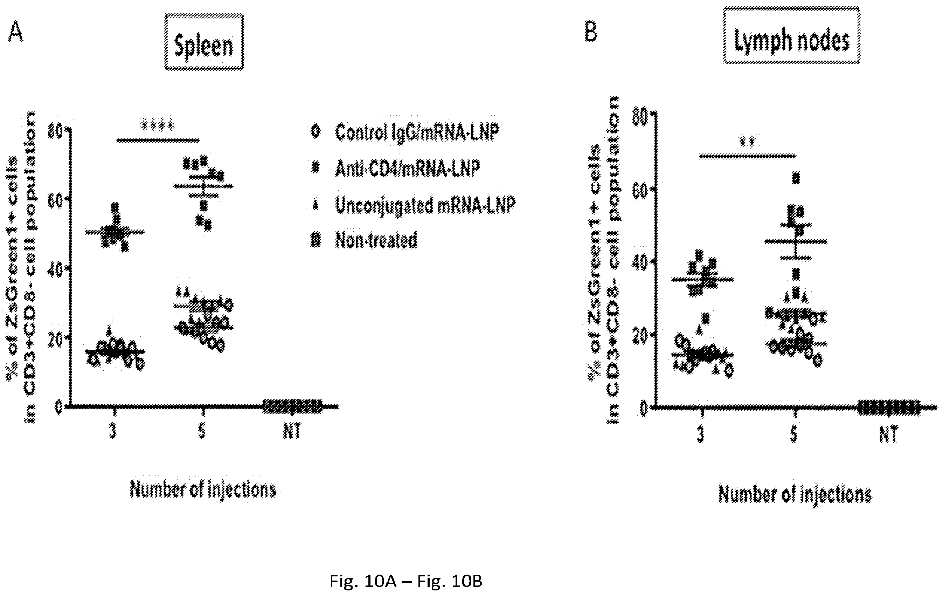

Figure 10A and Figure 10B depict the results of example experiments demonstrating mRNA-LNP targeting efficiency using multiple administrations. Ai6 mice received 10 µg (0.4 mg/kg) of anti-CD4/, control IgG/ or unconjugated Cre mRNA-LNP via IV administration as daily injections for 3 or 5 days. Spleens and lymph nodes were harvested after three or five sequential injections, and the % of ZsGreen1⁺ cells in the CD3⁺CD8⁻ cell population was determined in splenic (Figure 10A) and lymph node (Figure 10B) single cell suspensions using flow cytometry. Group size is 9 animals. Each symbol represents one animal and horizontal lines show the mean. Error bars indicate SEM. Statistical analysis was performed by two-way ANOVA with Bonferroni correction. %ZsGreen1⁺ cells after different number of injections of anti-CD4/mRNA-LNP [**P < 0.01, ****P< 0.0001] were compared.

### DETAILED DESCRIPTION

The present invention relates to compositions comprising a delivery vehicle conjugated to a CD4+ T cell targeting domain, wherein the delivery vehicle comprises at least one agent. In one embodiment, the targeting domain specifically binds to CD4.

In certain embodiments, the delivery vehicle is a lipid nanoparticle comprising a PEG-lipid conjugated to the targeting domain. In some embodiments, the at least one agent is a nucleic acid. In some embodiments, the at least one agent is an mRNA molecule. In one embodiment, the mRNA molecule is a nucleoside-modified mRNA. The present invention also relates to methods of treating a disease or disorder using the compositions described herein.

### Definitions

Unless defined otherwise, all technical and scientific terms used herein have the same meaning as commonly understood by one of ordinary skill in the art to which this invention belongs.

As used herein, each of the following terms has the meaning associated with it in this section.

The articles "a" and "an" are used herein to refer to one or to more than one (*i.e*., to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, or ±0.1% from the specified value, as such variations are appropriate to perform the disclosed methods.

The term "antibody," as used herein, refers to an immunoglobulin molecule, which specifically binds with an antigen or epitope. Antibodies can be intact immunoglobulins derived from natural sources or from recombinant sources and can be immunoreactive portions of intact immunoglobulins. Antibodies are typically tetramers of immunoglobulin molecules. The antibodies in the present invention may exist in a variety of forms including, for example, polyclonal antibodies, monoclonal antibodies, Fv, Fab and F(ab)₂, as well as single chain antibodies and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883: Bird et al., 1988, Science 242:423-426).

The term "antibody fragment" refers to a portion of an intact antibody and refers to the antigenic determining variable regions of an intact antibody. Examples of antibody fragments include, but are not limited to, Fab, Fab', F(ab')2, and Fv fragments, linear antibodies, scFv antibodies, and multispecific antibodies formed from antibody fragments.

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations. k and 1 light chains refer to the two major antibody light chain isotypes.

By the term "synthetic antibody" as used herein, is meant an antibody, which is generated using recombinant DNA technology, such as, for example, an antibody expressed by a bacteriophage. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art. The term should also be construed to mean an antibody, which has been generated by the synthesis of an RNA molecule encoding the antibody. The RNA molecule expresses an antibody protein, or an amino acid sequence specifying the antibody, wherein the RNA has been obtained by transcribing DNA (synthetic or cloned) or other technology, which is available and well known in the art.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

An "effective amount" as used herein, means an amount which provides a therapeutic or prophylactic benefit.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

"Expression vector" refers to a vector comprising a recombinant polynucleotide comprising expression control sequences operatively linked to a nucleotide sequence to be expressed. An expression vector comprises sufficient cis-acting elements for expression: other elements for expression can be supplied by the host cell or in an in vitro expression system. Expression vectors include all those known in the art, such as cosmids, plasmids (e.g., naked or contained in liposomes) RNA, and viruses (e.g., lentiviruses, retroviruses, adenoviruses, and adeno-associated viruses) that incorporate the recombinant polynucleotide.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared X 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleosides (nucleobase bound to ribose or deoxyribose sugar via N-glycosidic linkage) are used. "A" refers to adenosine, "C" refers to cytidine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

By the term "modulating," as used herein, is meant mediating a detectable increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.

Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns. In addition, the nucleotide sequence may contain modified nucleosides that are capable of being translation by translational machinery in a cell. For example, in some aspects the nucleotide sequence comprises an mRNA where some or all of the uridines have been replaced with pseudouridine, 1-methyl psuedouridine, or another modified nucleoside.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA or RNA sequences are contiguous and, where necessary to join two protein coding regions, in the same reading frame.

The terms "patient," "subject," "individual," and the like are used interchangeably herein, and refer to any animal, or cells thereof whether in vitro or in situ, amenable to the methods described herein. In certain non-limiting embodiments, the patient, subject or individual is a human.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means.

In certain instances, the polynucleotide or nucleic acid of the invention is a "nucleoside-modified nucleic acid," which refers to a nucleic acid comprising at least one modified nucleoside. A "modified nucleoside" refers to a nucleoside with a modification. For example, over one hundred different nucleoside modifications have been identified in RNA (Rozenski, et al., 1999, The RNA Modification Database: 1999 update. Nucl Acids Res 27: 196-197).

In certain embodiments, "pseudouridine" refers, in another embodiment, to m¹acp³Y (1-methyl-3-(3-amino-3-carboxypropyl) pseudouridine. In another embodiment, the term refers to m¹Y (1-methylpseudouridine). In another embodiment, the term refers to Ym (2'-O-methylpseudouridine. In another embodiment, the term refers to m⁵D (5-methyldihydrouridine). In another embodiment, the term refers to m³Y (3-methylpseudouridine). In another embodiment, the term refers to a pseudouridine moiety that is not further modified. In another embodiment, the term refers to a monophosphate, diphosphate, or triphosphate of any of the above pseudouridines. In another embodiment, the term refers to any other pseudouridine known in the art. Each possibility represents a separate embodiment of the present invention.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein is defined as a DNA sequence recognized by the synthetic machinery of the cell, or introduced synthetic machinery, required to initiate the specific transcription of a polynucleotide sequence. For example, the promoter that is recognized by bacteriophage RNA polymerase and is used to generate the mRNA by in vitro transcription.

By the term "specifically binds," as used herein with respect to an affinity ligand, in particular, an antibody, is meant an antibody which recognizes a specific antigen, but does not substantially recognize or bind other molecules in a sample. For example, an antibody that specifically binds to an antigen from one species may also bind to that antigen from one or more other species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the terms "specific binding" or "specifically binding," can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

The term "therapeutic" as used herein means a treatment and/or prophylaxis. A therapeutic effect is obtained by suppression, diminution, remission, or eradication of at least one sign or symptom of a disease or disorder.

The term "therapeutically effective amount" refers to the amount of the subject compound that will elicit the biological or medical response of a tissue, system, or subject that is being sought by the researcher, veterinarian, medical doctor or other clinician. The term "therapeutically effective amount" includes that amount of a compound that, when administered, is sufficient to prevent development of, or alleviate to some extent, one or more of the signs or symptoms of the disorder or disease being treated. The therapeutically effective amount will vary depending on the compound, the disease and its severity and the age, weight, etc., of the subject to be treated.

To "treat" a disease as the term is used herein, means to reduce the frequency or severity of at least one sign or symptom of a disease or disorder experienced by a subject.

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The phrase "under transcriptional control" or "operatively linked" as used herein means that the promoter is in the correct location and orientation in relation to a polynucleotide to control the initiation of transcription by RNA polymerase and expression of the polynucleotide.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

"Alkyl" refers to a straight or branched hydrocarbon chain radical consisting solely of carbon and hydrogen atoms, which is saturated or unsaturated (i.e., contains one or more double and/or triple bonds), having from one to twenty-four carbon atoms (C₁-C₂₄ alkyl), one to twelve carbon atoms (C₁-C₁₂ alkyl), one to eight carbon atoms (C₁-C₈ alkyl) or one to six carbon atoms (C₁-C₆ alkyl) and which is attached to the rest of the molecule by a single bond, e.g., methyl, ethyl, n propyl, 1-methylethyl (iso propyl), n butyl, n pentyl, 1,1 dimethylethyl (t butyl), 3 methylhexyl, 2 methylhexyl, ethenyl, prop 1 enyl, but-1-enyl, pent-1-enyl, penta-1,4-dienyl, ethynyl, propynyl, butynyl, pentynyl, hexynyl, and the like. Unless specifically stated otherwise, an alkyl group is optionally substituted.

"Alkylene" or "alkylene chain" refers to a straight or branched divalent hydrocarbon chain linking the rest of the molecule to a radical group, consisting solely of carbon and hydrogen, which is saturated or unsaturated (*i.e*., contains one or more double (alkenylene) and/or triple bonds (alkynylene)), and having, for example, from one to twenty-four carbon atoms (C₁-C₂₄ alkylene), one to fifteen carbon atoms (C₁-C₁₅ alkylene),one to twelve carbon atoms (C₁-C₁₂ alkylene), one to eight carbon atoms (C₁-C₈ alkylene), one to six carbon atoms (C₁-C₆ alkylene), two to four carbon atoms (C₂-C₄ alkylene), one to two carbon atoms (C₁-C₂ alkylene), *e.g*., methylene, ethylene, propylene, *n*-butylene, ethenylene, propenylene, *n*-butenylene, propynylene, *n*-butynylene, and the like. The alkylene chain is attached to the rest of the molecule through a single or double bond and to the radical group through a single or double bond. The points of attachment of the alkylene chain to the rest of the molecule and to the radical group can be through one carbon or any two carbons within the chain. Unless stated otherwise specifically in the specification, an alkylene chain may be optionally substituted.

"Cycloalkyl" or "carbocyclic ring" refers to a stable non aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, 7,7 dimethyl bicyclo[2.2.1]heptanyl, and the like. Unless specifically stated otherwise, a cycloalkyl group is optionally substituted.

"Cycloalkylene" is a divalent cycloalkyl group. Unless otherwise stated specifically in the specification, a cycloalkylene group may be optionally substituted.

"Heterocyclyl" or "heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems: and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless specifically stated otherwise, a heterocyclyl group may be optionally substituted.

The term "substituted" used herein means any of the above groups (e.g., alkyl, cycloalkyl or heterocyclyl) wherein at least one hydrogen atom is replaced by a bond to a non-hydrogen atoms such as, but not limited to: a halogen atom such as F, Cl, Br, and I; oxo groups (=O); hydroxyl groups (-OH); alkoxy groups (-OR^{a}, where R³ is C₁-C₁₂ alkyl or cycloalkyl); carboxyl groups (-OC(=O)R^{a} or -C(=O)OR^{a}, where R³ is H, C₁-C₁₂ alkyl or cycloalkyl); amine groups (-NR^{a}R^{b}, where R^{a} and R^{b} are each independently H, C₁-C₁₂ alkyl or cycloalkyl); C₁-C₁₂ alkyl groups; and cycloalkyl groups. In some embodiments the substituent is a C₁-C₁₂ alkyl group. In other embodiments, the substituent is a cycloalkyl group. In other embodiments, the substituent is a halo group, such as fluoro. In other embodiments, the substituent is a oxo group. In other embodiments, the substituent is a hydroxyl group. In other embodiments, the substituent is an alkoxy group. In other embodiments, the substituent is a carboxyl group. In other embodiments, the substituent is an amine group.

"Optional" or "optionally" (e.g., optionally substituted) means that the subsequently described event of circumstances may or may not occur, and that the description includes instances where said event or circumstance occurs and instances in which it does not. For example, "optionally substituted alkyl" means that the alkyl radical may or may not be substituted and that the description includes both substituted alkyl radicals and alkyl radicals having no substitution.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present invention relates in part to compositions and methods for targeted delivery of a delivery vehicle. In one aspect, the present invention relates to composition comprising a delivery vehicle conjugated to a targeting domain. In one embodiment, the delivery vehicle comprises at least one agent. In one embodiment, the delivery vehicle comprises an RNA molecule, including but not limited to mRNA, nucleoside-modified RNA, siRNA, miRNA, shRNA, or antisense RNA. In one embodiment, the delivery vehicle comprises a therapeutic agent. In one embodiment, the therapeutic agent is a nucleoside-modified RNA.

In various embodiments, the targeting domain binds to a cell surface molecule of a T cell antigen. In one embodiment, the T cell antigen is a surface antigen of a CD4+ T cell. In one embodiment, the T cell surface antigen is CD4.

In one embodiment, the composition comprises a delivery vehicle conjugated to a targeting domain that binds CD4 or a surface antigen of a CD4+ T cell, thereby directing the composition to CD4+ T cells.

In one embodiment, the delivery vehicle comprises or encapsulates an agent for modulation of CD4+ T cells. In some embodiments the agent is nucleic acid molecule. In some embodiments the nucleic acid molecule is a nucleoside modified mRNA.

In one embodiment, the delivery vehicle comprises or encapsulates a therapeutic agent for modulation of CD4+ T cells. In some embodiments the therapeutic agent is a nucleoside-modified mRNA. In some embodiments the therapeutic agent is an mRNA-based immunotherapeutic.

The present invention also relates in part to methods of treating diseases or disorders in subjects in need thereof, the method comprising the administration of a composition including a delivery vehicle comprising an agent conjugated to a targeting domain that binds CD4 or a surface antigen of a CD4+ T cell.

Exemplary diseases and disorders that can be treated using the CD4 T cell targeted therapeutic compositions of the invention include, but are not limited to, cancers, infectious diseases, and immunological disorders.

As a non-limiting example, in one embodiment, the CD4+ T cell-targeted delivery vehicle of the invention comprises or encapsulates a nucleoside-modified 1086C Env mRNA, encoding the clade C transmitted/founder human immunodeficiency virus (HIV)-1 envelope (Env) 1086C, for the treatment or prevention of HIV infection or a disease or disorder associated therewith.

### Delivery vehicle cargo

In various embodiments, the delivery vehicle comprises a cargo of one or more nucleic acid molecules (e.g., mRNA, expression vector, or genome editing vector) which genetically modify the immune cell. After cellular uptake of the delivery vehicle by the target immune cell (e.g., by endocytosis), the cargo nucleic acid becomes released from the endosome. Once released, the cargo nucleic acid modifies the target immune cell of the subject to express one or more surface moieties (e.g., a cell receptor).

In one embodiment, the delivery vehicle comprises at least one agent. In some embodiments, the agent is a therapeutic agent, an imaging agent, diagnostic agent, a contrast agent, a labeling agent, a detection agent, or a disinfectant. The agent may also include substances with biological activities which are not typically considered to be active ingredients, such as fragrances, sweeteners, flavorings and flavor enhancer agents, pH adjusting agents, effervescent agents, emollients, bulking agents, soluble organic salts, permeabilizing agents, anti-oxidants, colorants or coloring agents, and the like.

In one embodiment, the delivery vehicle comprises at least one therapeutic agent. The present invention is not limited to any particular therapeutic agent, but rather encompasses any suitable therapeutic agent that can be included within the delivery vehicle. Exemplary therapeutic agents include, but are not limited to, anti-viral agents, anti-bacterial agents, anti-oxidant agents, thrombolytic agents, chemotherapeutic agents, anti-inflammatory agents, immunogenic agents, antiseptics, anesthetics, analgesics, pharmaceutical agents, small molecules, peptides, nucleic acids, and the like. In one embodiment, the agent is an mRNA molecule (e.g., a nucleoside modified mRNA molecule) as described elsewhere herein.

### Nucleic acid agents

In one aspect, the present disclosure provides delivery vehicles comprising a nucleic acid cargo (e.g., DNA or RNA), including, but not limited to, an mRNA, expression vector, genome editing vector, siRNA, shRNA, an miRNA for use in inhibiting, inactivating, and/or destroying activated fibroblasts. In various embodiments, the nucleic acid cargo may be a nucleoside modified nucleic acid molecule (e.g., a nucleoside modified mRNA molecule). In various embodiments, the agent is an isolated nucleic acid. In some embodiments, the isolated nucleic acid molecule is a cDNA, mRNA, siRNA, shRNA or miRNA molecule. In some embodiments, the isolated nucleic acid molecule is a nucleoside modified RNA molecule. In some embodiments, the nucleoside modified RNA molecule is an siRNA, miRNA, shRNA, or an antisense molecule.

In various embodiments, that delivery vehicles comprise a cargo of one or more nucleic acid molecules (e.g., mRNA, expression vector, or genome editing vector, DNA, or RNA) which genetically modify the immune cell. After cellular uptake of the delivery vehicle by the target immune cell (e.g., by endocytosis), the cargo nucleic acid becomes released from the endosome. Once released, the cargo nucleic acid modifies the target immune cell of the subject.

In one embodiment, the nucleic acid comprises a promoter/regulatory sequence such that the nucleic acid is capable of directing expression of the nucleic acid. Thus, the invention encompasses expression vectors and methods for the introduction of exogenous nucleic acid into cells with concomitant expression of the exogenous nucleic acid in the cells such as those described, for example, in Sambrook et al. (2012, Molecular Cloning: A Laboratory Manual, Cold Spring Harbor Laboratory, New York), and in Ausubel et al. (1997, Current Protocols in Molecular Biology, John Wiley & Sons, New York) and as described elsewhere herein.

### Nucleoside-modified RNA agents

In one embodiment, the composition of the present invention comprises a nucleoside-modified nucleic acid (e.g., a nucleoside-modified mRNA molecule). In one embodiment, the composition of the invention comprises a nucleoside-modified RNA encoding a protein, such as a therapeutic protein.

For example, in one embodiment, the composition comprises a nucleoside-modified RNA. In one embodiment, the composition comprises a nucleoside-modified mRNA. Nucleoside-modified mRNA have particular advantages over non-modified mRNA, including for example, increased stability, low or absent innate immunogenicity, and enhanced translation. Nucleoside-modified mRNA useful in the present invention is further described in U.S. Patent Nos. 8,278,036,8,691,966, and 8,835,108, each of which is incorporated by reference herein in its entirety.

In certain embodiments, nucleoside-modified mRNA does not activate any pathophysiologic pathways, translates very efficiently and almost immediately following delivery, and serve as templates for continuous protein production in vivo lasting for several days (Kariko et al., 2008, Mol Ther 16:1833-1840; Kariko et al., 2012, Mol Ther 20:948-953). The amount of mRNA required to exert a physiological effect is small and that makes it applicable for human therapy.

In certain instances, expressing a protein by delivering the encoding mRNA has many benefits over methods that use protein, plasmid DNA or viral vectors. During mRNA transfection, the coding sequence of the desired protein is the only substance delivered to cells, thus avoiding all the side effects associated with plasmid backbones, viral genes, and viral proteins. More importantly, unlike DNA- and viral-based vectors, the mRNA does not carry the risk of being incorporated into the genome and protein production starts immediately after mRNA delivery. For example, high levels of circulating proteins have been measured within 15 to 30 minutes of in vivo injection of the encoding mRNA. In certain embodiments, using mRNA rather than the protein also has many advantages. Half-lives of proteins in the circulation are often short, thus protein treatment would need frequent dosing, while mRNA provides a template for continuous protein production for several days. Purification of proteins is problematic and they can contain aggregates and other impurities that cause adverse effects (Kromminga and Schellekens, 2005, Ann NY Acad Sci 1050:257-265).

In certain embodiments, the nucleoside-modified RNA comprises the naturally occurring modified-nucleoside pseudouridine. In certain embodiments, inclusion of pseudouridine makes the mRNA more stable, non-immunogenic, and highly translatable (Kariko et al., 2008, Mol Ther 16:1833-1840: Anderson et al., 2010, Nucleic Acids Res 38:5884-5892; Anderson et al., 2011, Nucleic Acids Research 39:9329-9338: Kariko et al., 2011, Nucleic Acids Research 39:e142; Kariko et al., 2012, Mol Ther 20:948-953: Kariko et al., 2005, Immunity 23:165-175).

It has been demonstrated that the presence of modified nucleosides, including pseudouridines in RNA suppress their innate immunogenicity (Kariko et al., 2005, Immunity 23:165-175). Further, protein-encoding, in vitro-transcribed RNA containing pseudouridine can be translated more efficiently than RNA containing no or other modified nucleosides (Kariko et al., 2008, Mol Ther 16:1833-1840). Subsequently, it is shown that the presence of pseudouridine improves the stability of RNA (Anderson et al., 2011, Nucleic Acids Research 39:9329-9338) and abates both activation of PKR and inhibition of translation (Anderson et al., 2010, Nucleic Acids Res 38:5884-5892).

Similar effects as described for pseudouridine have also been observed for RNA containing 1-methyl-pseudouridine.

In some embodiments, the nucleoside-modified nucleic acid molecule is a purified nucleoside-modified nucleic acid molecule. For example, in some embodiments, the composition is purified to remove double-stranded contaminants. In some instances, a preparative HPLC purification procedure is used to obtain pseudouridine-containing RNA that has superior translational potential and no innate immunogenicity (Kariko et al., 2011, Nucleic Acids Research 39:e142). Administering HPLC-purified, pseudourine-containing RNA coding for erythropoietin into mice and macaques resulted in a significant increase of serum EPO levels (Kariko et al., 2012, Mol Ther 20:948-953), thus confirming that pseudouridine-containing mRNA is suitable for in vivo protein therapy. In some embodiments, the nucleoside-modified nucleic acid molecule is purified using non-HPLC methods. In some instances, the nucleoside-modified nucleic acid molecule is purified using chromatography methods, including but not limited to HPLC and fast protein liquid chromatography (FPLC). An exemplary FPLC-based purification procedure is described in Weissman et al., 2013, Methods Mol Biol, 969: 43-54. Exemplary purification procedures are also described in U.S. Patent Application Publication No. US2016/0032316, which is hereby incorporated by reference in its entirety.

The present invention encompasses RNA, oligoribonucleotide, and polyribonucleotide molecules comprising pseudouridine or a modified nucleoside. In certain embodiments, the composition comprises an isolated nucleic acid, wherein the nucleic acid comprises a pseudouridine or a modified nucleoside. In certain embodiments, the composition comprises a vector, comprising an isolated nucleic acid, wherein the nucleic acid comprises a pseudouridine or a modified nucleoside.

In one embodiment, the nucleoside-modified RNA of the invention is IVT RNA, as described elsewhere herein. For example, in certain embodiments, the nucleoside-modified RNA is synthesized by T7 phage RNA polymerase. In another embodiment, the nucleoside-modified mRNA is synthesized by SP6 phage RNA polymerase. In another embodiment, the nucleoside-modified RNA is synthesized by T3 phage RNA polymerase.

In one embodiment, the modified nucleoside is m¹acp³Ψ (1-methyl-3-(3-amino-3-carboxypropyl) pseudouridine. In another embodiment, the modified nucleoside is m¹Ψ (1-methylpseudouridine). In another embodiment, the modified nucleoside is Ψm (2'-O-methylpseudouridine. In another embodiment, the modified nucleoside is m⁵D (5-methyldihydrouridine). In another embodiment, the modified nucleoside is m³Ψ (3-methylpseudouridine). In another embodiment, the modified nucleoside is a pseudouridine moiety that is not further modified. In another embodiment, the modified nucleoside is a monophosphate, diphosphate, or triphosphate of any of the above pseudouridines. In another embodiment, the modified nucleoside is any other pseudouridine-like nucleoside known in the art.

In another embodiment, the nucleoside that is modified in the nucleoside-modified RNA the present invention is uridine (U). In another embodiment, the modified nucleoside is cytidine (C). In another embodiment, the modified nucleoside is adenosine (A). In another embodiment, the modified nucleoside is guanosine (G).

In another embodiment, the modified nucleoside of the present invention is m⁵C (5-methylcytidine). In another embodiment, the modified nucleoside is m⁵U (5-methyluridine). In another embodiment, the modified nucleoside is m⁶A (N⁶-methyladenosine). In another embodiment, the modified nucleoside is s²U (2-thiouridine). In another embodiment, the modified nucleoside is Ψ (pseudouridine). In another embodiment, the modified nucleoside is Um (2'-O-methyluridine).

In other embodiments, the modified nucleoside is m¹A (1-methyladenosine); m²A (2-methyladenosine); Am (2'-O-methyladenosine); ms²m⁶A (2-methylthio-N⁶-methyladenosine); i⁶A (N⁶-isopentenyladenosine); ms²i6A (2-methylthio-N⁶isopentenyladenosine); io⁶A (N⁶-(cis-hydroxyisopentenyl)adenosine); ms²io⁶A (2-methylthio-N⁶-(cis-hydroxyisopentenyl) adenosine); g⁶A (N⁶-glycinylcarbamoyladenosine); t⁶A (N⁶-threonylcarbamoyladenosine); ms²t⁶A (2-methylthio-N⁶-threonyl carbamoyladenosine); m⁶t⁶A (N⁶-methyl-N⁶-threonylcarbamoyladenosine); hn⁶A(N⁶-hydroxynorvalylcarbamoyladenosine); ms²hn⁶A (2-methylthio-N⁶-hydroxynorvalyl carbamoyladenosine); Ar(p) (2'-O-ribosyladenosine (phosphate)); I (inosine); m¹I (1-methylinosine); m¹Im (1,2'-O-dimethylinosine); m³C (3-methylcytidine); Cm (2'-O-methylcytidine): s²C (2-thiocytidine): ac⁴C (N⁴-acetylcytidine); f⁵C (5-formylcytidine); m⁵Cm (5,2'-O-dimethylcytidine); ac⁴Cm (N⁴-acetyl-2'-O-methylcytidine); k²C (lysidine); m¹G (1-methylguanosine): m²G (N²-methylguanosine); m⁷G (7-methylguanosine); Gm (2'-O-methylguanosine); m²₂G (N²,N²-dimethylguanosine); m²Gm (N²,2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OHyW (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosyl-queuosine); preQ₀ (7-cyano-7-deazaguanosine); preQ₁ (7-aminomethyl-7-deazaguanosine); G⁺ (archaeosine): D (dihydrouridine); m⁵Um (5,2'-O-dimethyluridine); s⁴U (4-thiouridine): m⁵s²U (5-methyl-2-thiouridine); s²Um (2-thio-2'-O-methyluridine); acp³U (3-(3-amino-3-carboxypropyl)uridine): ho⁵U (5-hydroxyuridine); mo⁵U (5-methoxyuridine): cmo⁵U (uridine 5-oxyacetic acid): mcmo⁵U (uridine 5-oxyacetic acid methyl ester); chm⁵U (5-(carboxyhydroxymethyl)uridine)); mchm⁵U (5-(carboxyhydroxymethyl)uridine methyl ester); mcm⁵U (5-methoxycarbonylmethyluridine); mcm⁵Um (5-methoxycarbonylmethyl-2'-O-methyluridine): mcm⁵s²U (5-methoxycarbonylmethyl-2-thiouridine); nm⁵s²U (5-aminomethyl-2-thiouridine): mnm⁵U (5-methylaminomethyluridine); mnm⁵s²U (5-methylaminomethyl-2-thiouridine): mnm⁵se²U (5-methylaminomethyl-2-selenouridine); ncm⁵U (5-carbamoylmethyluridine): ncm⁵Um (5-carbamoylmethyl-2'-O-methyluridine); cmnm⁵U (5-carboxymethylaminomethyluridine); cmnm⁵Um (5-carboxymethylaminomethyl-2'-O-methyluridine): cmnm⁵s²U (5-carboxymethylaminomethyl-2-thiouridine); m⁶₂A (N⁶,N⁶-dimethyladenosine); Im (2'-O-methylinosine); m⁴C (N⁴-methylcytidine); m⁴Cm (N⁴,2'-O-dimethylcytidine); hm⁵C (5-hydroxymethylcytidine); m³U (3-methyluridine); cm⁵U (5-carboxymethyluridine): m⁶Am (N⁶,2'-O-dimethyladenosine); m⁶₂Am (N⁶,N⁶,O-2'-trimethyladenosine); m^{2,7}G (N²,7-dimethylguanosine); m^{2,2,7}G (N²,N²,7-trimethylguanosine); m³Um (3,2'-O-dimethyluridine): m⁵D (5-methyldihydrouridine); f⁵Cm
(5-formyl-2'-O-methylcytidine): m¹Gm (1,2'-O-dimethylguanosine); m¹Am (1,2'-O-dimethyladenosine); τm⁵U (5-taurinomethyluridine); τm⁵s²U (5-taurinomethyl-2-thiouridine)); imG-14 (4-demethylwyosine); imG2 (isowyosine); or ac⁶A (N⁶-acetyladenosine).

In another embodiment, a nucleoside-modified RNA of the present invention comprises a combination of 2 or more of the above modifications. In another embodiment, the nucleoside-modified RNA comprises a combination of 3 or more of the above modifications. In another embodiment, the nucleoside-modified RNA comprises a combination of more than 3 of the above modifications.

In another embodiment, between 0.1% and 100% of the residues in the nucleoside-modified of the present invention are modified (e.g. either by the presence of pseudouridine or a modified nucleoside base). In another embodiment, 0.1% of the residues are modified. In another embodiment, the fraction of modified residues is 0.2%. In another embodiment, the fraction is 0.3%. In another embodiment, the fraction is 0.4%. In another embodiment, the fraction is 0.5%. In another embodiment, the fraction is 0.6%. In another embodiment, the fraction is 0.8%. In another embodiment, the fraction is 1%. In another embodiment, the fraction is 1.5%. In another embodiment, the fraction is 2%. In another embodiment, the fraction is 2.5%. In another embodiment, the fraction is 3%. In another embodiment, the fraction is 4%. In another embodiment, the fraction is 5%. In another embodiment, the fraction is 6%. In another embodiment, the fraction is 8%. In another embodiment, the fraction is 10%. In another embodiment, the fraction is 12%. In another embodiment, the fraction is 14%. In another embodiment, the fraction is 16%. In another embodiment, the fraction is 18%. In another embodiment, the fraction is 20%. In another embodiment, the fraction is 25%. In another embodiment, the fraction is 30%. In another embodiment, the fraction is 35%. In another embodiment, the fraction is 40%. In another embodiment, the fraction is 45%. In another embodiment, the fraction is 50%. In another embodiment, the fraction is 60%. In another embodiment, the fraction is 70%. In another embodiment, the fraction is 80%. In another embodiment, the fraction is 90%. In another embodiment, the fraction is 100%.

In another embodiment, the fraction is less than 5%. In another embodiment, the fraction is less than 3%. In another embodiment, the fraction is less than 1%. In another embodiment, the fraction is less than 2%. In another embodiment, the fraction is less than 4%. In another embodiment, the fraction is less than 6%. In another embodiment, the fraction is less than 8%. In another embodiment, the fraction is less than 10%. In another embodiment, the fraction is less than 12%. In another embodiment, the fraction is less than 15%. In another embodiment, the fraction is less than 20%. In another embodiment, the fraction is less than 30%. In another embodiment, the fraction is less than 40%. In another embodiment, the fraction is less than 50%. In another embodiment, the fraction is less than 60%. In another embodiment, the fraction is less than 70%.

In another embodiment, 0.1% of the residues of a given nucleoside (i.e., uridine, cytidine, guanosine, or adenosine) are modified. In another embodiment, the fraction of the given nucleotide that is modified is 0.2%. In another embodiment, the fraction is 0.3%. In another embodiment, the fraction is 0.4%. In another embodiment, the fraction is 0.5%. In another embodiment, the fraction is 0.6%. In another embodiment, the fraction is 0.8%. In another embodiment, the fraction is 1%. In another embodiment, the fraction is 1.5%. In another embodiment, the fraction is 2%. In another embodiment, the fraction is 2.5%. In another embodiment, the fraction is 3%. In another embodiment, the fraction is 4%. In another embodiment, the fraction is 5%. In another embodiment, the fraction is 6%. In another embodiment, the fraction is 8%. In another embodiment, the fraction is 10%. In another embodiment, the fraction is 12%. In another embodiment, the fraction is 14%. In another embodiment, the fraction is 16%. In another embodiment, the fraction is 18%. In another embodiment, the fraction is 20%. In another embodiment, the fraction is 25%. In another embodiment, the fraction is 30%. In another embodiment, the fraction is 35%. In another embodiment, the fraction is 40%. In another embodiment, the fraction is 45%. In another embodiment, the fraction is 50%. In another embodiment, the fraction is 60%. In another embodiment, the fraction is 70%. In another embodiment, the fraction is 80%. In another embodiment, the fraction is 90%. In another embodiment, the fraction is 100%.

In another embodiment, the fraction of the given nucleotide that is modified is less than 8%. In another embodiment, the fraction is less than 10%. In another embodiment, the fraction is less than 5%. In another embodiment, the fraction is less than 3%. In another embodiment, the fraction is less than 1%. In another embodiment, the fraction is less than 2%. In another embodiment, the fraction is less than 4%. In another embodiment, the fraction is less than 6%. In another embodiment, the fraction is less than 12%. In another embodiment, the fraction is less than 15%. In another embodiment, the fraction is less than 20%. In another embodiment, the fraction is less than 30%. In another embodiment, the fraction is less than 40%. In another embodiment, the fraction is less than 50%. In another embodiment, the fraction is less than 60%. In another embodiment, the fraction is less than 70%.

In some embodiments, the composition comprises a purified preparation of single-stranded nucleoside modified RNA. For example, in some embodiments, the purified preparation of single-stranded nucleoside modified RNA is substantially free of double stranded RNA (dsRNA). In some embodiments, the purified preparation is at least 90%, or at least 91%, or at least 92%, or at least 93 % or at least 94%, or at least 95%, or at least 96%, or at least 97%, or at least 98%, or at least 99%, or at least 99.5%, or at least 99.9% single stranded nucleoside modified RNA, relative to all other nucleic acid molecules (DNA, dsRNA, etc.).

In another embodiment, a nucleoside-modified RNA of the present invention is translated in the cell more efficiently than an unmodified RNA molecule with the same sequence. In another embodiment, the nucleoside-modified RNA exhibits enhanced ability to be translated by a target cell. In another embodiment, translation is enhanced by a factor of 2-fold relative to its unmodified counterpart. In another embodiment, translation is enhanced by a 3-fold factor. In another embodiment, translation is enhanced by a 5-fold factor. In another embodiment, translation is enhanced by a 7-fold factor. In another embodiment, translation is enhanced by a 10-fold factor. In another embodiment, translation is enhanced by a 15-fold factor. In another embodiment, translation is enhanced by a 20-fold factor. In another embodiment, translation is enhanced by a 50-fold factor. In another embodiment, translation is enhanced by a 100-fold factor. In another embodiment, translation is enhanced by a 200-fold factor. In another embodiment, translation is enhanced by a 500-fold factor. In another embodiment, translation is enhanced by a 1000-fold factor. In another embodiment, translation is enhanced by a 2000-fold factor. In another embodiment, the factor is 10-1000-fold. In another embodiment, the factor is 10-100-fold. In another embodiment, the factor is 10-200-fold. In another embodiment, the factor is 10-300-fold. In another embodiment, the factor is 10-500-fold. In another embodiment, the factor is 20-1000-fold. In another embodiment, the factor is 30-1000-fold. In another embodiment, the factor is 50-1000-fold. In another embodiment, the factor is 100-1000-fold. In another embodiment, the factor is 200-1000-fold. In another embodiment, translation is enhanced by any other significant amount or range of amounts.

In another embodiment, the nucleoside-modified RNA of the present invention exhibits significantly less innate immunogenicity than an unmodified in vitro-synthesized RNA molecule of the same sequence. In another embodiment, the modified RNA molecule exhibits an innate immune response that is 2-fold less than its unmodified counterpart. In another embodiment, innate immunogenicity is reduced by a 3-fold factor. In another embodiment, innate immunogenicity is reduced by a 4-fold factor. In another embodiment, innate immunogenicity is reduced by a 5-fold factor. In another embodiment, innate immunogenicity is reduced by a 6-fold factor. In another embodiment, innate immunogenicity is reduced by a 7-fold factor. In another embodiment, innate immunogenicity is reduced by a 8-fold factor. In another embodiment, innate immunogenicity is reduced by a 9-fold factor. In another embodiment, innate immunogenicity is reduced by a 10-fold factor. In another embodiment, innate immunogenicity is reduced by a 15-fold factor. In another embodiment, innate immunogenicity is reduced by a 20-fold factor. In another embodiment, innate immunogenicity is reduced by a 50-fold factor. In another embodiment, innate immunogenicity is reduced by a 100-fold factor. In another embodiment, innate immunogenicity is reduced by a 200-fold factor. In another embodiment, innate immunogenicity is reduced by a 500-fold factor. In another embodiment, innate immunogenicity is reduced by a 1000-fold factor. In another embodiment, innate immunogenicity is reduced by a 2000-fold factor. In another embodiment, innate immunogenicity is reduced by another fold difference.

In another embodiment, "exhibits significantly less innate immunogenicity" refers to a detectable decrease in innate immunogenicity. In another embodiment, the term refers to a fold decrease in innate immunogenicity (e.g., 1 of the fold decreases enumerated above). In another embodiment, the term refers to a decrease such that an effective amount of the nucleoside-modified RNA can be administered without triggering a detectable innate immune response. In another embodiment, the term refers to a decrease such that the nucleoside-modified RNA can be repeatedly administered without eliciting an innate immune response sufficient to detectably reduce production of the protein encoded by the modified RNA. In another embodiment, the decrease is such that the nucleoside-modified RNA can be repeatedly administered without eliciting an innate immune response sufficient to eliminate detectable production of the protein encoded by the modified RNA.

### RNA interference agents

In one embodiment, siRNA is used to decrease the level of a targeted protein. RNA interference (RNAi) is a phenomenon in which the introduction of double-stranded RNA (dsRNA) into a diverse range of organisms and cell types causes degradation of the complementary mRNA. In the cell, long dsRNAs are cleaved into short 21-25 nucleotide small interfering RNAs, or siRNAs, by a ribonuclease known as Dicer. The siRNAs subsequently assemble with protein components into an RNA-induced silencing complex (RISC), unwinding in the process. Activated RISC then binds to complementary transcript by base pairing interactions between the siRNA antisense strand and the mRNA. The bound mRNA is cleaved and sequence specific degradation of mRNA results in gene silencing. See, for example, U.S. Patent No. 6,506,559; Fire et al., 1998, Nature 391(19):306-311: Timmons et al., 1998, Nature 395:854; Montgomery et al., 1998, TIG 14 (7):255-258: David R. Engelke, Ed., RNA Interference (RNAi) Nuts & Bolts of RNAi Technology, DNA Press, Eagleville, PA (2003); and Gregory J. Hannon, Ed., RNAi A Guide to Gene Silencing, Cold Spring Harbor Laboratory Press, Cold Spring Harbor, NY (2003). Soutschek et al. (2004, Nature 432:173-178) describe a chemical modification to siRNAs that aids in intravenous systemic delivery. Optimizing siRNAs involves consideration of overall G/C content, C/T content at the termini, Tm and the nucleotide content of the 3' overhang. See, for instance, Schwartz et al., 2003, Cell, 115:199-208 and Khvorova et al., 2003, Cell 115:209-216. Therefore, the present invention also includes methods of decreasing levels of PTPN22 using RNAi technology.

In one aspect, the invention includes a vector comprising an siRNA or an antisense polynucleotide. Preferably, the siRNA or antisense polynucleotide is capable of inhibiting the expression of a target polypeptide. The incorporation of a desired polynucleotide into a vector and the choice of vectors are well-known in the art as described in, for example, Sambrook et al. (2012), and in Ausubel et al. (1997), and elsewhere herein.

In certain embodiments, the expression vectors described herein encode a short hairpin RNA (shRNA) agents. shRNA molecules are well known in the art and are directed against the mRNA of a target, thereby decreasing the expression of the target. In certain embodiments, the encoded shRNA is expressed by a cell, and is then processed into siRNA. For example, in certain instances, the cell possesses native enzymes (e.g., dicer) that cleave the shRNA to form siRNA.

In order to assess the expression of the siRNA, shRNA, or antisense polynucleotide, the expression vector to be introduced into a cell can also contain either a selectable marker gene or a reporter gene or both to facilitate identification of expressing cells from the population of cells sought to be transfected or infected using the delivery vehicle of the invention. In other embodiments, the selectable marker may be carried on a separate piece of DNA and also be contained within the delivery vehicle. Both selectable markers and reporter genes may be flanked with appropriate regulatory sequences to enable expression in the host cells. Useful selectable markers are known in the art and include, for example, antibiotic-resistance genes, such as neomycin resistance and the like.

Therefore, in one aspect, the delivery vehicle may contain a vector, comprising the nucleotide sequence or the construct to be delivered. The choice of the vector will depend on the host cell in which it is to be subsequently introduced. In a particular embodiment, the vector of the invention is an expression vector. Suitable host cells include a wide variety of prokaryotic and eukaryotic host cells. In specific embodiments, the expression vector is selected from the group consisting of a viral vector, a bacterial vector and a mammalian cell vector. Prokaryote- and/or eukaryote-vector based systems can be employed for use with the present invention to produce polynucleotides, or their cognate polypeptides. Many such systems are commercially and widely available.

By way of illustration, the vector in which the nucleic acid sequence is introduced can be a plasmid, which is or is not integrated in the genome of a host cell when it is introduced in the cell. Illustrative, non-limiting examples of vectors in which the nucleotide sequence of the invention or the gene construct of the invention can be inserted include a tet-on inducible vector for expression in eukaryote cells.

The vector may be obtained by conventional methods known by persons skilled in the art (Sambrook et al., 2012). In a particular embodiment, the vector is a vector useful for transforming animal cells.

In one embodiment, the recombinant expression vectors may also contain nucleic acid molecules, which encode a peptide or peptidomimetic.

A promoter may be one naturally associated with a gene or polynucleotide sequence, as may be obtained by isolating the 5' non-coding sequences located upstream of the coding segment and/or exon. Such a promoter can be referred to as "endogenous." Similarly, an enhancer may be one naturally associated with a polynucleotide sequence, located either downstream or upstream of that sequence. Alternatively, certain advantages will be gained by positioning the coding polynucleotide segment under the control of a recombinant or heterologous promoter, which refers to a promoter that is not normally associated with a polynucleotide sequence in its natural environment. A recombinant or heterologous enhancer refers also to an enhancer not normally associated with a polynucleotide sequence in its natural environment. Such promoters or enhancers may include promoters or enhancers of other genes, and promoters or enhancers isolated from any other prokaryotic, viral, or eukaryotic cell, and promoters or enhancers not "naturally occurring," i.e., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR^{™}, in connection with the compositions disclosed herein (U.S. Patent 4,683,202, U.S. Patent 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the cell type, organelle, and organism chosen for expression. Those of skill in the art of molecular biology generally know how to use promoters, enhancers, and cell type combinations for protein expression, for example, see Sambrook et al. (2012). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

The recombinant expression vectors may also contain a selectable marker gene, which facilitates the selection of host cells. Suitable selectable marker genes are genes encoding proteins such as G418 and hygromycin, which confer resistance to certain drugs, β-galactosidase, chloramphenicol acetyltransferase, firefly luciferase, or an immunoglobulin or portion thereof such as the Fc portion of an immunoglobulin preferably IgG. The selectable markers may be introduced on a separate vector from the nucleic acid of interest.

Following the generation of the siRNA polynucleotide, a skilled artisan will understand that the siRNA polynucleotide will have certain characteristics that can be modified to improve the siRNA as a therapeutic compound. Therefore, the siRNA polynucleotide may be further designed to resist degradation by modifying it to include phosphorothioate, or other linkages, methylphosphonate, sulfone, sulfate, ketyl, phosphorodithioate, phosphoramidate, phosphate esters, and the like (see, e.g., Agrawal et al., 1987, Tetrahedron Lett. 28:3539-3542; Stec et al., 1985 Tetrahedron Lett. 26:2191-2194; Moody et al., 1989 Nucleic Acids Res. 12:4769-4782: Eckstein, 1989 Trends Biol. Sci. 14:97-100; Stein, In: Oligodeoxynucleotides. Antisense Inhibitors of Gene Expression, Cohen, ed., Macmillan Press, London, pp. 97-117 (1989)).

Any polynucleotide may be further modified to increase its stability in vivo. Possible modifications include, but are not limited to, the addition of flanking sequences at the 5' and/or 3' ends; the use of phosphorothioate or 2' O-methyl rather than phosphodiester linkages in the backbone; and/or the inclusion of nontraditional bases such as inosine, queuosine, and wybutosine and the like, as well as acetyl- methyl-, thio- and other modified forms of adenine, cytidine, guanine, thymine, and uridine.

In one embodiment of the invention, an antisense nucleic acid sequence, which is expressed by a plasmid vector is used as an agent to inhibit the expression of a target protein. The antisense expressing vector is used to transfect a mammalian cell or the mammal itself, thereby causing reduced endogenous expression of the target protein.

Antisense molecules and their use for inhibiting gene expression are well known in the art (see, e.g., Cohen, 1989, In: Oligodeoxyribonucleotides, Antisense Inhibitors of Gene Expression, CRC Press). Antisense nucleic acids are DNA or RNA molecules that are complementary, as that term is defined elsewhere herein, to at least a portion of a specific mRNA molecule (Weintraub, 1990, Scientific American 262:40). In the cell, antisense nucleic acids hybridize to the corresponding mRNA, forming a double-stranded molecule thereby inhibiting the translation of genes.

The use of antisense methods to inhibit the translation of genes is known in the art, and is described, for example, in Marcus-Sakura (1988, Anal. Biochem. 172:289). Such antisense molecules may be provided to the cell via genetic expression using DNA encoding the antisense molecule as taught by Inoue, 1993, U.S. Patent No. 5,190,931.

Alternatively, antisense molecules of the invention may be made synthetically and then provided to the cell. Antisense oligomers of between about 10 to about 30, and more preferably about 15 nucleotides, are preferred, since they are easily synthesized and introduced into a target cell. Synthetic antisense molecules contemplated by the invention include oligonucleotide derivatives known in the art which have improved biological activity compared to unmodified oligonucleotides (see U.S. Patent No. 5,023,243).

In one embodiment of the invention, a ribozyme is used as an agent to inhibit expression of a target protein. Ribozymes useful for inhibiting the expression of a target molecule may be designed by incorporating target sequences into the basic ribozyme structure, which are complementary, for example, to the mRNA sequence encoding the target molecule. Ribozymes targeting the target molecule, may be synthesized using commercially available reagents (Applied Biosystems, Inc., Foster City, CA) or they may be genetically expressed from DNA encoding them.

In one embodiment, the agent may comprise one or more components of a CRISPR-Cas system, where a guide RNA (gRNA) targeted to a gene encoding a target molecule, and a CRISPR-associated (Cas) peptide form a complex to induce mutations within the targeted gene. In one embodiment, the agent comprises a gRNA or a nucleic acid molecule encoding a gRNA. In one embodiment, the agent comprises a Cas peptide or a nucleic acid molecule encoding a Cas peptide.

### microRNA agents

In one embodiment, the agent comprises a miRNA or a mimic of a miRNA. In one embodiment, the agent comprises a nucleic acid molecule that encodes a miRNA or mimic of a miRNA.

MiRNAs are small non-coding RNA molecules that are capable of causing post-transcriptional silencing of specific genes in cells by the inhibition of translation or through degradation of the targeted mRNA. A miRNA can be completely complementary or can have a region of non complementarity with a target nucleic acid, consequently resulting in a "bulge" at the region of non-complementarity. A miRNA can inhibit gene expression by repressing translation, such as when the miRNA is not completely complementary to the target nucleic acid, or by causing target RNA degradation, which is believed to occur only when the miRNA binds its target with perfect complementarity. The disclosure also can include double-stranded precursors of miRNA. A miRNA or pri-miRNA can be 18- 100 nucleotides in length, or from 18-80 nucleotides in length. Mature miRNAs can have a length of 19-30 nucleotides, or 21-25 nucleotides, particularly 21, 22, 23, 24, or 25 nucleotides. MiRNA precursors typically have a length of about 70-100 nucleotides and have a hairpin conformation. miRNAs are generated in vivo from pre- miRNAs by the enzymes Dicer and Drosha, which specifically process long pre-miRNA into functional miRNA. The hairpin or mature microRNAs, or pri-microRNA agents featured in the disclosure can be synthesized in vivo by a cell-based system or in vitro by chemical synthesis.

In various embodiments, the agent comprises an oligonucleotide that comprises the nucleotide sequence of a disease-associated miRNA. In certain embodiments, the oligonucleotide comprises the nucleotide sequence of a disease-associated miRNA in a pre -microRNA, mature or hairpin form. In other embodiments, a combination of oligonucleotides comprising a sequence of one or more disease-associated miRNAs, any pre -miRNA, any fragment, or any combination thereof is envisioned.

MiRNAs can be synthesized to include a modification that imparts a desired characteristic. For example, the modification can improve stability, hybridization thermodynamics with a target nucleic acid, targeting to a particular tissue or cell -type, or cell permeability, e.g., by an endocytosis-dependent or -independent mechanism.

Modifications can also increase sequence specificity, and consequently decrease off-site targeting. Methods of synthesis and chemical modifications are described in greater detail below. If desired, miRNA molecules may be modified to stabilize the miRNAs against degradation, to enhance half-life, or to otherwise improve efficacy. Desirable modifications are described, for example, in U.S. Patent Publication Nos. 20070213292, 20060287260, 20060035254. 20060008822. and 2005028824, each of which is hereby incorporated by reference in its entirety. For increased nuclease resistance and/or binding affinity to the target, the single- stranded oligonucleotide agents featured in the disclosure can include 2'-O-methyl, 2'-fluorine, 2'-O-methoxyethyl, 2'-O-aminopropyl, 2'-amino, and/or phosphorothioate linkages. Inclusion of locked nucleic acids (LNA), ethylene nucleic acids (ENA), e.g., 2'-4'-ethylene- bridged nucleic acids, and certain nucleotide modifications can also increase binding affinity to the target. The inclusion of pyranose sugars in the oligonucleotide backbone can also decrease endonucleolytic cleavage. An oligonucleotide can be further modified by including a 3' cationic group, or by inverting the nucleoside at the 3'-terminus with a 3 -3' linkage. In another alternative, the 3 '-terminus can be blocked with an aminoalkyl group. Other 3' conjugates can inhibit 3'-5' exonucleolytic cleavage. While not being bound by theory, a 3' may inhibit exonucleolytic cleavage by sterically blocking the exonuclease from binding to the 3' end of the oligonucleotide. Even small alkyl chains, aryl groups, or heterocyclic conjugates or modified sugars (D-ribose, deoxyribose, glucose etc.) can block 3'-5'-exonucleases.

In one embodiment, the miRNA includes a 2'-modified oligonucleotide containing oligodeoxynucleotide gaps with some or all internucleotide linkages modified to phosphorothioates for nuclease resistance. The presence of methylphosphonate modifications increases the affinity of the oligonucleotide for its target RNA and thus reduces the IC₅Q. This modification also increases the nuclease resistance of the modified oligonucleotide. It is understood that the methods and reagents of the present disclosure may be used in conjunction with any technologies that may be developed to enhance the stability or efficacy of an inhibitory nucleic acid molecule.

miRNA molecules include nucleotide oligomers containing modified backbones or non-natural internucleoside linkages. Oligomers having modified backbones include those that retain a phosphorus atom in the backbone and those that do not have a phosphorus atom in the backbone. For the purposes of this disclosure, modified oligonucleotides that do not have a phosphorus atom in their internucleoside backbone are also considered to be nucleotide oligomers. Nucleotide oligomers that have modified oligonucleotide backbones include, for example, phosphorothioates, chiral phosphorothioates, phosphorodithioates, phosphotriesters, aminoalkyl-phosphotriesters, methyl and other alkyl phosphonates including 3'-alkylene phosphonates and chiral phosphonates, phosphinates, phosphoramidates, thionophosphoramidates, thionoalkylphosphonates, thionoalkylphosphotriest- ers, and boranophosphates. Various salts, mixed salts and free acid forms are also included.

A miRNA described herein, which may be in the mature or hairpin form, may be provided as a naked oligonucleotide. In some cases, it may be desirable to utilize a formulation that aids in the delivery of a miRNA or other nucleotide oligomer to cells (see, e.g., U.S. Pat. Nos. 5,656,61 1, 5,753,613, 5,785,992, 6,120,798, 6.221.959, 6,346,613, and 6,353,055, each of which is hereby incorporated by reference).

In some examples, the miRNA composition is at least partially crystalline, uniformly crystalline, and/or anhydrous (e.g., less than 80, 50, 30, 20, or 10% water). In another example, the miRNA composition is in an aqueous phase, e.g., in a solution that includes water. The aqueous phase or the crystalline compositions can be incorporated into a delivery vehicle, e.g., a liposome (particularly for the aqueous phase), or a particle (e.g., a microparticle as can be appropriate for a crystalline composition). Generally, the miRNA composition is formulated in a manner that is compatible with the intended method of administration. A miRNA composition can be formulated in combination with another agent, e.g., another therapeutic agent or an agent that stabilizes an oligonucleotide agent, e.g., a protein that complexes with the oligonucleotide agent. Still other agents include chelators, e.g., EDTA (e.g., to remove divalent cations such as Mg), salts, and RNAse inhibitors (e.g., a broad specificity RNAse inhibitor). In one embodiment, the miRNA composition includes another miRNA, e.g., a second miRNA composition (e.g., a microRNA that is distinct from the first). Still other preparations can include at least three, five, ten, twenty, fifty, or a hundred or more different oligonucleotide species.

In certain embodiments, the composition comprises an oligonucleotide composition that mimics the activity of a miRNA. In certain embodiments, the composition comprises oligonucleotides having nucleobase identity to the nucleobase sequence of a miRNA, and are thus designed to mimic the activity of the miRNA. In certain embodiments, the oligonucleotide composition that mimics miRNA activity comprises a double-stranded RNA molecule which mimics the mature miRNA hairpins or processed miRNA duplexes.

In one embodiment, the oligonucleotide shares identity with endogenous miRNA or miRNA precursor nucleobase sequences. An oligonucleotide selected for inclusion in a composition of the present invention may be one of a number of lengths. Such an oligonucleotide can be from 7 to 100 linked nucleosides in length. For example, an oligonucleotide sharing nucleobase identity with a miRNA may be from 7 to 30 linked nucleosides in length. An oligonucleotide sharing identity with a miRNA precursor may be up to 100 linked nucleosides in length. In certain embodiments, an oligonucleotide comprises 7 to 30 linked nucleosides. In certain embodiments, an oligonucleotide comprises 7, 8, 9, 10, 11, 12, 13, 14, 15, 16, 17, 18, 19, 20, 21, 22, 23, 24, 25, 26, 28, 29, or 30 linked nucleotides. In certain embodiments, an oligonucleotide comprises 19 to 23 linked nucleosides. In certain embodiments, an oligonucleotide is from 40 up to 50, 60, 70, 80, 90, or 100 linked nucleosides in length.

In certain embodiments, an oligonucleotide has a sequence that has a certain identity to a miRNA or a precursor thereof. Nucleobase sequences of mature miRNAs and their corresponding stem-loop sequences described herein are the sequences found in miRBase, an online searchable database of miRNA sequences and annotation. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the re-naming of certain miRNAs. A sequence database release may result in a variation of a mature miRNA sequence. The compositions of the present invention encompass oligomeric compound comprising oligonucleotides having a certain identity to any nucleobase sequence version of a miRNAs described herein.

In certain embodiments, an oligonucleotide has a nucleobase sequence at least 60%, 65%, 70%, 75%, 80%, 85%, 90%, 95%, 97%, 98% or 99% identical to the miRNA over a region of 7, 8, 9, 10, 11, 12, 13, 14, 15, 16, 17, 18, 19, 20, 21, 22, 23, 24, 25, 26, 27, 28, 29, or 30 nucleobases. Accordingly, in certain embodiments the nucleobase sequence of an oligonucleotide may have one or more non-identical nucleobases with respect to the miRNA.

In certain embodiments, the composition comprises a nucleic acid molecule encoding a miRNA, precursor, mimic, or fragment thereof. For example, the composition may comprise a viral vector, plasmid, cosmid, or other expression vector suitable for expressing the miRNA, precursor, mimic, or fragment thereof in a desired mammalian cell or tissue.

### In vitro transcribed RNA agents

In one embodiment, the agent of the invention comprises in vitro transcribed (IVT) RNA. In one embodiment, the agent of the invention comprises in vitro transcribed (IVT) RNA encoding a therapeutic protein. In one embodiment, the agent of the invention comprises IVT RNA encoding a plurality of therapeutic proteins.

In one embodiment, an IVT RNA can be introduced to a cell as a form of transient transfection. The RNA is produced by in vitro transcription using a plasmid DNA template generated synthetically. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. In one embodiment, the desired template for in vitro transcription is a therapeutic protein, as described elsewhere herein.

In one embodiment, the DNA to be used for PCR contains an open reading frame. The DNA can be from a naturally occurring DNA sequence from the genome of an organism. In one embodiment, the DNA is a full-length gene of interest of a portion of a gene. The gene can include some or all of the 5' and/or 3' untranslated regions (UTRs). The gene can include exons and introns. In one embodiment, the DNA to be used for PCR is a human gene. In another embodiment, the DNA to be used for PCR is a human gene including the 5' and 3' UTRs. In another embodiment, the DNA to be used for PCR is a gene from a pathogenic or commensal organism, including bacteria, viruses, parasites, and fungi. In another embodiment, the DNA to be used for PCR is from a pathogenic or commensal organism, including bacteria, viruses, parasites, and fungi, including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

Genes that can be used as sources of DNA for PCR include genes that encode polypeptides that induce or enhance an adaptive immune response in an organism. Preferred genes are genes which are useful for a short-term treatment, or where there are safety concerns regarding dosage or the expressed gene.

In various embodiments, a plasmid is used to generate a template for in vitro transcription of RNA which is used for transfection.

Chemical structures with the ability to promote stability and/or translation efficiency may also be used. The RNA preferably has 5' and 3' UTRs. In one embodiment, the 5' UTR is between zero and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the gene of interest. Alternatively, UTR sequences that are not endogenous to the gene of interest can be added by incorporating the UTR sequences into the forward and reverse primers or by any other modifications of the template. The use of UTR sequences that are not endogenous to the gene of interest can be useful for modifying the stability and/or translation efficiency of the RNA. For example, it is known that AU-rich elements in 3' UTR sequences can decrease the stability of RNA. Therefore, 3' UTRs can be selected or designed to increase the stability of the transcribed RNA based on properties of UTRs that are well known in the art.

In one embodiment, the 5' UTR can contain the Kozak sequence of the endogenous gene. Alternatively, when a 5' UTR that is not endogenous to the gene of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many RNAs is known in the art. In other embodiments the 5' UTR can be derived from an RNA virus whose RNA genome is stable in cells. In other embodiments various nucleotide analogues can be used in the 3' or 5' UTR to impede exonuclease degradation of the RNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 RNA polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the RNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized RNA which is effective in eukaryotic transfection when it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which can be ameliorated through the use of recombination incompetent bacterial cells for plasmid propagation.

Poly(A) tails of RNAs can be further extended following in vitro transcription with the use of a poly(A) polymerase, such as E. coli polyA polymerase (E-PAP) or yeast polyA polymerase. In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase RNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods to include a 5' cap1 structure. Such cap1 structure can be generated using Vaccinia capping enzyme and 2'-O-methyltransferase enzymes (CellScript, Madison, WI). Alternatively, 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

### Polypeptide agents

In other related aspects, the agent includes an isolated peptide that modulates a target. For example, in one embodiment, the peptide of the invention inhibits or activates a target directly by binding to the target thereby modulating the normal functional activity of the target. In one embodiment, the peptide of the invention modulates the target by competing with endogenous proteins. In one embodiment, the peptide of the invention modulates the activity of the target by acting as a transdominant negative mutant.

The variants of the polypeptide agents may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the polypeptide is an alternative splice variant of the polypeptide of the present invention, (iv) fragments of the polypeptides and/or (v) one in which the polypeptide is fused with another polypeptide, such as a leader or secretory sequence or a sequence which is employed for purification (for example, His-tag) or for detection (for example, Sv5 epitope tag). The fragments include polypeptides generated via proteolytic cleavage (including multi-site proteolysis) of an original sequence. Variants may be post-translationally, or chemically modified. Such variants are deemed to be within the scope of those skilled in the art from the teaching herein.

### Antibody agents

The invention also contemplates a delivery vehicle comprising an antibody, or antibody fragment, specific for a target. That is, the antibody can inhibit a target to provide a beneficial effect.

The antibodies may be intact monoclonal or polyclonal antibodies, and immunologically active fragments (e.g., a Fab or (Fab)2 fragment), an antibody heavy chain, an antibody light chain, humanized antibodies, a genetically engineered single chain FV molecule (Ladner et al, U.S. Pat. No. 4,946,778), or a chimeric antibody, for example, an antibody which contains the binding specificity of a murine antibody, but in which the remaining portions are of human origin. Antibodies including monoclonal and polyclonal antibodies, fragments and chimeras, may be prepared using methods known to those skilled in the art.

Antibodies can be prepared using intact polypeptides or fragments containing an immunizing antigen of interest. The polypeptide or oligopeptide used to immunize an animal may be obtained from the translation of RNA or synthesized chemically and can be conjugated to a carrier protein, if desired. Suitable carriers that may be chemically coupled to peptides include bovine serum albumin and thyroglobulin, keyhole limpet hemocyanin. The coupled polypeptide may then be used to immunize the animal (e.g., a mouse, a rat, or a rabbit).

### CAR agents

In one embodiment, the agent comprises a recombinant nucleic acid sequence encoding a chimeric antigen receptor (CAR). In one embodiment, the agent comprises a mRNA molecule encoding a CAR. In one embodiment, the agent comprises a nucleoside modified mRNA molecule encoding a CAR.

The term "chimeric antigen receptor" or "CAR." as used herein, refers to an artificial T cell receptor that is engineered to be expressed on an immune effector cell and specifically bind an antigen. CARs may be used as a therapy with adoptive cell transfer. T cells are removed from a patient and modified so that they express the receptors specific to a particular form of antigen. In some embodiments, the CARs have specificity to a selected target. CARs may also comprise an intracellular activation domain, a transmembrane domain and an extracellular domain comprising an antigen binding region that specifically binds to a selected target. In some aspects, CARs comprise an extracellular domain comprising an anti-B cell binding domain fused to CD3~zeta transmembrane and intracellular domain.

In one embodiment, the invention relates to a delivery vehicle comprising an agent, wherein the agent comprises a recombinant nucleic acid sequence (e.g., an mRNA) encoding a chimeric antigen receptor (CAR). In one embodiment, the agent comprises an mRNA molecule (e.g., a modified nucleoside mRNA molecule) encoding a chimeric antigen receptor (CAR). In one embodiment, agent comprises an mRNA molecule encoding a CAR. In one embodiment, agent comprises a nucleoside modified mRNA molecule encoding a CAR.

In various embodiments, the CARs contemplated herein comprise an extracellular domain, a transmembrane domain, and an intracellular domain. The extracellular domain comprises a target-specific binding element otherwise referred to as an antigen binding domain. In some embodiments, the extracellular domain also comprises a hinge domain. In certain embodiments, the intracellular domain or otherwise the cytoplasmic domain comprises, a costimulatory signaling region and a zeta chain portion. The costimulatory signaling region refers to a portion of the CAR comprising the intracellular domain of a costimulatory molecule. Costimulatory molecules are cell surface molecules other than antigens receptors or their ligands that are required for an efficient response of lymphocytes to antigen.

Between the extracellular domain and the transmembrane domain of the CAR, or between the cytoplasmic domain and the transmembrane domain of the CAR, there may be incorporated a spacer domain. As used herein, the term "spacer domain" generally means any oligo- or polypeptide that functions to link the transmembrane domain to, either the extracellular domain or, the cytoplasmic domain in the polypeptide chain. A spacer domain may comprise up to 5 amino acids, or 10 amino acids, or 20 amino acids, or 30 amino acids, or 40 amino acids, or 50 amino acids, or 60 amino acids, or 70 amino acids, or 80 amino acids, or 90 amino acids, or 100 amino acids, or 110 amino acids, or 120 amino acids, or 130 amino acids, or 140 amino acids, or 150 amino acids, or 160 amino acids, or 170 amino acids, or 180 amino acids, or 190 amino acids, or 200 amino acids, or 210 amino acids, or 220 amino acids, or 230 amino acids, or 240 amino acids, or 250 amino acids, or 260 amino acids, or 270 amino acids, or 280 amino acids, or 290 amino acids, or 300 amino acids.

The extracellular domain, transmembrane domain, and intracellular domain can be derived from any desired source of such domains.

### CAR antigen binding domain

The antigen binding domain may be obtained from any of the wide variety of extracellular domains or secreted proteins associated with ligand binding and/or signal transduction. In one embodiment, the antigen binding domain may consist of an Ig heavy chain which may in turn be covalently associated with Ig light chain by virtue of the presence of CHI and hinge regions, or may become covalently associated with other Ig heavy/light chain complexes by virtue of the presence of hinge, CH2 and CH3 domains. In the latter case, the heavy/light chain complex that becomes joined to the chimeric construct may constitute an antibody with a specificity distinct from the antibody specificity of the chimeric construct. Depending on the function of the antibody, the desired structure and the signal transduction, the entire chain may be used or a truncated chain may be used, where all or a part of the CHI, CH2, or CH3 domains may be removed or all or part of the hinge region may be removed.

In various embodiments, the CAR antigen binding domain may be humanized or comprise a fully human sequence.

### CAR transmembrane domain

With respect to the transmembrane domain, a CAR of the disclosure can be designed to comprise a transmembrane domain that is fused to the extracellular domain of the CAR. In one embodiment, the transmembrane domain that naturally is associated with one of the domains in the CAR is used. In some instances, the transmembrane domain can be selected or modified by amino acid substitution to avoid binding of such domains to the transmembrane domains of the same or different surface membrane proteins to minimize interactions with other members of the receptor complex.

The transmembrane domain may be derived either from a natural or from a synthetic source. Where the source is natural, the domain may be derived from any membrane-bound or transmembrane protein. Transmembrane regions of particular use in this invention may be derived from (i.e., comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD30, CD86, CD134, CD137, or CD 154. Alternatively, the transmembrane domain may be synthetic, in which case it will comprise predominantly hydrophobic residues such as leucine and valine. In one embodiment, a triplet of phenylalanine, tryptophan and valine can be found at each end of a synthetic transmembrane domain. Optionally, a short oligo- or polypeptide linker, for example, but not limited to between 2 and 10 amino acids in length, may form the linkage between the transmembrane domain and the cytoplasmic signaling domain of the CAR. In another embodiment, the linker comprises a glycine-serine doublet.

### CAR intracellular domain

In various embodiments, the cytoplasmic domain or otherwise the intracellular domain of a CAR may be responsible for activation of at least one of the normal effector functions of the immune cell in which the CAR is expressed. The term "effector function" refers to a specialized function of a cell. Effector function of a T cell, for example, may be cytolytic activity or helper activity, including the secretion of cytokines. The term "intracellular signaling domain" refers to the portion of a protein which transduces the effector function signal and directs the cell to perform a specialized function. While usually the entire intracellular domain can be employed, in many cases it is not necessary to use the entire chain. To the extent that a truncated portion of the intracellular domain is used, such truncated portion may be used in place of the intact chain as long as it transduces the effector function signal. The term intracellular domain is thus meant to include any truncated portion of the intracellular domain sufficient to transduce the effector function signal.

Preferred examples of intracellular domains for use in the CARs of the disclosure include the cytoplasmic sequences of the T cell receptor (TCR) and co-receptors that act in concert to initiate signal transduction following antigen receptor engagement, as well as any derivative or variant of these sequences and any synthetic sequence that has the same functional capability.

It is known that signals generated through the TCR alone are insufficient for full activation of the T cell and that a secondary or co-stimulatory signal is also required. Thus, T cell activation can be said to be mediated by two classes of intracellular signaling sequences: those that initiate antigen-dependent primary activation through the TCR (primary cytoplasmic signaling sequences) and those that act in an antigen-independent manner to provide a secondary or co-stimulatory signal (secondary cytoplasmic signaling sequences).

Primary intracellular signaling sequences regulate primary activation of the TCR complex either in a stimulatory way, or in an inhibitory way. Primary intracellular signaling sequences that act in a stimulatory manner may contain signaling motifs which are known as immunoreceptor tyrosine-based activation motifs or ITAMs.

Examples of ITAMs containing primary intracellular signaling sequences that are of particular use in the invention include those derived from TCR zeta, FcR gamma, FcR beta, CD3 gamma, CD3 delta, CD3 epsilon, CD5, CD22, CD79a, CD79b, and CD66d. In one embodiment, the intracellular signaling molecule in the CAR of the invention comprises an intracellular signaling sequence derived from CD3 zeta.

In another embodiment, the intracellular domain of the CAR can be designed to comprise the CD3-zeta signaling domain by itself or combined with any other desired cytoplasmic domain(s) useful in the context of the CAR of the invention. For example, the intracellular domain of the CAR can comprise a CD3 zeta chain portion and a costimulatory signaling region. The costimulatory signaling region refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD2, CD27, CD28, 4-1BB (CD137), Ox40, CD30, CD40, PD-1, ICOS, lymphocyte function-associated antigen-1 (LFA-1), CD2, CD7, LIGHT, NKG2C, B7-H3, and a ligand that specifically binds with CD83, and the like.

The intracellular signaling sequences within the intracellular domain of the CAR of the invention may be linked to each other in a random or specified order. Optionally, a short oligo- or polypeptide linker, for example, between 2 and 10 amino acids in length may form the linkage. A glycine-serine doublet provides a suitable linker in some embodiments.

In one embodiment, the intracellular domain is designed to comprise the signaling domain of CD3-zeta and the signaling domain of CD28. In yet another embodiment, the intracellular domain is designed to comprise the signaling domain of CD3-zeta and the signaling domain of 4- IBB.

The antigen binding domain can be any domain that binds to the antigen including but not limited to monoclonal antibodies, polyclonal antibodies, synthetic antibodies, scFvs, human antibodies, humanized antibodies, and fragments thereof. In one non-limiting embodiment, the antigen binding region specifically binds to a selected target, e.g., an activated fibroblast cell surface receptor (such as CD90, FAP, FSP-1, CD140a, CD140b, CD49b, CD87, CD95, α smooth muscle actin (αSMA), or platelet derived growth factor β (PDGFR β.

In various embodiments, the CAR can be a "first generation," "second generation," "third generation," "fourth generation" or "fifth generation" CAR (see, for example, Sadelain et al., Cancer Discov. 3(4):388-398 (2013); Jensen et al., Immunol. Rev. 257:127-133 (2014): Sharpe et al., Dis. Model Mech. 8(4):337-350 (2015); Brentjens et al., Clin. Cancer Res. 13:5426-5435 (2007); Gade et al., Cancer Res. 65:9080-9088 (2005): Maher et al., Nat. Biotechnol. 20:70-75 (2002); Kershaw et al., J. Immunol. 173:2143-2150 (2004): Sadelain et al., Curr. Opin. Immunol. (2009); Hollyman et al., J. Immunother. 32:169-180 (2009), each of which are incorporated by reference in its entirety).

"First generation" CARs for use in the invention comprise an antigen binding domain, for example, a single-chain variable fragment (scFv), fused to a transmembrane domain, which is fused to a cytoplasmic/intracellular domain of the T cell receptor chain. "First generation" CARs typically have the intracellular domain from the CD3ζ-chain, which is the primary transmitter of signals from endogenous T cell receptors (TCRs). "First generation" CARs can provide de novo antigen recognition and cause activation of both CD4+ and CD8+ T cells through their CD3ζ chain signaling domain in a single fusion molecule, independent of HLA-mediated antigen presentation.

"Second-generation" CARs for use in the invention comprise an antigen binding domain, for example, a single-chain variable fragment (scFv), fused to an intracellular signaling domain capable of activating T cells and a co-stimulatory domain designed to augment T cell potency and persistence (Sadelain et al., Cancer Discov. 3:388-398 (2013)). CAR design can therefore combine antigen recognition with signal transduction, two functions that are physiologically borne by two separate complexes, the TCR heterodimer and the CD3 complex. "Second generation" CARs include an intracellular domain from various co-stimulatory molecules, for example, CD28, 4-1BB, ICOS, OX40, and the like, in the cytoplasmic tail of the CAR to provide additional signals to the cell.

"Second generation" CARs provide both co-stimulation, for example, by CD28 or 4-1BB domains, and activation, for example, by a CD3ζ signaling domain. Preclinical studies have indicated that "Second Generation" CARs can improve the anti-tumor activity of T cells. For example, robust efficacy of "Second Generation" CAR modified T cells was demonstrated in clinical trials targeting the CD19 molecule in patients with chronic lymphoblastic leukemia (CLL) and acute lymphoblastic leukemia (ALL) (Davila et al., Oncoimmunol. 1(9):1577-1583 (2012)).

"Third generation" CARs provide multiple co-stimulation, for example, by comprising both CD28 and 4-1BB domains, and activation, for example, by comprising a CD3ζ activation domain.

"Fourth generation" CARs provide co-stimulation, for example, by CD28 or 4-1BB domains, and activation, for example, by a CD3ζ signaling domain in addition to a constitutive or inducible chemokine component.

"Fifth generation" CARs provide co-stimulation, for example, by CD28 or 4-1BB domains, and activation, for example, by a CD3ζ signaling domain, a constitutive or inducible chemokine component, and an intracellular domain of a cytokine receptor, for example, IL-2Rβ.

In various embodiments, the CAR can be included in a multivalent CAR system, for example, a DualCAR or "TandemCAR" system. Multivalent CAR systems include systems or cells comprising multiple CARs and systems or cells comprising bivalent/bispecific CARs targeting more than one antigen.

In the embodiments disclosed herein, the CARs generally comprise an antigen binding domain, a transmembrane domain and an intracellular domain, as described above. In a particular non-limiting embodiment, the antigen-binding domain is an scFv.

In one embodiment, the antigen binding domain is a targeting domain, wherein the targeting domain directs the T cell expressing the CAR to a specific cell or tissue of interest. For example, in one embodiment, the targeting domain comprises an antibody, antibody fragment, or peptide that specifically binds to an antigen (e.g., a salef-antigen or a foreign antigen) thereby directing the T cell expressing the CAR to a cell or tissue expressing the antigen.

The antigen binding domain of the CAR molecule of the invention can be generated to be reactive to any desirable antigen of interest, or fragment thereof, including, but not limited to a tumor antigen, a foreign antigen (e.g, a bacterial antigen, or a viral antigen) or a self-antigen.

Tumor antigens are proteins that are produced by tumor cells that elicit an immune response. The selection of the antigen binding domain of the VM-domain containing fusion molecule of the invention will depend on the particular type of cancer to be treated. Tumor antigens are well known in the art and include, for example, a glioma-associated antigen, carcinoembryonic antigen (CEA), β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostate-specific antigen (PSA), PAP, NY-ESO-1, LAGE-1a, p53, prostein, PSMA, Her2/neu, survivin and telomerase, prostate-carcinoma tumor antigen-1 (PCTA-1), MAGE, ELF2M, neutrophil elastase, ephrinB2, CD22, insulin growth factor (IGF)-I, IGF-II, IGF-I receptor and mesothelin. Another exemplary tumor antigen is chondroitin sulfate proteoglycan 4 (CSPG4) (also referred to as melanoma-associated chondroitin sulfate proteoglycan (MCSP), high-molecular-weight melanoma-associated antigen (HMW-MAA), or neuron-glial antigen 2 (NG2)).

In one embodiment, the tumor antigen comprises one or more antigenic cancer epitopes associated with a malignant tumor. Malignant tumors express a number of proteins that can serve as target antigens for an immune attack. These molecules include but are not limited to tissue-specific antigens such as MART-1, tyrosinase and GP 100 in melanoma and prostatic acid phosphatase (PAP) and prostate-specific antigen (PSA) in prostate cancer. Other target molecules belong to the group of transformation-related molecules such as the oncogene HER-2/Neu/ErbB-2. Yet another group of target antigens are onco-fetal antigens such as carcinoembryonic antigen (CEA). In B-cell lymphoma the tumor-specific idiotype immunoglobulin constitutes a truly tumor-specific immunoglobulin antigen that is unique to the individual tumor. B-cell differentiation antigens such as CD 19, CD20 and CD37 are other candidates for target antigens in B-cell lymphoma. Some of these antigens (CEA, HER-2, CD19, CD20, idiotype) have been used as targets for passive immunotherapy with monoclonal antibodies with limited success.

The type of tumor antigen referred to in the invention may also be a tumor-specific antigen (TSA) or a tumor-associated antigen (TAA). A TSA is unique to tumor cells and does not occur on other cells in the body. A TAA associated antigen is not unique to a tumor cell and instead is also expressed on a normal cell under conditions that fail to induce a state of immunologic tolerance to the antigen. The expression of the antigen on the tumor may occur under conditions that enable the immune system to respond to the antigen. TAAs may be antigens that are expressed on normal cells during fetal development when the immune system is immature and unable to respond or they may be antigens that are normally present at extremely low levels on normal cells but which are expressed at much higher levels on tumor cells.

Non-limiting examples of TSA or TAA antigens include the following: differentiation antigens such as MART-1/MelanA (MART-I), gp100 (Pmel 17), tyrosinase, TRP-1, TRP-2 and tumor-specific multilineage antigens such as MAGE-1, MAGE-3, BAGE, GAGE-1, GAGE-2, p15; overexpressed embryonic antigens such as CEA; overexpressed oncogenes and mutated tumor-suppressor genes such as p53, Ras, HER-2/neu; unique tumor antigens resulting from chromosomal translocations: such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCAS1, SDCCAG16, TA-90\Mac-2 binding protein\cyclophilin C-associated protein, TAAL6, TAG72, TLP, and TPS.

A foreign antigen can be a viral antigen, a bacterial antigen, a fungal antigen, a parasitic antigen or fragment thereof, or variant thereof. Exemplary viruses, bacterium, fungi and parasites that can be targeted using the compositions and methods of the invention are discussed elsewhere herein.

### Bispecific T-cell engager (e.g., BiTE) agents

In still another embodiment, the nucleic acid cargo molecule (e.g., mRNA, expression vector, CRISPR genome editing system, or nucleoside modified mRNA molecule) of the disclosure may encode a bispecific T-cell engager that specifically binds to both an antigen on an immune cell (e.g., a CD4+ T cell) and an antigen on a cell of interest, e.g., an pathogen.

Bispecific T-cell engagers are bispecific molecules that are created by linking the targeting regions (i.e., antigen binding domains) of two antibodies as a single molecule. One arm of the molecule is engineered to bind with a protein found on the surface of CD4+ T cells, and the other arm is designed to bind to a specific protein found primarily on a target cell. When both targets are engaged, the bispecific T-cell engager (i.e., a BiTE molecule) forms a bridge between the CD4+ T cell and the target cell. For example, in one embodiment, a target cell is an activated fibroblast and the BiTE molecule comprises a binding arm specific for binding to a fibroblast-specific marker. Fibroblast-specific markers, include, but are not limited to, CD90, FAP, FSP-1, CD140a, CD140b, CD49b, CD87, CD95, α smooth muscle actin (αSMA), and platelet derived growth factor β (PDGFR β). Further reference may be made to Diego Ellerman, "Bispecific T-cell engagers: Towards understanding variable influencing the in vitro potency and tumor selectivity and their modulation to enhance their efficacy and safety," Methods, Vol.154, Feb. 2019, pp.102-117. which is incorporated herein by reference.

The term "bispecific" means that the bispecific molecule (e.g., a bispecific T-cell engager) is able to specifically bind to at least two distinct antigenic determinants (e.g., one from a CD4+ T cell and another from a target cell, such as a pathogen). Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The present disclosure is not limited to the BiTE format but contemplates the use of any suitable bispecific format suitable for T cell redirection, including diabodies (Holliger et al, Prot Eng 9, 299-305 (1996)) and derivatives thereof, such as tandem diabodies (Kipriyanov et al, J Mol Biol 293, 41-66 (1999)), DART (dual affinity retargeting) molecules, which are based on the diabody format but feature a C-terminal disulfide bridge for additional stabilization (Moore et al, Blood 117, 4542-51 (2011)), and triomabs, which are whole hybrid mouse/rat IgG molecules and also currently being evaluated in clinical trials, represent a larger sized format (reviewed in Seimetz et al, Cancer Treat Rev 36, 458-467 (2010)). Each of the aforementioned references are incorporated herein by reference.

Methods for making bispecific antibodies are known in the art. (See, e.g., Millstein et al., Nature, 305:537-539 (1983); Traunecker et al., EMBOJ., 10:3655-3659 (1991); Suresh et al., Methods in Enzymology, 121:210 (1986); Kostelny et al., J. Immunol. 148(5):1547-1553 (1992); Hollinger et al., PNAS USA, 90:6444-6448 (1993): Gruber et al., J. Immunol. 152:5368 (1994); U.S. Pat. Nos. 4,474,893; 4,714,681; 4,925,648: 5,573,920: 5,601,81; 95,731,168; 4,676,980; and 4,676,980, WO 94/04690; WO 91/00360; WO 92/200373: WO 93/17715; WO 92/08802; and EP 03089.) Each of these aforementioned references relating to making bispecific antibodies, including BiTEs, are incorporated herein by reference.

Exemplary bispecific antibody molecules useful in practicing the methods described herein contain (i) two antibodies, a first antibody with a binding specificity to an antigen expressed on the surface of a target cell and a second antibody with a binding specificity for an antigen expressed on the surface of an immune cell (e.g., a CD4+ T cell), (ii) a single antibody that has one chain or arm with a binding specificity to an antigen expressed on the surface of a target cell and a second chain or arm with a binding specificity to an immune cell (e.g., a CD4+ T cell), (iii) a single chain antibody that has binding specificity to an antigen expressed on the surface of a target cell and also binding specificity to an immune cell (e.g., a CD4+ T cell), e.g., via two scFvs linked in tandem by an extra peptide linker; (iv) a dual-variable-domain antibody (DVD-Ig), where each light chain and heavy chain contains two variable domains in tandem through a short peptide linkage: (v) a chemically-linked bispecific (Fab')2 fragment; (vi) a Tandab, which is a fusion of two single chain diabodies resulting in a tetravalent bispecific antibody that has two binding sites for each of the target antigens; (vii) a flexibody (a combination of scFvs with a diabody resulting in a multivalent molecule); (viii) a so called "dock and lock" molecule (an adaptation of the "dimerization and docking domain" in Protein Kinase A, that can be applied to Fabs to generate a trivalent bispecific binding protein containing two identical Fab fragments linked to a different Fab fragment; (ix) a so-called "Scorpion" molecule, containing for example, two scFvs fused to both termini of a human Fc-region: (x) a diabody: and (xi) a so-called "ImmTAC" molecule (Immune mobilising mTCR Against Cancer: see e.g., Liddy et al., Nat. Med. 18:980-987 (2012)).

### Imaging Agents

In one embodiment, the delivery vehicle comprises an imaging agent. Imaging agents are materials that allow the delivery vehicle to be visualized after exposure to a cell or tissue. Visualization includes imaging for the naked eye, as well as imaging that requires detecting with instruments or detecting information not normally visible to the eye, and includes imaging that requires detecting of photons, sound or other energy quanta. Examples include stains, vital dyes, fluorescent markers, radioactive markers, enzymes or plasmid constructs encoding markers or enzymes. Many materials and methods for imaging and targeting that may be used in the delivery vehicle are provided in the Handbook of Targeted delivery of Imaging Agents, Torchilin, ed. (1995) CRC Press, Boca Raton, Fla.

Visualization based on molecular imaging typically involves detecting biological processes or biological molecules at a tissue, cell, or molecular level. Molecular imaging can be used to assess specific targets for gene therapies, cell-based therapies, and to visualize pathological conditions as a diagnostic or research tool. Imaging agents that are able to be delivered intracellularly are particularly useful because such agents can be used to assess intracellular activities or conditions. Imaging agents must reach their targets to be effective; thus, in some embodiments, an efficient uptake by cells is desirable. A rapid uptake may also be desirable to avoid the RES, see review in Allport and Weissleder, Experimental Hematology 1237-1246 (2001).

Further, imaging agents preferably should provide high signal to noise ratios so that they may be detected in small quantities, whether directly, or by effective amplification techniques that increase the signal associated with a particular target. Amplification strategies are reviewed in Allport and Weissleder, Experimental Hematology 1237-1246 (2001), and include, for example, avidin-biotin binding systems, trapping of converted ligands, probes that change physical behavior after being bound by a target, and taking advantage of relaxation rates. Examples of imaging technologies include magnetic resonance imaging, radionuclide imaging, computed tomography, ultrasound, and optical imaging.

Delivery vehicles as set forth herein may advantageously be used in various imaging technologies or strategies, for example by incorporating imaging agents into delivery vehicles. Many imaging techniques and strategies are known, e.g., see review in Allport and Weissleder, Experimental Hematology 1237-1246 (2001); such strategies may be adapted to use with delivery vehicles. Suitable imaging agents include, for example, fluorescent molecules, labeled antibodies, labeled avidin:biotin binding agents, colloidal metals (e.g., gold, silver), reporter enzymes (e.g., horseradish peroxidase), superparamagnetic transferrin, second reporter systems (e.g., tyrosinase), and paramagnetic chelates.

In some embodiments, the imaging agent is a magnetic resonance imaging contrast agent. Examples of magnetic resonance imaging contrast agents include, but are not limited to, 1,4,7,10-tetraazacyclododecane-N,N',N"N‴-tetracetic acid (DOTA), diethylenetriaminepentaacetic (DTPA), 1,4,7,10-tetraazacyclododecane-N,N', N",N‴-tetraethylphosphorus (DOTEP), 1,4,7,10-tetraazacyclododecane-N,N',N"-triacetic acid (DOTA) and derivatives thereof (see U.S. Pat. Nos. 5,188,816, 5,219,553, and 5,358,704). In some embodiments, the imaging agent is an X-Ray contrast agent. X-ray contrast agents already known in the art include a number of halogenated derivatives, especially iodinated derivatives, of 5-amino-isophthalic acid.

### Small molecule agents

In various embodiments, the agent is a small molecule. When the agent is a small molecule, a small molecule may be obtained using standard methods known to the skilled artisan. Such methods include chemical organic synthesis or biological means. Biological means include purification from a biological source, recombinant synthesis and in vitro translation systems, using methods well known in the art. In one embodiment, a small molecule agents comprises an organic molecule, inorganic molecule, biomolecule, synthetic molecule, and the like.

Combinatorial libraries of molecularly diverse chemical compounds potentially useful in treating a variety of diseases and conditions are well known in the art, as are method of making the libraries. The method may use a variety of techniques well-known to the skilled artisan including solid phase synthesis, solution methods, parallel synthesis of single compounds, synthesis of chemical mixtures, rigid core structures, flexible linear sequences, deconvolution strategies, tagging techniques, and generating unbiased molecular landscapes for lead discovery vs. biased structures for lead development. In some embodiments of the invention, the agent is synthesized and/or identified using combinatorial techniques.

In a general method for small library synthesis, an activated core molecule is condensed with a number of building blocks, resulting in a combinatorial library of covalently linked, core-building block ensembles. The shape and rigidity of the core determines the orientation of the building blocks in shape space. The libraries can be biased by changing the core, linkage, or building blocks to target a characterized biological structure ("focused libraries") or synthesized with less structural bias using flexible cores. In some embodiments of the invention, the agent is synthesized via small library synthesis.

The small molecule and small molecule compounds described herein may be present as salts even if salts are not depicted, and it is understood that the invention embraces all salts and solvates of the agents depicted here, as well as the non-salt and non-solvate form of the agents, as is well understood by the skilled artisan. In some embodiments, the salts of the agents of the invention are pharmaceutically acceptable salts.

Where tautomeric forms may be present for any of the agents described herein, each and every tautomeric form is intended to be included in the present invention, even though only one or some of the tautomeric forms may be explicitly depicted. For example, when a 2-hydroxypyridyl moiety is depicted, the corresponding 2-pyridone tautomer is also intended.

The invention also includes any or all of the stereochemical forms, including any enantiomeric or diastereomeric forms of the agents described. The recitation of the structure or name herein is intended to embrace all possible stereoisomers of agents depicted. All forms of the agents are also embraced by the invention, such as crystalline or non-crystalline forms of the agent. Compositions comprising an agent of the invention are also intended, such as a composition of substantially pure agent, including a specific stereochemical form thereof, or a composition comprising mixtures of agents of the invention in any ratio, including two or more stereochemical forms, such as in a racemic or non-racemic mixture.

The invention also includes any or all active analog or derivative, such as a prodrug, of any agent described herein. In one embodiment, the agent is a prodrug. In one embodiment, the small molecules described herein are candidates for derivatization. As such, in certain instances, the analogs of the small molecules described herein that have modulated potency, selectivity, and solubility are included herein and provide useful leads for drug discovery and drug development. Thus, in certain instances, during optimization new analogs are designed considering issues of drug delivery, metabolism, novelty, and safety.

In some instances, small molecule agents described herein are derivatives or analogs of known agents, as is well known in the art of combinatorial and medicinal chemistry. The analogs or derivatives can be prepared by adding and/or substituting functional groups at various locations. As such, the small molecules described herein can be converted into derivatives/analogs using well known chemical synthesis procedures. For example, all of the hydrogen atoms or substituents can be selectively modified to generate new analogs. Also, the linking atoms or groups can be modified into longer or shorter linkers with carbon backbones or hetero atoms. Also, the ring groups can be changed so as to have a different number of atoms in the ring and/or to include hetero atoms. Moreover, aromatics can be converted to cyclic rings, and vice versa. For example, the rings may be from 5-7 atoms, and may be carbocyclic or heterocyclic.

As used herein, the term "analog," "analogue," or "derivative" is meant to refer to a chemical compound or molecule made from a parent compound or molecule by one or more chemical reactions. As such, an analog can be a structure having a structure similar to that of the small molecule agents described herein or can be based on a scaffold of a small molecule agents described herein, but differing from it in respect to certain components or structural makeup, which may have a similar or opposite action metabolically. An analog or derivative of any of a small molecule inhibitor in accordance with the present invention can be used to treat a disease or disorder.

In one embodiment, the small molecule agents described herein can independently be derivatized, or analogs prepared therefrom, by modifying hydrogen groups independently from each other into other substituents. That is, each atom on each molecule can be independently modified with respect to the other atoms on the same molecule. Any traditional modification for producing a derivative/analog can be used. For example, the atoms and substituents can be independently comprised of hydrogen, an alkyl, aliphatic, straight chain aliphatic, aliphatic having a chain hetero atom, branched aliphatic, substituted aliphatic, cyclic aliphatic, heterocyclic aliphatic having one or more hetero atoms, aromatic, heteroaromatic, polyaromatic, polyamino acids, peptides, polypeptides, combinations thereof, halogens, halo-substituted aliphatics, and the like. Additionally, any ring group on a compound can be derivatized to increase and/or decrease ring size as well as change the backbone atoms to carbon atoms or hetero atoms.

### Delivery Vehicle

In some embodiments, the invention relates to composition comprising delivery vehicles for delivery of one or more agent. In some embodiments, the agent comprises an mRNA molecule (e.g., a nucleoside modified mRNA molecule) of the invention.

In some embodiments, the delivery vehicle is a colloidal dispersion system, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

The use of lipid formulations is contemplated for the introduction of the at least one agent into a host cell (in vitro, ex vivo or in vivo). In another aspect, the at least one agent may be associated with a lipid. The at least one agent associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/nucleic acid or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

### Lipids and their derivatives

In various embodiments, the delivery vehicle may comprise lipids or a derivative thereof.

Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, aldehydes, and polymers (e.g. PEGylated lipids).

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Chol") can be obtained from Calbiochem-Behring: dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol.

In some embodiments, cationic lipids are preferred. In certain embodiments, the cationic lipid comprises any of a number of lipid species which carry a net positive charge at a selective pH, such as physiological pH. Such lipids include, but are not limited to, N,N-dioleyl-N,N-dimethylammonium chloride (DODAC); N-(2,3-dioleyloxy)propyl)-N,N,N-trimethylammonium chloride (DOTMA): N,N-distearyl-N,N-dimethylammonium bromide (DDAB); N-(2,3-dioleoyloxy)propyl)-N,N,N-trimethylammonium chloride (DOTAP); 3-(N-(N',N'-dimethylaminoethane)-carbamoyl)cholesterol (DC-Chol), N-(1-(2,3-dioleoyloxy)propyl)-N-2-(sperminecarboxamido)ethyl)-N,N-dimethylammonium trifluoracetate (DOSPA), dioctadecylamidoglycyl carboxyspermine (DOGS), 1,2-dioleoyl-3-dimethylammonium propane (DODAP), N,N-dimethyl-2,3-dioleoyloxy)propylamine (DODMA), and N-(1,2-dimyristyloxyprop-3-yl)-N,N-dimethyl-N-hydroxyethyvl ammonium bromide (DMRIE). Additionally, a number of commercial preparations of cationic lipids are available which can be used in the present invention. These include, for example, LIPOFECTIN^{®} (commercially available cationic liposomes comprising DOTMA and 1,2-dioleoyl-sn-3-phosphoethanolamine (DOPE), from GIBCO/BRL, Grand Island, N.Y.): LIPOFECTAMINE^{®} (commercially available cationic liposomes comprising N-(1-(2,3-dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.). The following lipids are cationic and have a positive charge at below physiological pH: DODAP, DODMA, DMDMA, 1,2-dilinoleyloxy-N,N-dimethylaminopropane (DLinDMA), 1,2-dilinolenyloxy-N,N-dimethylaminopropane (DLenDMA).

In one embodiment, the cationic lipid is an amino lipid. Suitable amino lipids useful in the invention include those described in WO 2012/016184, incorporated herein by reference in its entirety. Representative amino lipids include, but are not limited to, 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA).

Suitable amino lipids include those having the formula: 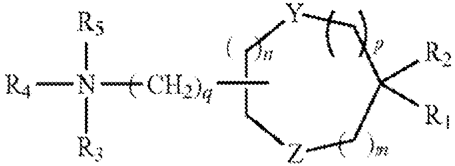
wherein R₁ and R₂ are either the same or different and independently optionally substituted C₁₀-C₂₄ alkyl, optionally substituted C₁₀-C₂₄ alkenyl, optionally substituted C₁₀-C₂₄ alkynyl, or optionally substituted C₁₀-C₂₄ acyl;
R₃ and R₄ are either the same or different and independently optionally substituted C₁-C₆ alkyl, optionally substituted C₂-C₆ alkenyl, or optionally substituted C₂-C₆ alkynyl or R₃ and R₄ may join to form an optionally substituted heterocyclic ring of 4 to 6 carbon atoms and 1 or 2 heteroatoms chosen from nitrogen and oxygen:
R₅ is either absent or present and when present is hydrogen or C₁-C₆ alkyl;
m, n, and p are either the same or different and independently either 0 or 1 with the proviso that m, n, and p are not simultaneously 0:
q is 0, 1, 2, 3, or 4; and
Y and Z are either the same or different and independently O, S, or NH.
In one embodiment, R₁ and R₂ are each linoleyl, and the amino lipid is a dilinoleyl amino lipid. In one embodiment, the amino lipid is a dilinoleyl amino lipid.

A representative useful dilinoleyl amino lipid has the formula: 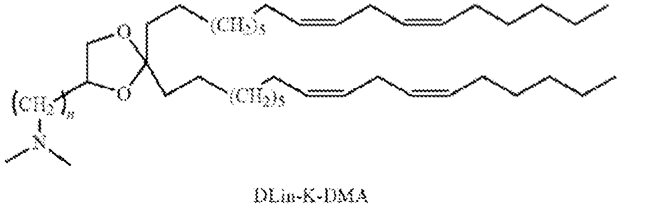 wherein n is 0, 1, 2, 3, or 4.

In one embodiment, the cationic lipid is a DLin-K-DMA. In one embodiment, the cationic lipid is DLin-KC2-DMA (DLin-K-DMA above, wherein n is 2).

In one embodiment, the cationic lipid component of the LNPs has the structure of Formula (I): 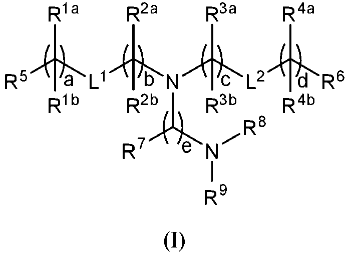 or a pharmaceutically acceptable salt, tautomer, prodrug or stereoisomer thereof, wherein:
L¹ and L² are each independently -O(C=O)-, -(C=O)O- or a carbon-carbon double bond;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently methyl or cycloalkyl;
R⁷ is, at each occurrence, independently H or C₁-C₁₂ alkyl;
R⁸ and R⁹ are each independently C₁-C₁₂ alkyl; or R⁸ and R⁹, together with the nitrogen atom to which they are attached, form a 5, 6 or 7-membered heterocyclic ring comprising one nitrogen atom:
a and d are each independently an integer from 0 to 24;
b and c are each independently an integer from 1 to 24; and
e is 1 or 2.

In certain embodiments of Formula (I), at least one of R^{1a}, R^{2a}, R^{3a} or R^{4a} is C₁-C₁₂ alkyl, or at least one of L¹ or L² is -O(C=O)- or -(C=O)O-. In other embodiments, R^{1a} and R^{1b} are not isopropyl when a is 6 or n-butyl when a is 8.

In still further embodiments of Formula (I), at least one of R^{1a}, R^{2a}, R^{3a} or R^{4a} is C₁-C₁₂ alkyl, or at least one of L¹ or L² is -O(C=O)- or -(C=O)O-: and
R^{1a} and R^{1b} are not isopropyl when a is 6 or n-butyl when a is 8.

In other embodiments of Formula (I), R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom:
In certain embodiments of Formula (I), any one of L¹ or L² may be -O(C=O)- or a carbon-carbon double bond. L¹ and L² may each be -O(C=O)- or may each be a carbon-carbon double bond.

In some embodiments of Formula (I), one of L¹ or L² is -O(C=O)-. In other embodiments, both L¹ and L² are -O(C=O)-.

In some embodiments of Formula (I), one of L¹ or L² is -(C=O)O-. In other embodiments, both L¹ and L² are -(C=O)O-.

In some other embodiments of Formula (I), one of L¹ or L² is a carbon-carbon double bond. In other embodiments, both L¹ and L² are a carbon-carbon double bond.

In still other embodiments of Formula (I), one of L¹ or L² is -O(C=O)- and the other of L¹ or L² is -(C=O)O-. In more embodiments, one of L¹ or L² is -O(C=O)- and the other of L¹ or L² is a carbon-carbon double bond. In yet more embodiments, one of L¹ or L² is -(C=O)O- and the other of L¹ or L² is a carbon-carbon double bond.

It is understood that "carbon-carbon" double bond, as used throughout the specification, refers to one of the following structures: 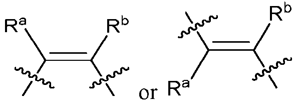 wherein R^{a} and R^{b} are, at each occurrence, independently H or a substituent. For example, in some embodiments R^{a} and R^{b} are, at each occurrence, independently H, C₁-C₁₂ alkyl or cycloalkyl, for example H or C₁-C₁₂ alkyl.

In other embodiments, the lipid compounds of Formula (I) have the following structure (Ia):

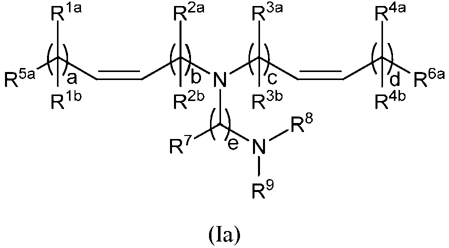

In other embodiments, the lipid compounds of Formula (I) have the following structure (Ib):

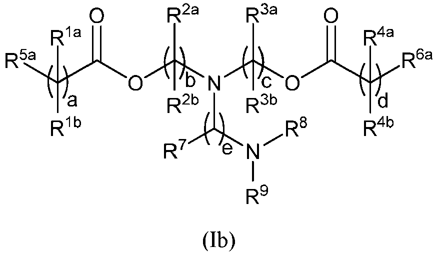

In yet other embodiments, the lipid compounds of Formula (I) have the following structure (Ic):

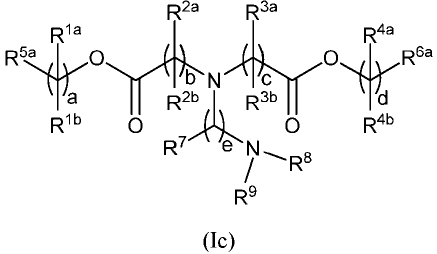

In certain embodiments of the lipid compound of Formula (I), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some other embodiments of Formula (I), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some more embodiments of Formula (I), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain other embodiments of Formula (I), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some other various embodiments of Formula (I), a and d are the same. In some other embodiments, b and c are the same. In some other specific embodiments, a and d are the same and b and c are the same.

The sum of a and b and the sum of c and d in Formula (I) are factors which may be varied to obtain a lipid of Formula (I) having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such the sum of a and b and the sum of c and d is 12 or greater.

In some embodiments of Formula (I), e is 1. In other embodiments, e is 2.

The substituents at R^{1a}, R^{2a}, R^{3a} and R^{4a} of Formula (I) are not particularly limited. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is C₁-C₈ alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (I), R^{1a}, R^{1b}, R^{4a} and R^{4b} are C₁-C₁₂ alkyl at each occurrence.

In further embodiments of Formula (I), at least one of R^{1b}, R^{2b}, R^{3b} and R^{4b} is H or R^{1b}, R^{2b}, R^{3b} and R^{4b} are H at each occurrence.

In certain embodiments of Formula (I), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (I) are not particularly limited in the foregoing embodiments. In certain embodiments one or both of R⁵ or R⁶ is methyl. In certain other embodiments one or both of R⁵ or R⁶ is cycloalkyl for example cyclohexyl. In these embodiments the cycloalkyl may be substituted or not substituted. In certain other embodiments the cycloalkyl is substituted with C₁-C₁₂ alkyl, for example tert-butyl.

The substituents at R⁷ are not particularly limited in the foregoing embodiments of Formula (I). In certain embodiments at least one R⁷ is H. In some other embodiments, R⁷ is H at each occurrence. In certain other embodiments R⁷ is C₁-C₁₂ alkyl.

In certain other of the foregoing embodiments of Formula (I), one of R⁸ or R⁹ is methyl. In other embodiments, both R⁸ and R⁹ are methyl.

In some different embodiments of Formula (I), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring.

In various different embodiments, exemplary lipid of Formula (I) can include

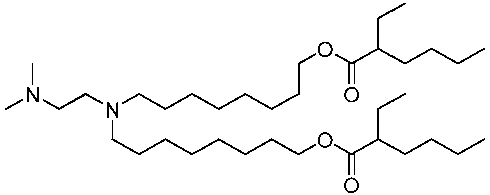

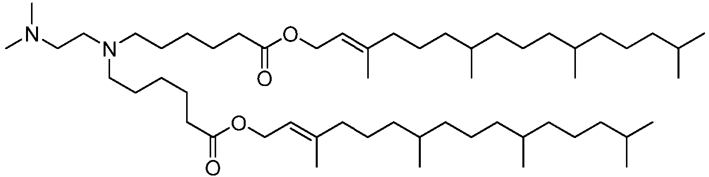

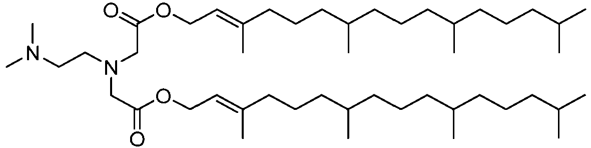

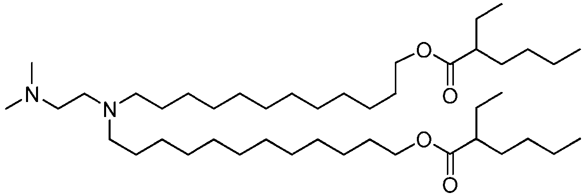

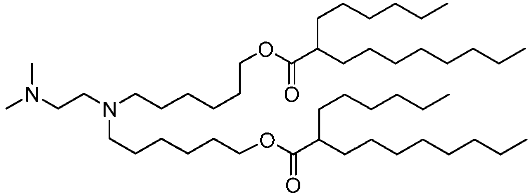

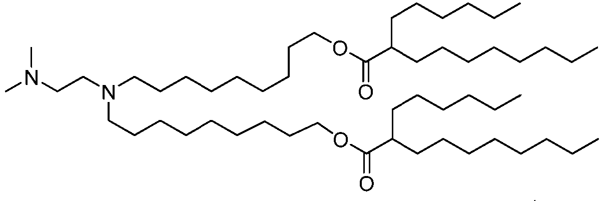

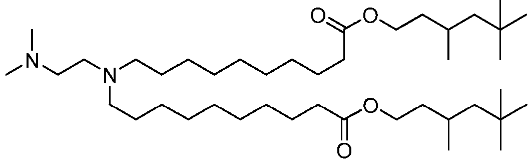

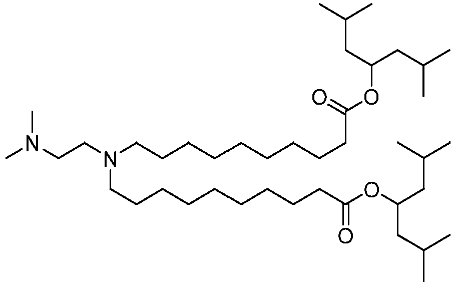

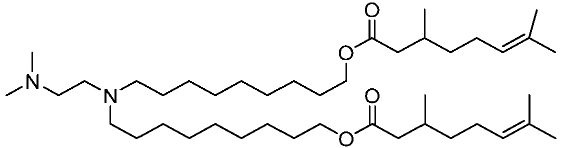

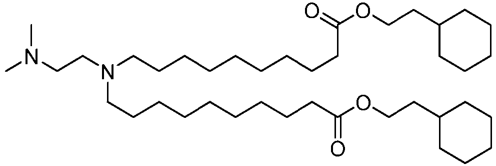

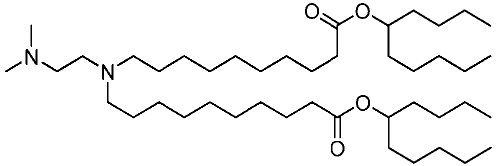

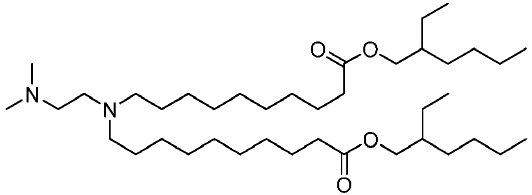

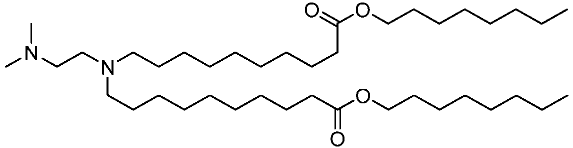

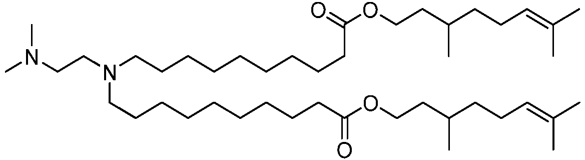

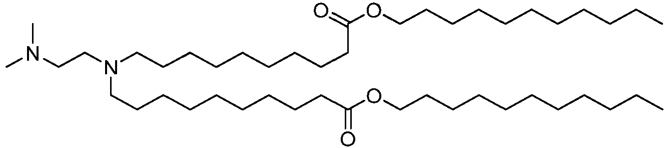

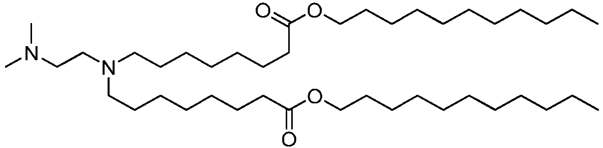

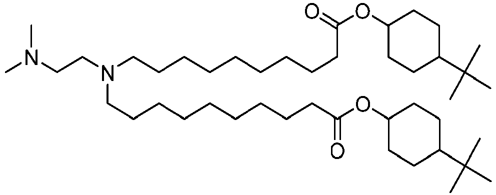

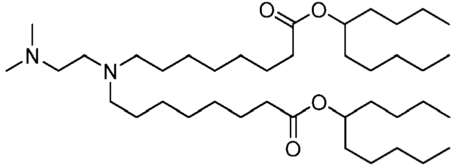

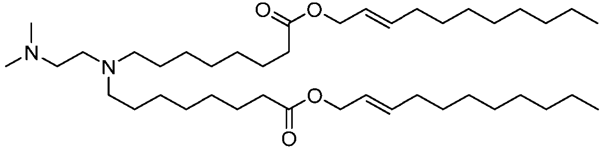

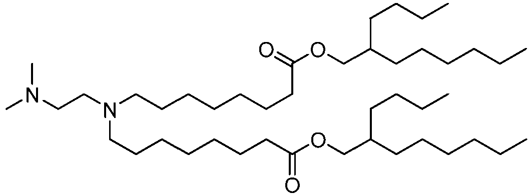

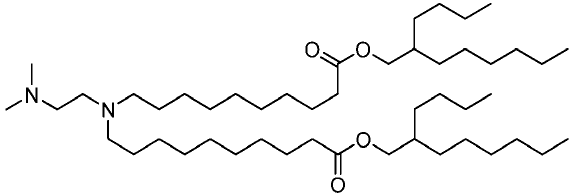

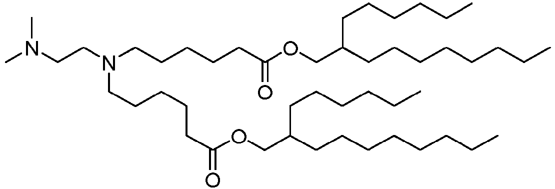

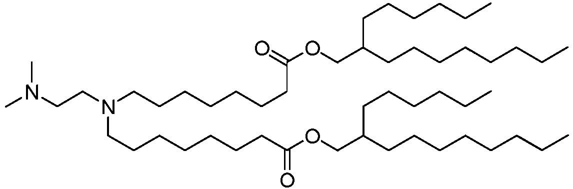

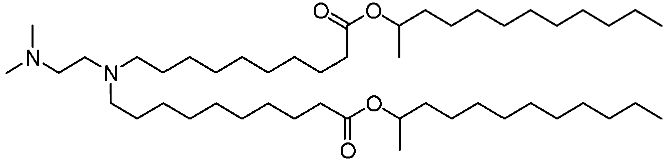

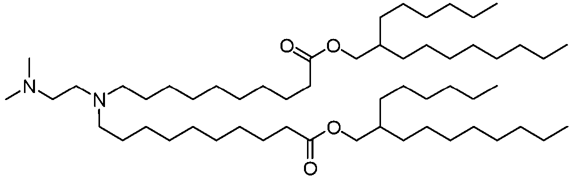

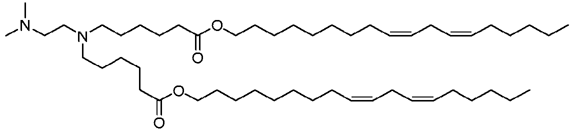

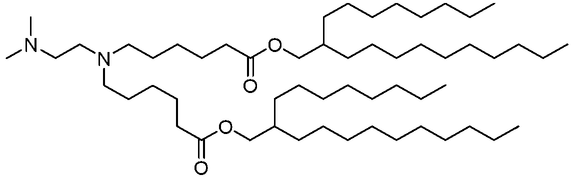

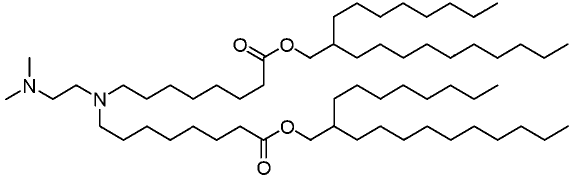

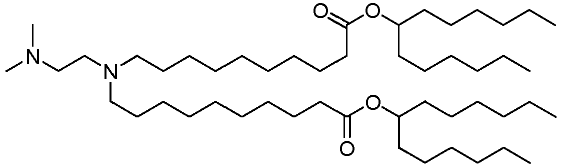

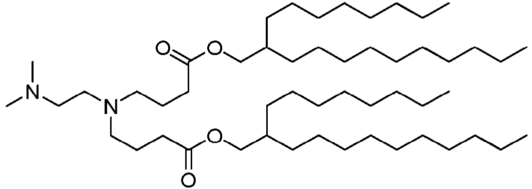

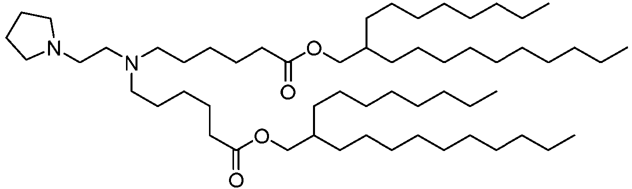

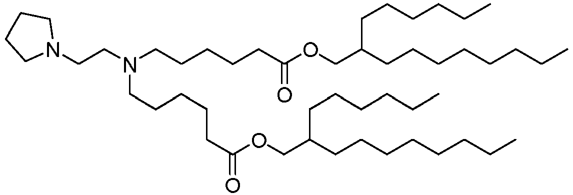

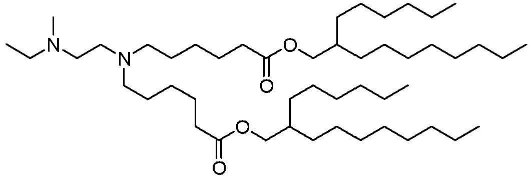

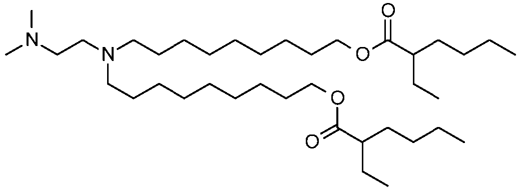

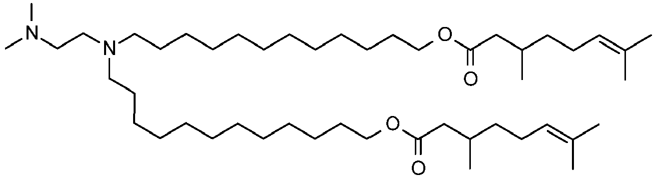

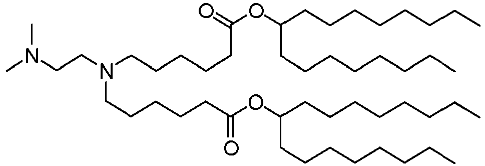

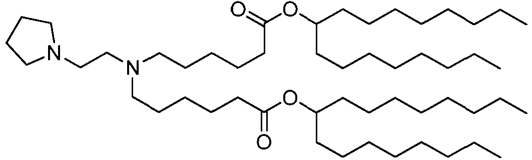

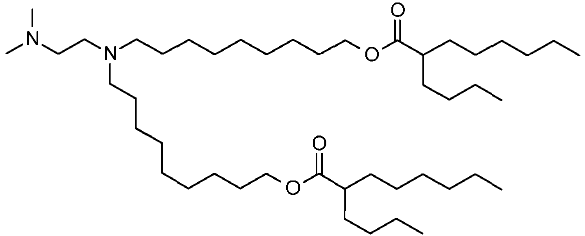

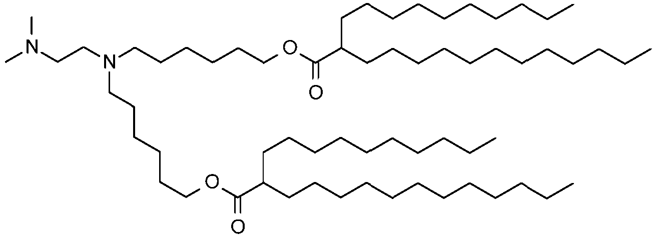

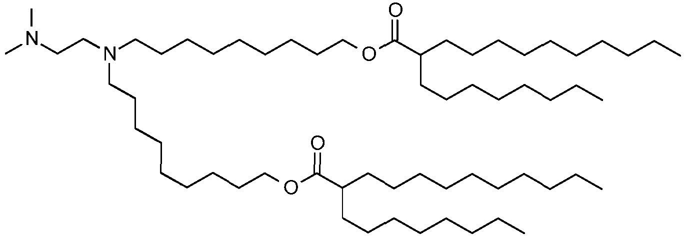

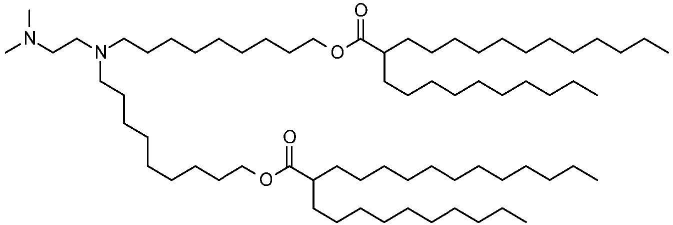

In some embodiments, the LNPs comprise a lipid of Formula (I), at least one agent, and one or more excipients selected from neutral lipids, steroids and pegylated lipids. In some embodiments the lipid of Formula (I) is compound I-5. In some embodiments the lipid of Formula (I) is compound I-6.

In some other embodiments, the cationic lipid component of the LNPs has the structure of Formula (II): 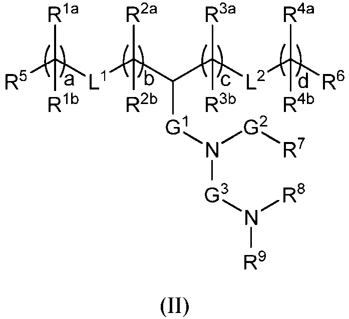 or a pharmaceutically acceptable salt, tautomer, prodrug or stereoisomer thereof, wherein:
L¹ and L² are each independently -O(C=O)-, -(C=O)O-, -C(=O)-, -O-,
-S(O)ₓ-, -S-S-, -C(=O)S-, -SC(=O)-, -NR^{a}C(=O)-, -C(=O)NR^{a}-, -NR^{a}C(=O)NR^{a}, -OC(=O)NR^{a}-, -NR^{a}C(=O)O-, or a direct bond:
G¹ is C₁-C₂ alkylene, -(C=O)- , -O(C=O)-, -SC(=O)-, -NR^{a}C(=O)- or a direct bond;
G² is -C(=O)- , -(C=O)O-, -C(=O)S-, -C(=O)NR^{a} or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either: (a) H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl: or R⁸ and R⁹, together with the nitrogen atom to which they are attached, form a 5, 6 or 7-membered heterocyclic ring:
a, b, c and d are each independently an integer from 1 to 24; and
x is 0, 1 or 2.

In some embodiments of Formula (II), L¹ and L² are each independently
-O(C=O)-, -(C=O)O- or a direct bond. In other embodiments, G¹ and G² are each independently -(C=O)- or a direct bond. In some different embodiments, L¹ and L² are each independently -O(C=O)-, -(C=O)O- or a direct bond: and G¹ and G² are each independently - (C=O)- or a direct bond.

In some different embodiments of Formula (II), L¹ and L² are each independently -C(=O)-, -O-, -S(O)ₓ-, -S-S-, -C(=O)S-, -SC(=O)-, -NR^{a}-, -NR^{a}C(=O)-,
-C(=O)NR^{a}-, -NR^{a}C(=O)NR^{a}, -OC(=O)NR^{a}-. -NR^{a}C(=O)O-, -NR^{a}S(O)ₓNR^{a}-,
-NR^{a}S(O)ₓ₋ or -S(O)ₓNR^{a}-.

In other of the foregoing embodiments of Formula (II), the lipid compound has one of the following structures (IIA) or (IIB):

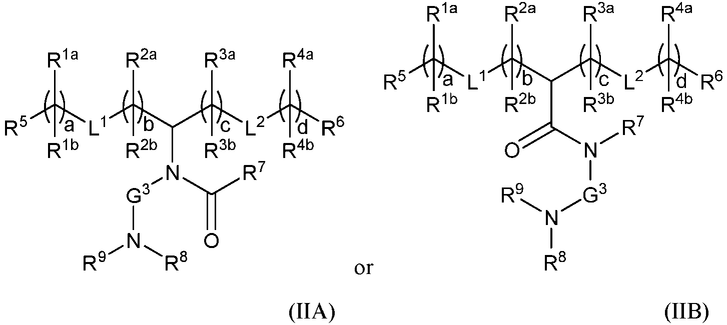

In some embodiments of Formula (II), the lipid compound has structure (IIA). In other embodiments, the lipid compound has structure (IIB).

In any of the foregoing embodiments of Formula (II), one of L' or L² is -O(C=O)-. For example, in some embodiments each of L¹ and L² are -O(C=O)-.

In some different embodiments of Formula (II), one of L¹ or L² is -(C=O)O-. For example, in some embodiments each of L¹ and L² is -(C=O)O-.

In different embodiments of Formula (II), one of L¹ or L² is a direct bond. As used herein, a "direct bond" means the group (e.g., L¹ or L²) is absent. For example, in some embodiments each of L¹ and L² is a direct bond.

In other different embodiments of Formula (II), for at least one occurrence of R^{1a} and R^{1b}, R^{1a} is H or C₁-C₁₂ alkyl, and R^{1b} together with the carbon atom to which it is bound is taken together with an adjacent R^{1b} and the carbon atom to which it is bound to form a carbon-carbon double bond.

In still other different embodiments of Formula (II), for at least one occurrence of R^{4a} and R^{4b}, R^{4a} is H or C₁-C₁₂ alkyl, and R^{4b} together with the carbon atom to which it is bound is taken together with an adjacent R^{4b} and the carbon atom to which it is bound to form a carbon-carbon double bond.

In more embodiments of Formula (II), for at least one occurrence of R^{2a} and R^{2b}, R^{2a} is H or C₁-C₁₂ alkyl, and R^{2b} together with the carbon atom to which it is bound is taken together with an adjacent R^{2b} and the carbon atom to which it is bound to form a carbon-carbon double bond.

In other different embodiments of Formula (II), for at least one occurrence of R^{3a} and R^{3b}, R^{3a} is H or C₁-C₁₂ alkyl, and R^{3b} together with the carbon atom to which it is bound is taken together with an adjacent R^{3b} and the carbon atom to which it is bound to form a carbon-carbon double bond.

In various other embodiments of Formula (II), the lipid compound has one of the following structures (IIC) or (IID): 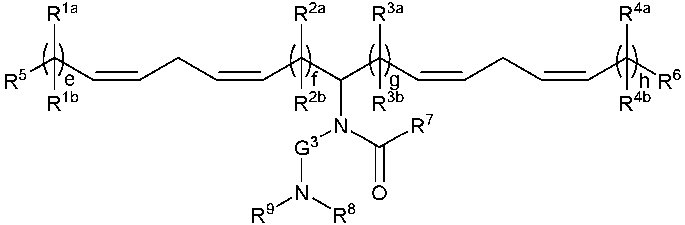 or 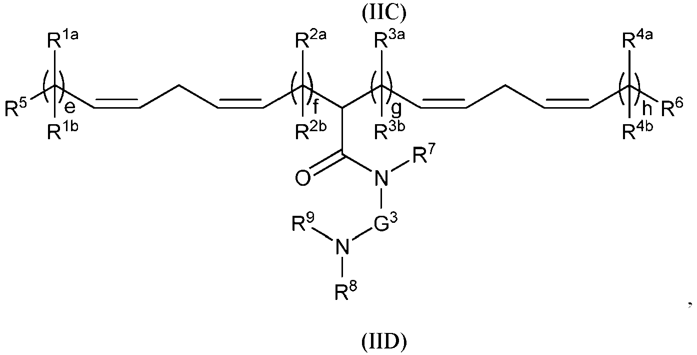 wherein e, f, g and h are each independently an integer from 1 to 12.

In some embodiments of Formula (II), the lipid compound has structure (IIC). In other embodiments, the lipid compound has structure (IID).

In various embodiments of structures (IIC) or (IID), e, f, g and h are each independently an integer from 4 to 10.

In certain embodiments of Formula (II), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some embodiments of Formula (II), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some embodiments of Formula (II), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain embodiments of Formula (II), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. **In** other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some embodiments of Formula (II), e is 1. In other embodiments, e is 2. In more embodiments, e is 3. In yet other embodiments, e is 4. In some embodiments, e is 5. In other embodiments, e is 6. In more embodiments, e is 7. In yet other embodiments, e is 8. In some embodiments, e is 9. In other embodiments, e is 10. In more embodiments, e is 11. In yet other embodiments, e is 12.

In some embodiments of Formula (II), f is 1. In other embodiments, f is 2. In more embodiments, f is 3. In yet other embodiments, f is 4. In some embodiments, f is 5. In other embodiments, f is 6. In more embodiments, f is 7. In yet other embodiments, f is 8. In some embodiments, f is 9. In other embodiments, f is 10. In more embodiments, f is 11. In yet other embodiments, f is 12.

In some embodiments of Formula (II), g is 1. In other embodiments, g is 2. In more embodiments, g is 3. In yet other embodiments, g is 4. In some embodiments, g is 5. In other embodiments, g is 6. In more embodiments, g is 7. In yet other embodiments, g is 8. In some embodiments, g is 9. In other embodiments, g is 10. In more embodiments, g is 11. In yet other embodiments, g is 12.

In some embodiments of Formula (II), h is 1. In other embodiments, e is 2. In more embodiments, h is 3. In yet other embodiments, h is 4. In some embodiments, e is 5. In other embodiments, h is 6. In more embodiments, h is 7. In yet other embodiments, h is 8. In some embodiments, h is 9. In other embodiments, h is 10. In more embodiments, h is 11. In yet other embodiments, h is 12.

In some other various embodiments of Formula (II), a and d are the same. In some other embodiments, b and c are the same. In some other specific embodiments and a and d are the same and b and c are the same.

The sum of a and b and the sum of c and d of Formula (II) are factors which may be varied to obtain a lipid having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such that the sum of a and b and the sum of c and d is 12 or greater.

The substituents at R^{1a}, R^{2a}, R^{3a} and R^{4a} of Formula (II) are not particularly limited. In some embodiments, at least one of R^{1a}, R^{2a}, R^{3a} and R^{4a} is H. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is C₁-C₈ alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (II), R^{1a}, R^{1b}, R^{4a} and R^{4b} are C₁-C₁₂ alkyl at each occurrence.

In further embodiments of Formula (II), at least one of R^{1b}, R^{2b}, R^{3b} and R^{4b} is H or R^{1b}, R^{2b}, R^{3b} and R^{4b} are H at each occurrence.

In certain embodiments of Formula (II), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (II) are not particularly limited in the foregoing embodiments. In certain embodiments one of R⁵ or R⁶ is methyl. In other embodiments each of R⁵ or R⁶ is methyl.

The substituents at R⁷ of Formula (II) are not particularly limited in the foregoing embodiments. In certain embodiments R⁷ is C₆-C₁₆ alkyl. In some other embodiments, R⁷ is C₆-C₉ alkyl. In some of these embodiments, R⁷ is substituted with -(C=O)OR^{b}, - O(C=O)R^{b}, -C(=O)R^{b}, -OR^{b}, -S(O)ₓR^{b}, -S-SR^{b}, -C(=O)SR^{b},
-SC(=O)R^{b}, -NR^{a}R^{b}, -NR^{a}C(=O)R^{b}, -C(=O)NR^{a}R^{b}, -NR^{a}C(=O)NR^{a}R^{b},
-OC(=O)NR^{a}R^{b}, -NR^{a}C(=O)OR^{b}, -NR^{a}S(O)ₓNR^{a}R^{b}, -NR^{a}S(O)ₓR^{b} or -S(O)ₓNR^{a}R^{b},
wherein: R^{a} is H or C₁-C₁₂ alkyl; R^{b} is C₁-C₁₅ alkyl: and x is 0, 1 or 2. For example, in some embodiments R⁷ is substituted with -(C=O)OR^{b} or -O(C=O)R^{b}.

In various of the foregoing embodiments of Formula (II), R^{b} is branched C₁-C₁₅ alkyl. For example, in some embodiments R^{b} has one of the following structures: 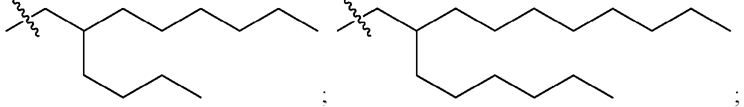 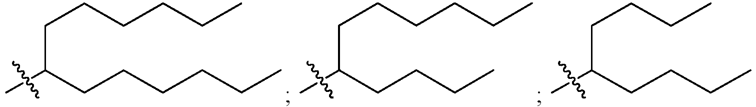 or

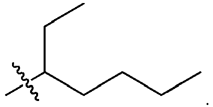

In certain other of the foregoing embodiments of Formula (II), one of R⁸ or R⁹ is methyl. In other embodiments, both R⁸ and R⁹ are methyl.

In some different embodiments of Formula (II), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring. In some different embodiments of the foregoing, R⁸ and R⁹, together with the nitrogen atom to which they are attached, form a 6-membered heterocyclic ring, for example a piperazinyl ring.

In still other embodiments of the foregoing lipids of Formula (II), G³ is C₂-C₄ alkylene, for example C₃ alkylene.

In various different embodiments, the lipid compound has one of the following structures:

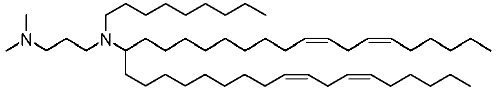

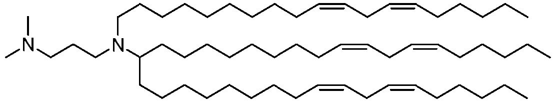

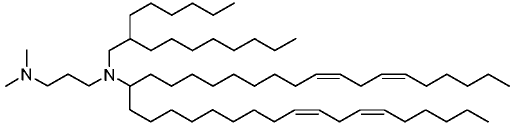

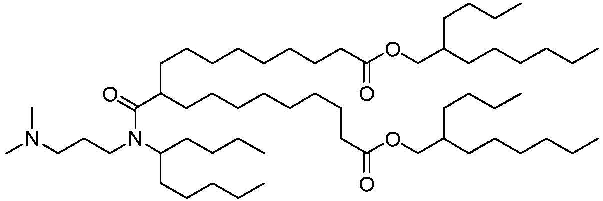

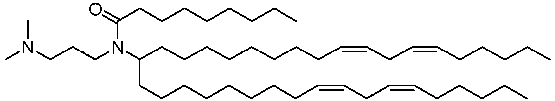

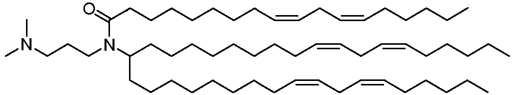

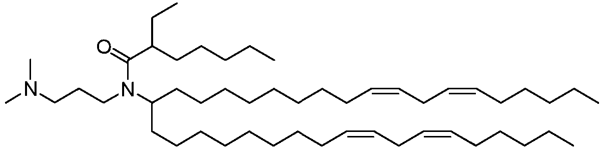

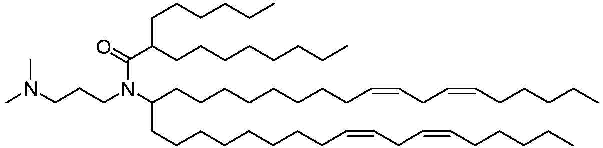

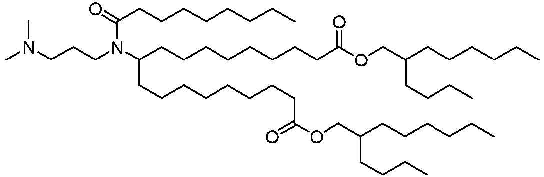

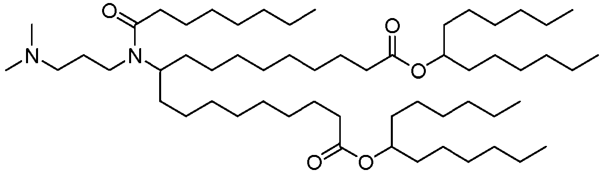

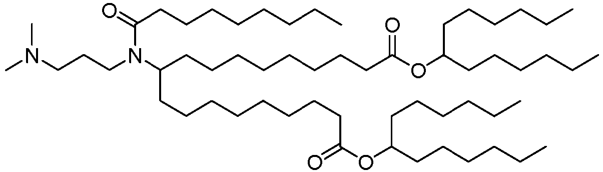

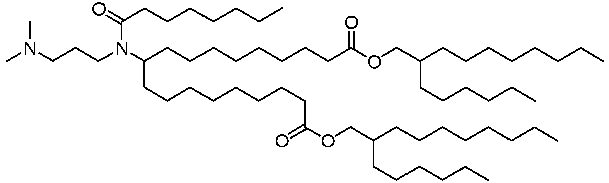

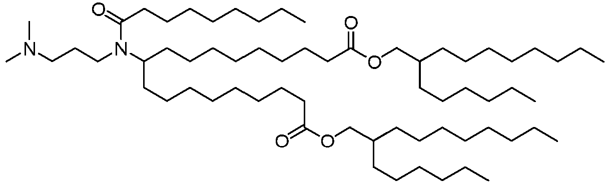

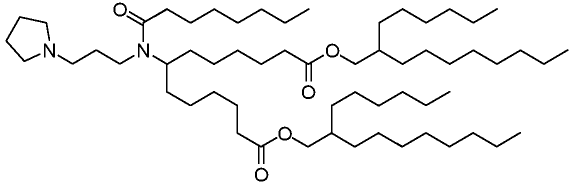

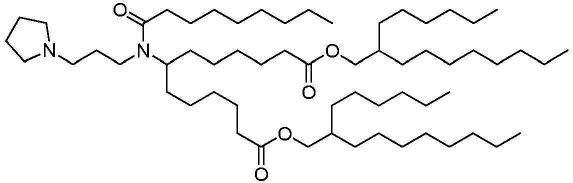

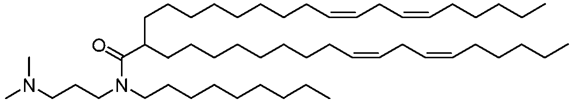

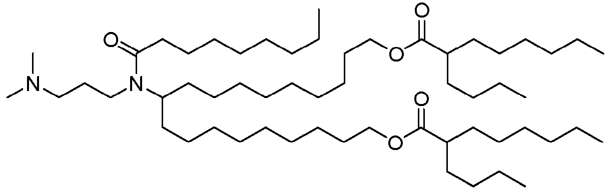

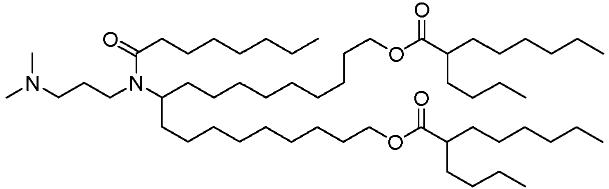

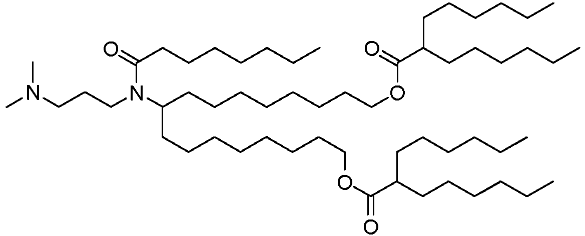

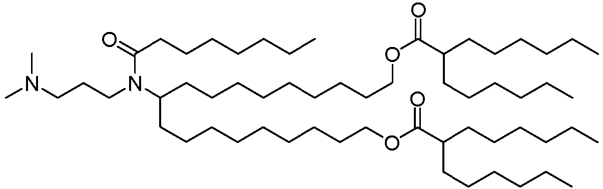

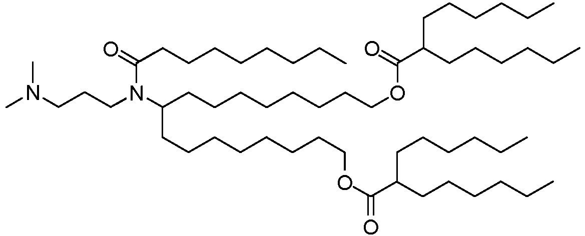

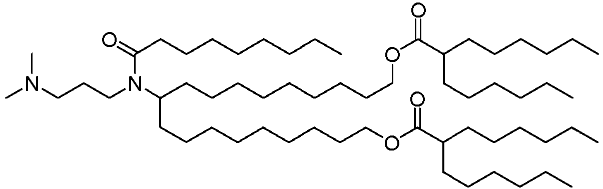

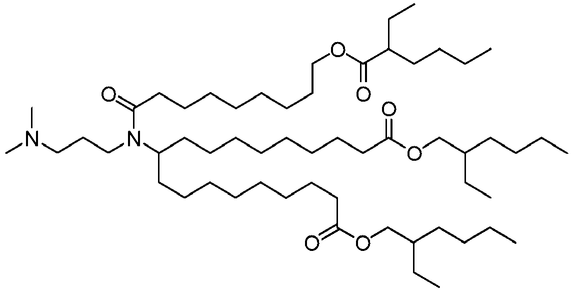

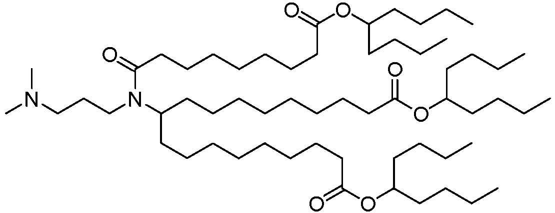

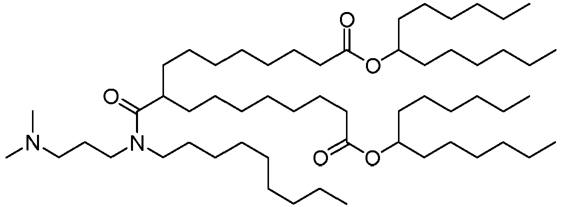

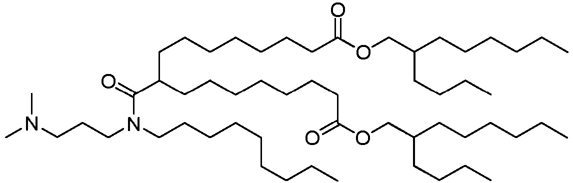

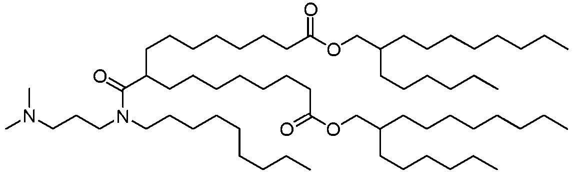

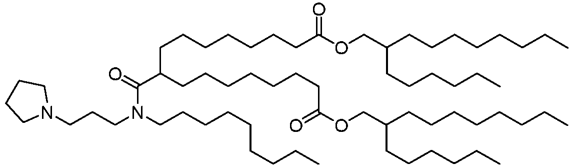

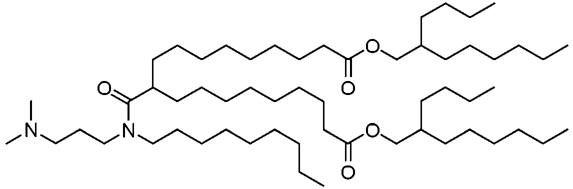

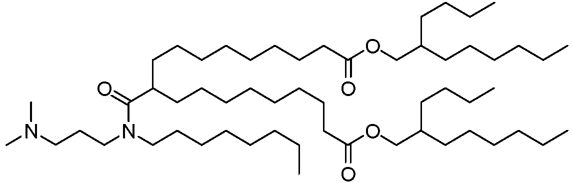

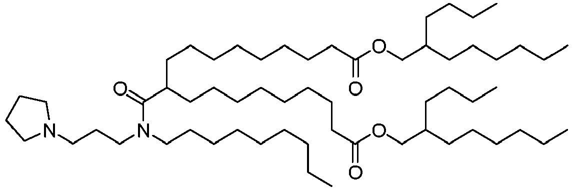

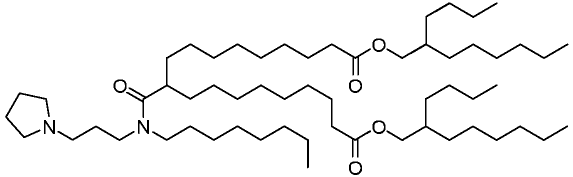

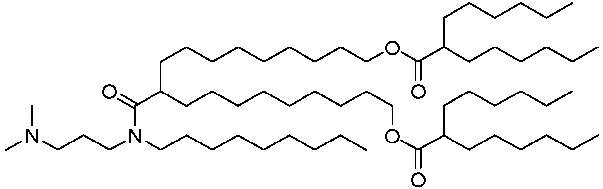

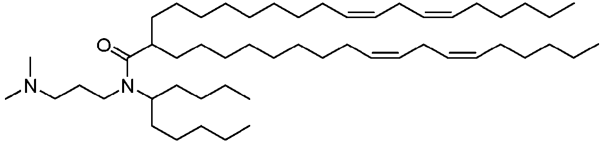

In some embodiments, the LNPs comprise a lipid of Formula (II), at least one agent, and one or more excipient selected from neutral lipids, steroids and pegylated lipids. In some embodiments, the lipid of Formula (II) is compound II-9. In some embodiments, the lipid of Formula (II) is compound II-10. In some embodiments, the lipid of Formula (II) is compound II-11. In some embodiments, the lipid of Formula (II) is compound II-12. In some embodiments, the lipid of Formula (II) is compound II-32.

In some other embodiments, the cationic lipid component of the LNPs has the structure of Formula (III): 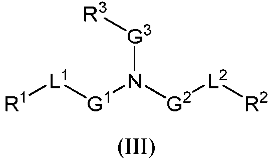 or a pharmaceutically acceptable salt, tautomer, prodrug or stereoisomer thereof, wherein:
one of L¹ or L² is -O(C=O)-, -(C=O)O-, -C(=O)-, -O-, -S(O)ₓ-, -S-S-,
-C(=O)S-, SC(=O)-, -NR^{a}C(=O)-, -C(=O)NR^{a}-, NR^{a}C(=O)NR^{a}-, -OC(=O)NR^{a}- or
-NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴;
R⁴ is C₁-C₁₂ alkyl;
R⁵ is H or C₁-C₆ alkyl; and
x is 0, 1 or 2.

In some of the foregoing embodiments of Formula (III), the lipid has one of the following structures (IIIA) or (IIIB): 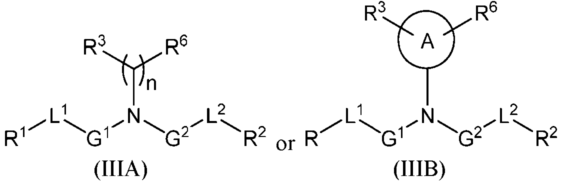 wherein:
A is a 3 to 8-membered cycloalkyl or cycloalkylene ring;
R⁶ is, at each occurrence, independently H, OH or C₁-C₂₄ alkyl;
n is an integer ranging from 1 to 15.

In some of the foregoing embodiments of Formula (III), the lipid has structure (IIIA), and in other embodiments, the lipid has structure (IIIB).

In other embodiments of Formula (III), the lipid has one of the following structures (IIIC) or (IIID): 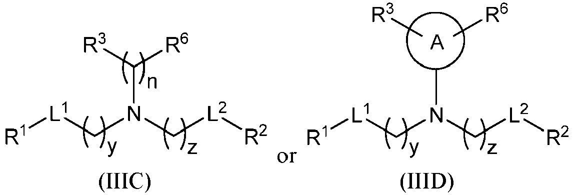 wherein y and z are each independently integers ranging from 1 to 12.

In any of the foregoing embodiments of Formula (III), one of L¹ or L² is -O(C=O)-. For example, in some embodiments each of L¹ and L² are -O(C=O)-. In some different embodiments of any of the foregoing, L¹ and L² are each independently -(C=O)O- or -O(C=O)-. For example, in some embodiments each of L¹ and L² is -(C=O)O-.

In some different embodiments of Formula (III), the lipid has one of the following structures (IIIE) or (IIIF):

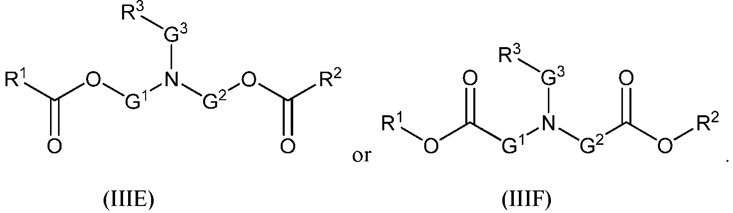

In some of the foregoing embodiments of Formula (III), the lipid has one of the following structures (IIIG), (IIIH), (IIII), or (IIIJ):

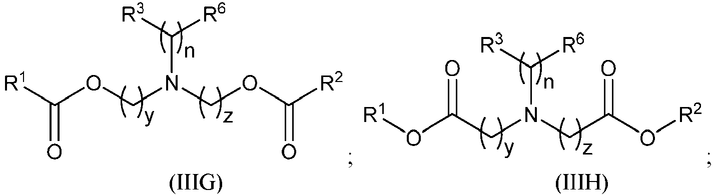

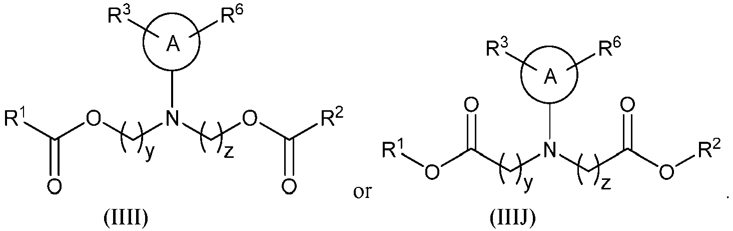

In some of the foregoing embodiments of Formula (III), n is an integer ranging from 2 to 12, for example from 2 to 8 or from 2 to 4. For example, in some embodiments, n is 3, 4, 5 or 6. In some embodiments, n is 3. In some embodiments, n is 4. In some embodiments, n is 5. In some embodiments, n is 6.

In some other of the foregoing embodiments of Formula (III), y and z are each independently an integer ranging from 2 to 10. For example, in some embodiments, y and z are each independently an integer ranging from 4 to 9 or from 4 to 6.

In some of the foregoing embodiments of Formula (III), R⁶ is H. In other of the foregoing embodiments, R⁶ is C₁-C₂₄ alkyl. In other embodiments, R⁶ is OH.

In some embodiments of Formula (III), G³ is unsubstituted. In other embodiments, G3 is substituted. In various different embodiments, G³ is linear C₁-C₂₄ alkylene or linear C₁-C₂₄ alkenylene.

In some other foregoing embodiments of Formula (III), R¹ or R², or both, is C₆-C₂₄ alkenyl. For example, in some embodiments, R¹ and R² each, independently have the following structure: 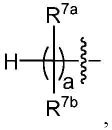 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (III), at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments of Formula (III), R¹ or R², or both, has one of the following structures:

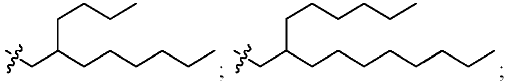

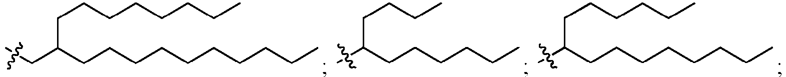

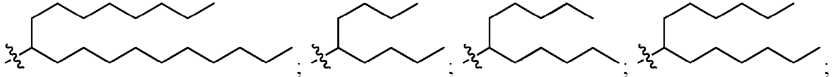

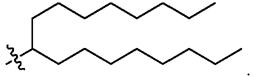

In some of the foregoing embodiments of Formula (III), R³ is OH, CN, -C(=O)OR⁴, -OC(=O)R⁴ or -NHC(=O)R⁴. In some embodiments, R⁴ is methyl or ethyl.

In various different embodiments, the cationic lipid of Formula (III) has one of the following structures:

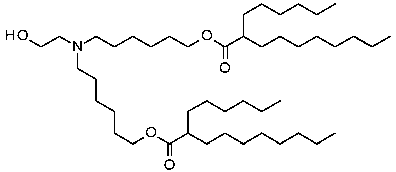

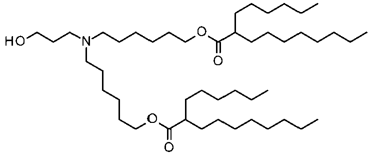

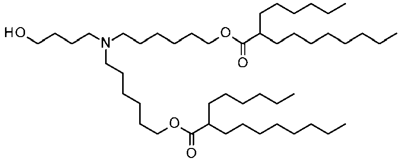

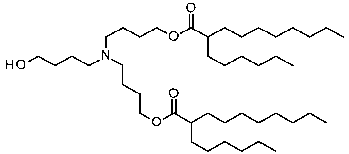

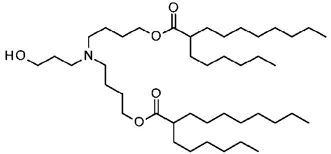

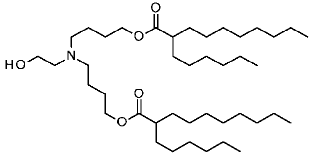

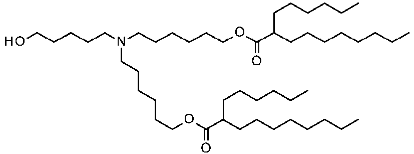

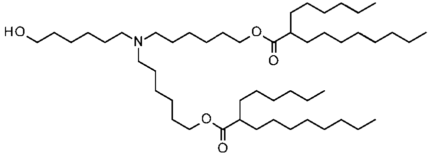

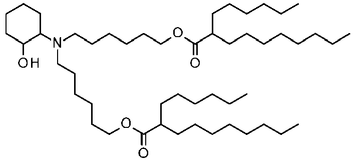

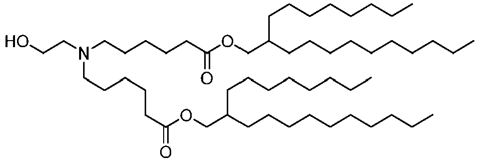

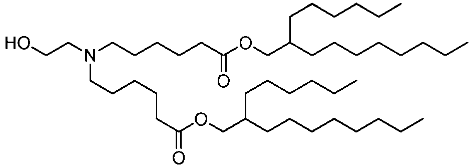

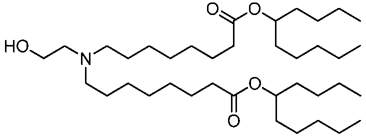

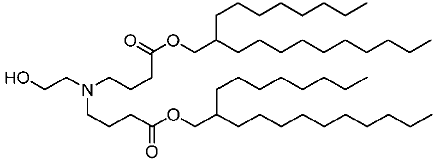

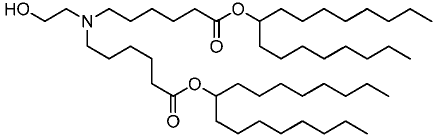

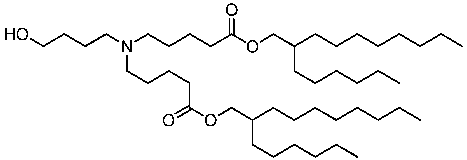

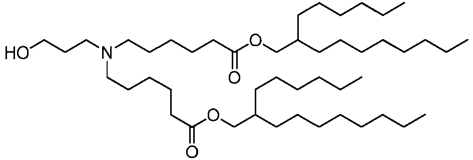

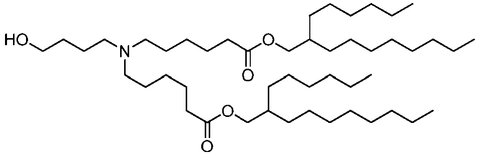

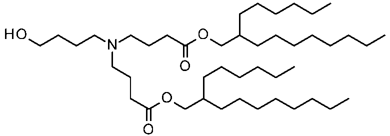

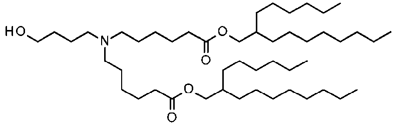

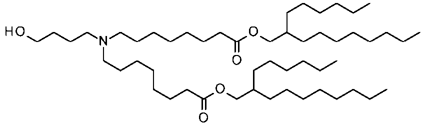

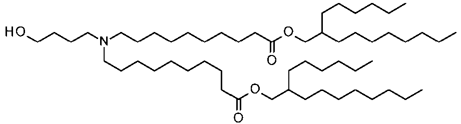

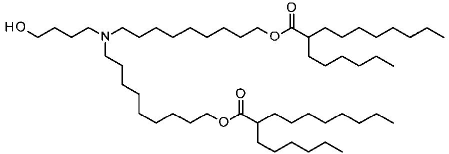

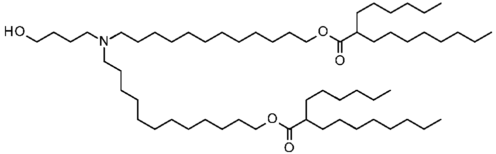

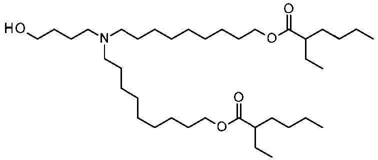

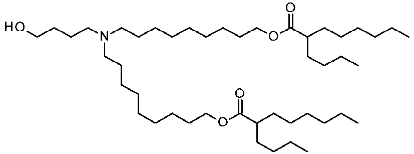

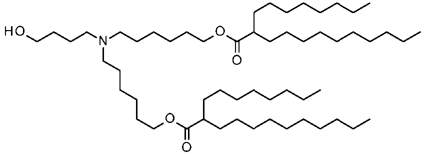

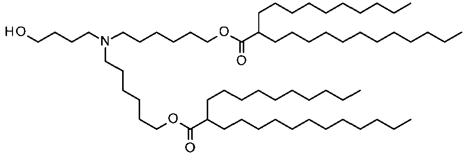

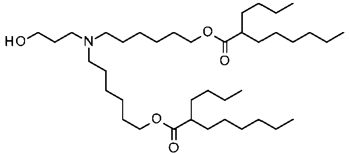

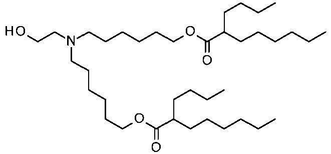

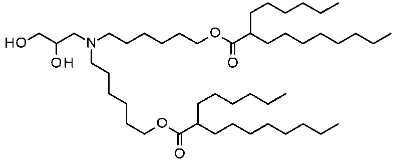

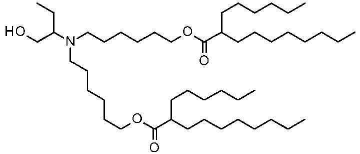

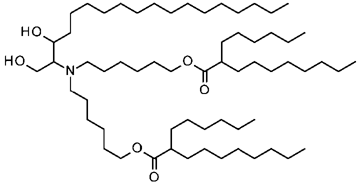

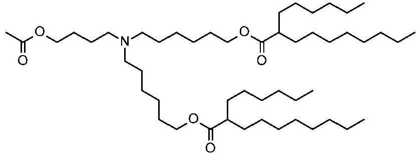

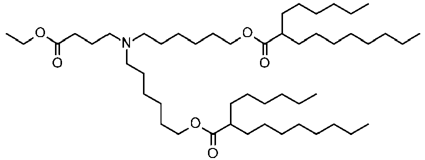

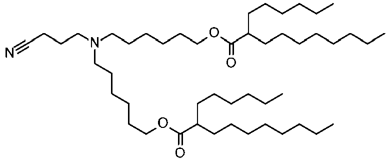

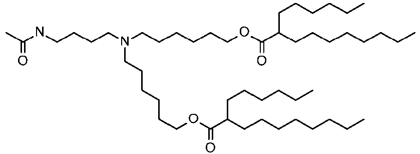

In some embodiments, the LNPs comprise a lipid of Formula (III), at least one agent, and one or more excipient selected from neutral lipids, steroids and pegylated lipids. In some embodiments, the lipid of Formula (III) is compound III-3. In some embodiments, the lipid of Formula (III) is compound III-7.

In certain embodiments, the cationic lipid is present in the LNP in an amount from about 30 to about 95 mole percent. In one embodiment, the cationic lipid is present in the LNP in an amount from about 30 to about 70 mole percent. In one embodiment, the cationic lipid is present in the LNP in an amount from about 40 to about 60 mole percent. In one embodiment, the cationic lipid is present in the LNP in an amount of about 50 mole percent. In one embodiment, the LNP comprises only cationic lipids.

In certain embodiments, the LNP comprises one or more additional lipids which stabilize the formation of particles during their formation.

Suitable stabilizing lipids include neutral lipids and anionic lipids.

The term "neutral lipid" refers to any one of a number of lipid species that exist in either an uncharged or neutral zwitterionic form at physiological pH. Representative neutral lipids include diacylphosphatidylcholines, diacylphosphatidylethanolamines, ceramides, sphingomyelins, dihydro sphingomyelins, cephalins, and cerebrosides.

Exemplary neutral lipids include, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoyl-phosphatidyethanol amine (SOPE), and 1,2-dielaidoyl-sn-glycero-3-phophoethanolamine (transDOPE). In one embodiment, the neutral lipid is 1,2-distearoyl-sn-glycero-3-phosphocholine (DSPC).

In some embodiments, the LNPs comprise a neutral lipid selected from DSPC, DPPC, DMPC, DOPC, POPC, DOPE and SM. In various embodiments, the molar ratio of the cationic lipid (e.g., lipid of Formula (I)) to the neutral lipid ranges from about 2:1 to about 8:1.

In various embodiments, the LNPs further comprise a steroid or steroid analogue. A "steroid" is a compound comprising the following carbon skeleton:

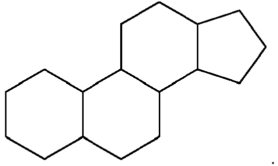

In certain embodiments, the steroid or steroid analogue is cholesterol. In some of these embodiments, the molar ratio of the cationic lipid (e.g., lipid of Formula (I)) to cholesterol ranges from about 2:1 to 1:1.

The term "anionic lipid" refers to any lipid that is negatively charged at physiological pH. These lipids include phosphatidylglycerol, cardiolipin, diacylphosphatidylserine, diacylphosphatidic acid, N-dodecanoylphosphatidylethanolamines, N-succinylphosphatidylethanolamines, N-glutarylphosphatidylethanolamines, lysylphosphatidylglycerols, palmitoyloleyolphosphatidylglycerol (POPG), and other anionic modifying groups joined to neutral lipids.

In certain embodiments, the LNP comprises glycolipids (e.g., monosialoganglioside GM₁). In certain embodiments, the LNP comprises a sterol, such as cholesterol.

In some embodiments, the LNPs comprise a polymer conjugated lipid. The term "polymer conjugated lipid" refers to a molecule comprising both a lipid portion and a polymer portion. An example of a polymer conjugated lipid is a pegylated lipid. The term "pegylated lipid" refers to a molecule comprising both a lipid portion and a polyethylene glycol portion. Pegylated lipids are known in the art and include 1-(monomethoxy-polyethyleneglycol)-2,3-dimyristoylglycerol (PEG-s- DMG) and the like.

In certain embodiments, the LNP comprises an additional, stabilizing -lipid which is a polyethylene glycol-lipid (pegylated lipid). Suitable polyethylene glycol-lipids include PEG-modified phosphatidylethanolamine, PEG-modified phosphatidic acid, PEG-modified ceramides (e.g., PEG-CerC14 or PEG-CerC20), PEG-modified dialkylamines, PEG-modified diacylglycerols, PEG-modified dialkylglycerols. Representative polyethylene glycol-lipids include PEG-c-DOMG, PEG-c-DMA, and PEG-s-DMG. In one embodiment, the polyethylene glycol-lipid is N-[(methoxy poly(ethylene glycol)₂₀₀₀)carbamyl]-1,2-dimyristyloxlpropyl-3-amine (PEG-c-DMA). In one embodiment, the polyethylene glycol-lipid is PEG-c-DOMG). In other embodiments, the LNPs comprise a pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-1-O-((ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3-di(tetradecanoxy)propyl)carbamate or 2,3-di(tetradecanoxy)propyl-N-(ω-methoxy(polyethoxy)ethyl)carbamate. In various embodiments, the molar ratio of the cationic lipid to the pegylated lipid ranges from about 100:1 to about 25:1.

In some embodiments, the LNPs comprise a pegylated lipid having the following structure (IV): 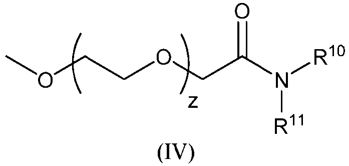 or a pharmaceutically acceptable salt, tautomer or stereoisomer thereof, wherein:
R¹⁰ and R¹¹ are each independently a straight or branched, saturated or unsaturated alkyl chain containing from 10 to 30 carbon atoms, wherein the alkyl chain is optionally interrupted by one or more ester bonds: and
z has mean value ranging from 30 to 60.

In some of the foregoing embodiments of the pegylated lipid (IV), R¹⁰ and R¹¹ are not both n-octadecyl when z is 42. In some other embodiments, R¹⁰ and R¹¹ are each independently a straight or branched, saturated or unsaturated alkyl chain containing from 10 to 18 carbon atoms. In some embodiments, R¹⁰ and R¹¹ are each independently a straight or branched, saturated or unsaturated alkyl chain containing from 12 to 16 carbon atoms. In some embodiments, R¹⁰ and R¹¹ are each independently a straight or branched, saturated or unsaturated alkyl chain containing 12 carbon atoms. In some embodiments, R¹⁰ and R¹¹ are each independently a straight or branched, saturated or unsaturated alkyl chain containing 14 carbon atoms. In other embodiments, R¹⁰ and R¹¹ are each independently a straight or branched, saturated or unsaturated alkyl chain containing 16 carbon atoms. In still more embodiments, R¹⁰ and R¹¹ are each independently a straight or branched, saturated or unsaturated alkyl chain containing 18 carbon atoms. In still other embodiments, R¹⁰ is a straight or branched, saturated or unsaturated alkyl chain containing 12 carbon atoms and R¹¹ is a straight or branched, saturated or unsaturated alkyl chain containing 14 carbon atoms.

In various embodiments, z spans a range that is selected such that the PEG portion of (II) has an average molecular weight of about 400 to about 6000 g/mol. In some embodiments, the average z is about 45.

In other embodiments, the pegylated lipid has one of the following structures: 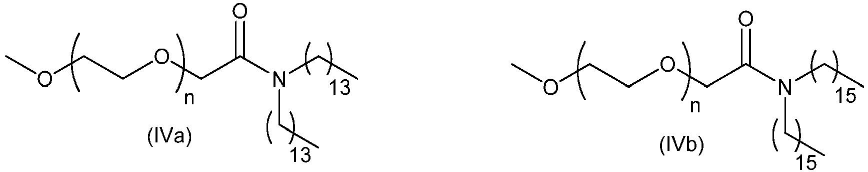 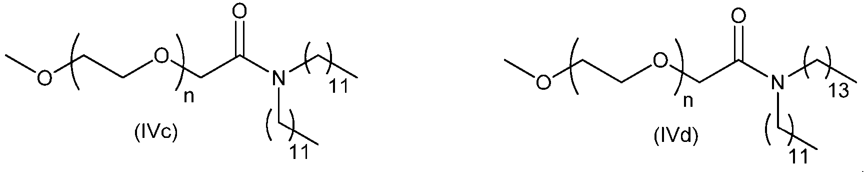 wherein n is an integer selected such that the average molecular weight of the pegylated lipid is about 2500 g/mol.

In certain embodiments, the additional lipid is present in the LNP in an amount from about 1 to about 10 mole percent. In one embodiment, the additional lipid is present in the LNP in an amount from about 1 to about 5 mole percent. In one embodiment, the additional lipid is present in the LNP in about 1 mole percent or about 1.5 mole percent.

In some embodiments, the LNPs comprise a lipid of Formula (I), a nucleoside-modified RNA, a neutral lipid, a steroid and a pegylated lipid. In some embodiments the lipid of Formula (I) is compound I-6. In different embodiments, the neutral lipid is DSPC. In other embodiments, the steroid is cholesterol. In still different embodiments, the pegylated lipid is compound IVa.

In certain embodiments, the LNP comprises one or more targeting moieties that targets the LNP to a cell or cell population. For example, in one embodiment, the targeting domain is a ligand which directs the LNP to a receptor found on a cell surface.

In certain embodiments, the LNP comprises one or more internalization domains. For example, in one embodiment, the LNP comprises one or more domains which bind to a cell to induce the internalization of the LNP. For example, in one embodiment, the one or more internalization domains bind to a receptor found on a cell surface to induce receptor-mediated uptake of the LNP. In certain embodiments, the LNP is capable of binding a biomolecule in vivo, where the LNP-bound biomolecule can then be recognized by a cell-surface receptor to induce internalization. For example, in one embodiment, the LNP binds systemic ApoE, which leads to the uptake of the LNP and associated cargo.

Other exemplary LNPs and their manufacture are described in the art, for example in U.S. Patent Application Publication No. US20120276209, Semple et al., 2010, Nat Biotechnol., 28(2):172-176; Akinc et al., 2010, Mol Ther., 18(7): 1357-1364; Basha et al., 2011, Mol Ther, 19(12): 2186-2200; Leung et al., 2012, J Phys Chem C Nanomater Interfaces, 116(34): 18440-18450; Lee et al., 2012, Int J Cancer., 131(5): E781-90; Belliveau et al., 2012, Mol Ther nucleic Acids, 1: e37; Jayaraman et al., 2012, Angew Chem Int Ed Engl., 51(34): 8529-8533; Mui et al., 2013, Mol Ther Nucleic Acids. 2, e139: Maier et al., 2013, Mol Ther., 21(8): 1570-1578; and Tam et al., 2013, Nanomedicine, 9(5): 665-74, each of which are incorporated by reference in their entirety.

The following Reaction Schemes illustrate methods to make lipids of Formula (I), (II) or (III).

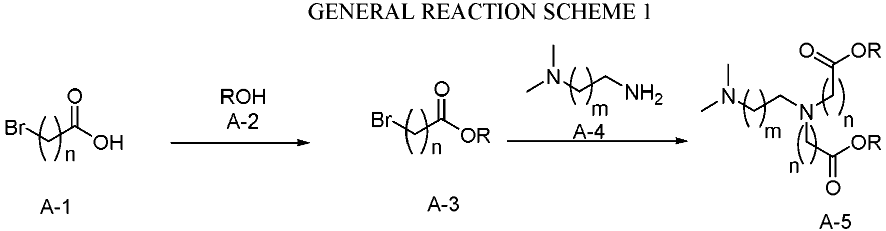

Embodiments of the lipid of Formula (I) (e.g., compound A-5) can be prepared according to General Reaction Scheme 1 ("Method A"), wherein R is a saturated or unsaturated C₁-C₂₄ alkyl or saturated or unsaturated cycloalkyl, m is 0 or 1 and n is an integer from 1 to 24. Referring to General Reaction Scheme 1, compounds of structure A-1 can be purchased from commercial sources or prepared according to methods familiar to one of ordinary skill in the art. A mixture of A-1, A-2 and DMAP is treated with DCC to give the bromide A-3. A mixture of the bromide A-3, a base (e.g., N,N-diisopropylethylamine) and the N,N-dimethyldiamine A-4 is heated at a temperature and time sufficient to produce A-5 after any necessarily workup and or purification step.

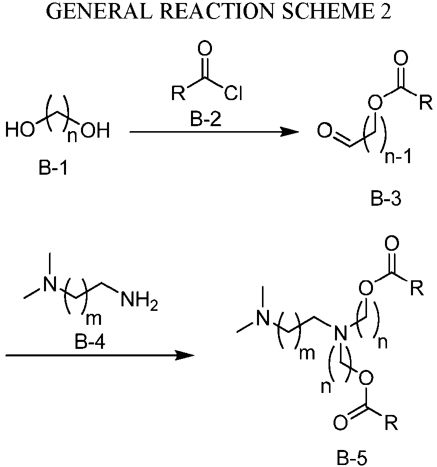

Other embodiments of the compound of Formula (I) (e.g., compound B-5) can be prepared according to General Reaction Scheme 2 ("Method B"), wherein R is a saturated or unsaturated C₁-C₂₄ alkyl or saturated or unsaturated cycloalkyl, m is 0 or 1 and n is an integer from 1 to 24. As shown in General Reaction Scheme 2, compounds of structure B-1 can be purchased from commercial sources or prepared according to methods familiar to one of ordinary skill in the art. A solution of B-1 (1 equivalent) is treated with acid chloride B-2 (1 equivalent) and a base (e.g., triethylamine). The crude product is treated with an oxidizing agent (e.g., pyridinum chlorochromate) and intermediate product B-3 is recovered. A solution of crude B-3, an acid (e.g., acetic acid), and N,N-dimethylaminoamine B-4 is then treated with a reducing agent (e.g., sodium triacetoxyborohydride) to obtain B-5 after any necessary work up and/or purification.

It should be noted that although starting materials A-1 and B-1 are depicted above as including only saturated methylene carbons, starting materials which include carbon-carbon double bonds may also be employed for preparation of compounds which include carbon-carbon double bonds.

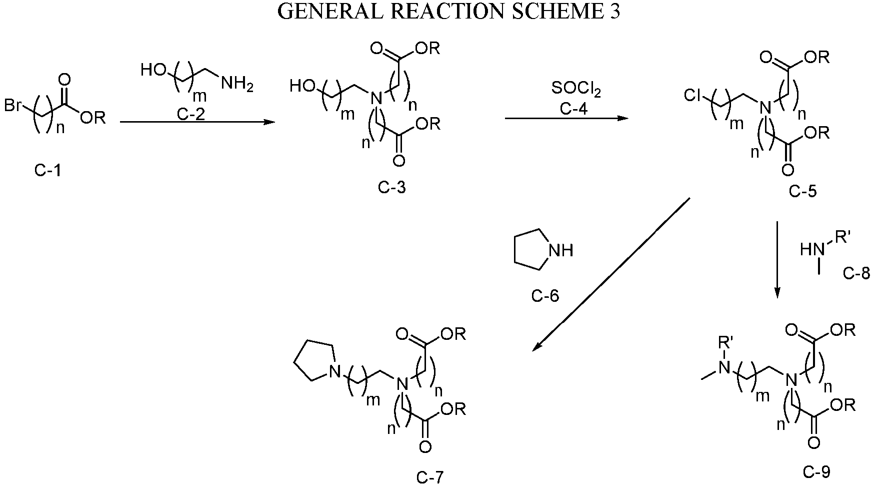

Different embodiments of the lipid of Formula (I) (e.g., compound C-7 or C9) can be prepared according to General Reaction Scheme 3 ("Method C"), wherein R is a saturated or unsaturated C₁-C₂₄ alkyl or saturated or unsaturated cycloalkyl, m is 0 or 1 and n is an integer from 1 to 24. Referring to General Reaction Scheme 3, compounds of structure C-1 can be purchased from commercial sources or prepared according to methods familiar to one of ordinary skill in the art.

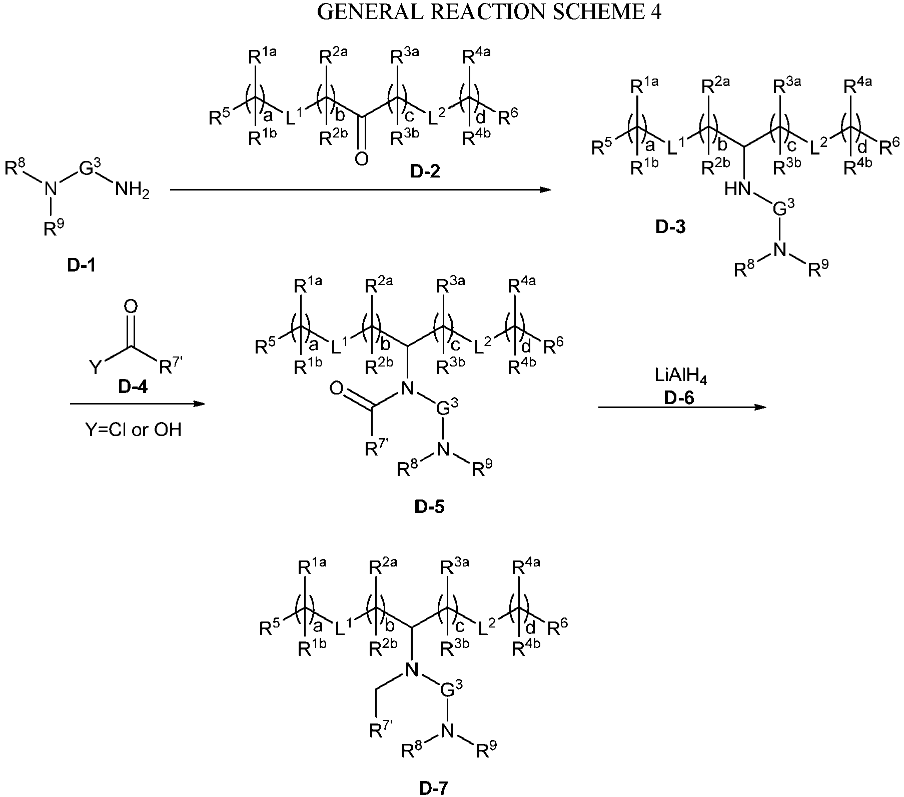

Embodiments of the compound of Formula (II) (e.g., compounds D-5 and D-7) can be prepared according to General Reaction Scheme 4 ("Method D"), wherein R^{1a}, R^{1b}, R^{2a}, R^{2b}, R^{3a}, R^{3b}, R^{4a}, R^{4b}, R⁵, R⁶, R⁸, R⁹, L¹, L², G¹, G², G³, a, b, c and d are as defined herein, and R^{7'} represents R⁷ or a C₃-C₁₉ alkyl. Referring to General Reaction Scheme 1, compounds of structure D-1 and D-2 can be purchased from commercial sources or prepared according to methods familiar to one of ordinary skill in the art. A solution of D-1 and D-2 is treated with a reducing agent (e.g., sodium triacetoxyborohydride) to obtain D-3 after any necessary work up. A solution of D-3 and a base (e.g. trimethylamine, DMAP) is treated with acyl chloride D-4 (or carboxylic acid and DCC) to obtain D-5 after any necessary work up and/or purification. D-5 can be reduced with LiAlH4 D-6 to give D-7 after any necessary work up and/or purification.

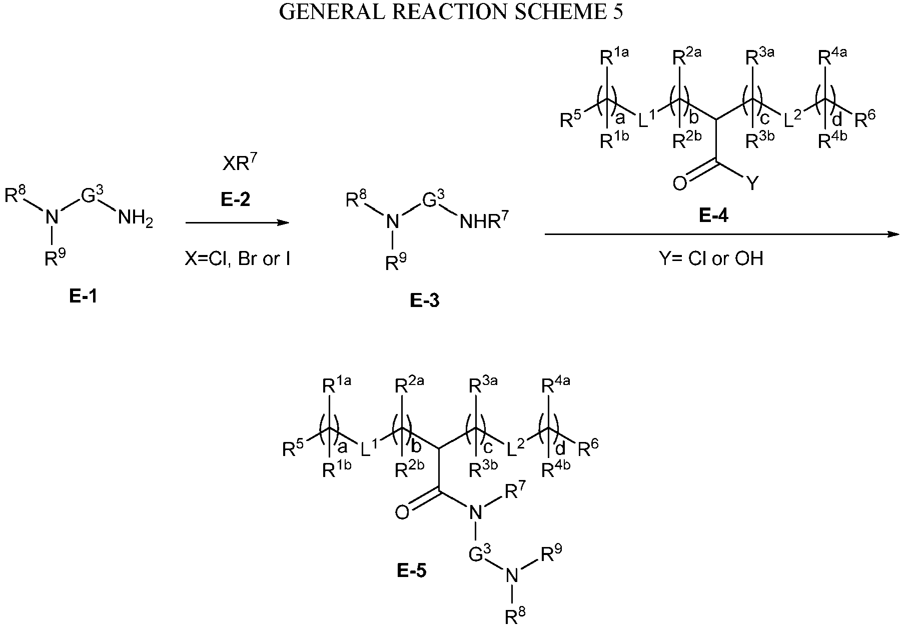

Embodiments of the lipid of Formula (II) (e.g., compound E-5) can be prepared according to General Reaction Scheme 5 ("Method E"), wherein R^{1a}, R^{1b}, R^{2a}, R^{2b}, R^{3a}, R^{3b}, R^{4a}, R^{4b}, R⁵, R⁶, R⁷, R⁸, R⁹, L¹, L², G³, a, b, c and d are as defined herein. Referring to General Reaction Scheme 2, compounds of structure E-1 and E-2 can be purchased from commercial sources or prepared according to methods familiar to one of ordinary skill in the art. A mixture of E-1 (in excess), E-2 and a base (e.g., potassium carbonate) is heated to obtain E-3 after any necessary work up. A solution of E-3 and a base (e.g. trimethylamine, DMAP) is treated with acyl chloride E-4 (or carboxylic acid and DCC) to obtain E-5 after any necessary work up and/or purification.

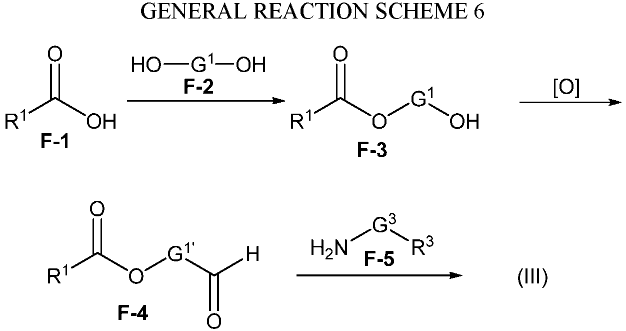

General Reaction Scheme 6 provides an exemplary method (Method F) for preparation of Lipids of Formula (III). G¹, G³, R¹ and R³ in General Reaction Scheme 6 are as defined herein for Formula (III), and G1' refers to a one-carbon shorter homologue of G1. Compounds of structure F-1 are purchased or prepared according to methods known in the art. Reaction of F-1 with diol F-2 under appropriate condensation conditions (e.g., DCC) yields ester/alcohol F-3, which can then be oxidized (e.g., PCC) to aldehyde F-4. Reaction of F-4 with amine F-5 under reductive amination conditions yields a lipid of Formula (III).

It should be noted that various alternative strategies for preparation of lipids of Formula (III) are available to those of ordinary skill in the art. For example, other lipids of Formula (III) wherein L¹ and L² are other than ester can be prepared according to analogous methods using the appropriate starting material. Further, General Reaction Scheme 6 depicts preparation of a lipids of Formula (III), wherein G¹ and G² are the same: however, this is not a required aspect of the invention and modifications to the above reaction scheme are possible to yield compounds wherein G¹ and G² are different.

It will be appreciated by those skilled in the art that in the process described herein the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, t-butyldimethylsilyl, t-butyldiphenylsilyl or trimethylsilyl), tetrahydropyranyl, benzyl, and the like. Suitable protecting groups for amino, amidino and guanidino include t-butoxycarbonyl, benzyloxycarbonyl, and the like. Suitable protecting groups for mercapto include -C(O)-R" (where R" is alkyl, aryl or arylalkyl), p-methoxybenzyl, trityl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

### Delivery Vehicle Embodiments

Any suitable delivery vehicle format is contemplated.

In some embodiments, the delivery vehicle is a colloidal dispersion system, such as macromolecule complexes, nanocapsules, microspheres, beads, and lipid-based systems including oil-in-water emulsions, micelles, mixed micelles, liposomes, and lipid nanoparticles. Exemplary colloidal systems for use as delivery vehicles in vitro and in vivo include liposomes (e.g., an artificial membrane vesicle) and lipid nanoparticles.

The use of lipid formulations, as described above, is contemplated for the introduction of the at least one agent into the host cell (in vitro, ex vivo, or in vivo). In another aspect, the at least one agent may be associated with a lipid. The at least one agent associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, complexed with a lipid, contained or complexed with a micelle, or otherwise associated with a lipid. Lipid, lipid/nucleic acid or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape.

In one embodiment, delivery of the at least one agent comprises any suitable delivery method, including exemplary delivery methods described elsewhere herein. In certain embodiments, delivery of the at least one agent to a subject comprises mixing the at least one agent with a transfection reagent prior to the step of contacting. In another embodiment, a method of the present invention further comprises administering the at least one agent together with the transfection reagent. In another embodiment, the transfection reagent is a cationic lipid reagent.

In another embodiment, the transfection reagent is a lipid-based transfection reagent. In another embodiment, the transfection reagent is a protein-based transfection reagent. In another embodiment, the transfection reagent is a polyethyleneimine based transfection reagent. In another embodiment, the transfection reagent is calcium phosphate. In another embodiment, the transfection reagent is Lipofectin^{®}, Lipofectamine^{®}, or TransIT^{®}. In another embodiment, the transfection reagent is any other transfection reagent known in the art.

In some embodiments, delivery of the at least one agent comprises liposomes. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules.

The at least one agent associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/nucleic acid or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape.

In another embodiment, the transfection reagent forms a liposome. Liposomes, in another embodiment, increase intracellular stability, increase uptake efficiency and improve biological activity. In another embodiment, liposomes are hollow spherical vesicles composed of lipids arranged in a similar fashion as those lipids which make up the cell membrane. In some embodiments, the liposomes comprise an internal aqueous space for entrapping water-soluble compounds. In another embodiment, liposomes can deliver the at least one agent to cells in an active form.

In one embodiment, the composition comprises a lipid nanoparticle (LNP) and at least one agent.

The term "Lipid nanoparticle" refers to a particle having at least one dimension on the order of nanometers (e.g., 1-1000nm) which includes one or more lipids. In some embodiments, LNPs comprise at least one agent that is either organized within inverse lipid micelles and encased within a lipid monolayer envelope or intercalated between adjacent lipid bilayers (e.g. lipid bilayer-agent-lipid bilayer). In some embodiments, the morphology of the LNPs are not like a traditional liposome, which are characterized by a lipid bilayer surrounding an aqueous core, as they possess an electron-dense core, where the cationic/ionizable lipids are organized into inverted micelles around the encapsulated agent (e.g. mRNA molecules)(Cullis and Hope, 2017; Guevara et al., 2019b). In various embodiments, the particle includes a lipid of Formula (I), (II) or (III). In some embodiments, lipid nanoparticles are included in a formulation comprising at least one agent as described herein. In some embodiments, such lipid nanoparticles comprise a cationic lipid (e.g., a lipid of Formula (I), (II) or (III)) and one or more excipients selected from neutral lipids, charged lipids, steroids and lipid-anchored polyethylene glycol (e.g., a pegylated lipid such as a pegylated lipid of structure (IV), such as compound IVa). In some embodiments, the at least one agent is encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells e.g. an adverse immune response.

In various embodiments, the lipid nanoparticles have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In one embodiment, the lipid nanoparticles have a mean diameter of about 83 nm. In one embodiment, the lipid nanoparticles have a mean diameter of about 102 nm. In one embodiment, the lipid nanoparticles have a mean diameter of about 103 nm. In some embodiments, the lipid nanoparticles are substantially non-toxic. In certain embodiments, the at least one agent, when present in the lipid nanoparticles, is resistant in aqueous solution to degradation by intra- or intercellular enzymes

The LNP may comprise any lipid capable of forming a particle to which the at least one agent is attached, or in which the at least one agent is encapsulated or complexed. The term "lipid" refers to a group of organic compounds that are derivatives of fatty acids (e.g., esters) and are generally characterized by being insoluble in water but soluble in many organic solvents. Exemplary lipids are shown elsewhere herein.

In one embodiment, the LNP comprises one or more cationic lipids, and one or more stabilizing lipids. Stabilizing lipids include neutral lipids, anionic lipids and pegylated lipids.

In one embodiment, the LNP comprises a cationic lipid. As used herein, the term "cationic or ionizabe lipid" refers to a lipid that is cationic or becomes cationic (protonated) as the pH is lowered below the pKa of the ionizable group of the lipid, but is progressively more neutral at higher pH values. At pH values below the pKa, the lipid is then able to associate with negatively charged nucleic acids. In certain embodiments, the cationic lipid comprises a zwitterionic lipid that assumes a positive charge on pH decrease.

In various embodiments, the LNP comprises a cationic or ionizable lipids, stabilizing lipids, sterol, and a lipid-anchored polyethylene glycol (i.e PEGylated lipids).

In some embodiments, the LNPs comprise an ionic lipid of Formula (I), at least one agent, and one or more excipients selected from neutral lipids, steroids and pegylated lipids. In some embodiments the lipid of Formula (I) is compound I-5. In some embodiments the lipid of Formula (I) is compound I-6.

In some embodiments, the LNPs comprise an ionic lipid of Formula (II), at least one agent, and one or more excipient selected from neutral lipids, steroids and pegylated lipids. In some embodiments, the lipid of Formula (II) is compound 11-9. In some embodiments, the lipid of Formula (II) is compound II-10. In some embodiments, the lipid of Formula (II) is compound II-11. In some embodiments, the lipid of Formula (II) is compound 11-12. In some embodiments, the lipid of Formula (II) is compound 11-32.

In some embodiments, the LNPs comprise an ionic lipid of Formula (III), at least one agent, and one or more excipient selected from neutral lipids, steroids and pegylated lipids. In some embodiments, the lipid of Formula (III) is compound III-3. In some embodiments, the lipid of Formula (III) is compound III-7.

In certain embodiments, the cationic lipid is present in the LNP in an amount from about 30 to about 95 mole percent. In one embodiment, the cationic lipid is present in the LNP in an amount from about 30 to about 70 mole percent. In one embodiment, the cationic lipid is present in the LNP in an amount from about 40 to about 60 mole percent. In one embodiment, the cationic lipid is present in the LNP in an amount of about 50 mole percent. In one embodiment, the LNP comprises only cationic lipids.

In certain embodiments, the LNP comprises one or more stabilizing lipids (e.g. neutral or anionic lipids) which help to encapsulate the cargo and stabilize the formation of particles during their formation. Exemplary neutral lipids include, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoyl-phosphatidyethanol amine (SOPE), and 1,2-dielaidoyl-sn-glycero-3-phophoethanolamine (transDOPE). In one embodiment, the neutral lipid is 1,2-distearoyl-sn-glycero-3-phosphocholine (DSPC). In various embodiments, the molar ratio of the cationic lipid (e.g., lipid of Formula (I)) to the neutral lipid ranges from about 2:1 to about 8:1.

In various embodiments, the LNPs further comprise a steroid or a steroid analogue. In certain embodiments, the steroid or steroid analogue is cholesterol. In some of these embodiments, the molar ratio of the cationic lipid (e.g., lipid of Formula (I)) to cholesterol ranges from about 2:1 to 1:1.

In certain embodiments, the LNP comprises glycolipids (e.g., monosialoganglioside GM1).

In certain embodiments, the LNP comprises an additional lipid which is a polyethylene glycol-lipid (pegylated lipid) to reduce immune system recognition and improve biodistribution. In one embodiment, the polyethylene glycol-lipid is PEG-c-DOMG. In other embodiments, the LNPs comprise a pegylated diacylglycerol (PEG-DAG) such as 1 (monomethoxy polyethyleneglycol) 2,3 dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3-di(tetradecanoxy)propyl)carbamate or 2,3-di(tetradecanoxy)propyl-N-(ω-methoxy(polyethoxy)ethyl)carbamate. In various embodiments, the molar ratio of the cationic lipid to the pegylated lipid ranges from about 100:1 to about 25:1.

In certain embodiments, the PEGylated lipid is present in the LNP in an amount from about 1 to about 10 mole percent. In one embodiment, the PEGylated lipid is present in the LNP in an amount from about 1 to about 5 mole percent. In one embodiment, the PEGylated lipid is present in the LNP in about 1 mole percent or about 1.5 mole percent.

In some embodiments, the LNPs comprise a lipid of Formula (I), a nucleoside-modified RNA, a neutral lipid, a steroid and a pegylated lipid. In some embodiments the lipid of Formula (I) is compound I-6. In different embodiments, the neutral lipid is DSPC. In other embodiments, the steroid is cholesterol. In still different embodiments, the pegylated lipid is compound IVa.

In certain embodiments, the LNP comprises one or more targeting moieties that targets the LNP to a cell or cell population. For example, in one embodiment, the targeting domain is a ligand which directs the LNP to a receptor found on a cell surface. Exemplary targeting domains include CD4.

In certain embodiments, the LNP comprises one or more internalization domains. For example, in one embodiment, the LNP comprises one or more domains which bind to a cell to induce the internalization of the LNP. For example, in one embodiment, the one or more internalization domains bind to a receptor found on a cell surface to induce receptor-mediated uptake of the LNP. In certain embodiments, the LNP is capable of binding a biomolecule in vivo, where the LNP-bound biomolecule can then be recognized by a cell-surface receptor to induce internalization. For example, in one embodiment, the LNP binds systemic ApoE, which leads to the uptake of the LNP and associated cargo.

Other exemplary LNPs and their manufacture are described in the art, for example in U.S. Patent Application Publication No. US20120276209, Semple et al., 2010, Nat Biotechnol., 28(2):172-176; Akinc et al., 2010, Mol Ther., 18(7): 1357-1364: Basha et al., 2011, Mol Ther, 19(12): 2186-2200; Leung et al., 2012, J Phys Chem C Nanomater Interfaces, 116(34): 18440-18450; Lee et al., 2012, Int J Cancer., 131(5): E781-90: Belliveau et al., 2012, Mol Ther nucleic Acids, 1: e37; Jayaraman et al., 2012, Angew Chem Int Ed Engl., 51(34): 8529-8533; Mui et al., 2013, Mol Ther Nucleic Acids. 2, e139; Maier et al., 2013, Mol Ther., 21(8): 1570-1578; and Tam et al., 2013, Nanomedicine, 9(5): 665-74, each of which are incorporated by reference in their entirety.

### Targeting moieties

As taught above, the delivery vehicles contemplated herein-which may include various formats, such as, but not limited to, macromolecule complexes, nanocapsules, microspheres, beads, and lipid-based systems including oil-in-water emulsions, micelles, mixed micelles, liposomes, and lipid nanoparticles (LNPs)-may comprise one or more targeting moieties (or equivalently "targeting domains" or "targeting ligands") which function to target the delivery vehicle (e.g., LNP) to a cell or cell population. Targeting moieties may include any suitable binding agent which is capable of specifically interacting with and bind to a target cell ligand on the surface of a target cell or tissue. The targeting moieties may be naturally occurring or engineered. Targeting moieties may include, but are not limited to, proteins, peptides, antibodies or antibody fragments, immunoglobulins or immunoglobulin fragments, small molecules, aptamers, vitamins, nucleic acid molecules, and the like. No limit is meant to be placed on the targeting moieties contemplated herein so long as any particular targeting moiety may be (a) coupled to a delivery vehicle (either covalently or non-covalently) and (b) is capable of causing or facilitating the localization or targeting of the delivery vehicle to a target cell or tissue by the binding or otherwise interaction between the targeting moiety on the delivery vehicle and a target cell ligand on a target cell or tissue.

The target cell ligand may include endogenous ligands occurring on the surface of a cell or in the extracellular space outside of a cell, such as carbohydrates, lipids, polysaccharides, proteins, glycoproteins, glycolipids, peptides, cell membrane components (e.g., cholesterol) or the like. In certain embodiments, the endogenous ligands on the target cell are specific for the target cell, i.e., are expressed and/or are contained only on the target cell, or at least, are minimally present in cells that are not the target cells. For example, the endogenous ligand on the target cell could be a disease-associated protein, e.g., a cancer cell protein cell surface protein that are not typically expressed in healthy cells. In other embodiments, the target ligand on the target cells can be an engineered or otherwise non-naturally occurring ligand, e.g., a genetically modified target cell that expresses a non-naturally occurring surface cell protein. Suitable targeting ligands can be selected so that the unique properties of the target cell are utilized, thus allowing the composition to differentiate between target and non-target cells.

This aspect may be referred to as "selective delivery" of a delivery vehicle to a target cell of interest (e.g., a lymphocyte, such as a T-cell). The term "selective delivery" means that delivery vehicles are localized by binding covalently or non-covalently to a target cell (e.g., a particular T-cell subpopulation) through the binding interaction between the targeting moiety of the delivery vehicle and the target cell ligand on the target cell of interest (e.g., a particular T-cell subpopulation), but wherein the delivery vehicles do not bind, or bind minimally, to cells that do not express the target cell ligand (i.e., such cells may be referred to as "non-target cells"). By "bind minimally," it is meant that binding of the delivery vehicle to non-target cells ranges between undetected to less than 1%, or less than 2%, or less than 3%, or less than 4%, or less than 5%, or less than 6%, or less than 7%, or less than 8%, or less than 9%, or less than 10% increased binding relative to a negative control (which can be a cell type known not to bind to the delivery vehicle).

Thus, the delivery vehicles of the present disclosure may be localized or targeted to a particular type of cell (e.g., a particular type of T cell) by utilizing a targeting moiety which is attached (either covalently or non-covalently) to a delivery vehicle. Preferably, the targeting moiety is attached such that the targeting moiety is presented or otherwise exposed on the outer surface of the delivery vehicle such that the moiety may interact with a cognate binding domain or ligand on the surface of a target cell or tissue (e.g., a particular CD4 antigen), thereby promoting or facilitating the binding of the delivery vehicle to the target cell or tissue (such as, CD4+ T cells, where it would then become internalized (e.g., through active internalization, such as endocytosis) with the concomitant release of the agent (e.g., mRNA) carried by the delivery vehicle once inside the cell.

It will be appreciated that a targeting moiety can be linked to the surface of a delivery vehicle during or after preparation. In some embodiments, the targeting moiety is attached to the surface of a delivery vehicle after the vehicles has been prepared. In other embodiments, the targeting moiety is attached to a component (e.g., a lipid) of an unassembled delivery vehicle before the vehicles has been prepared. Such attachment means may be carried out by any known means in the art, including any suitable conjugation chemistry already well known in the art and discussed herein.

In some other embodiments, the delivery vehicles or compositions comprising the delivery vehicles may further include one or more additional agents that enhance the localization of the delivery vehicles to a target cell. Such additional agents may include other peptides, aptamers, oligonucleotides, vitamins or other molecules that facilitate the localization of a delivery vehicle to a target cell, but which are not necessarily directly coupled to the delivery vehicle.

In one embodiment, the delivery vehicles of the present disclosure comprise one or more targeting moieties that are capable of targeting the delivery vehicle to a leukocyte, which generally include myeloid and lymphoid classes of immune system cells. Myeloid cells can include, for example, neutrophils, eosinophils, mast cells, basophils, and monocytes. Monocytes are further classified into dendritic cells and macrophages. Lymphoid cells (or lymphocytes) include T cells (subdivided into helper T cells, memory T cells, and cytotoxic T cells), B cells (subdivided into plasma cells and memory B cells), and natural killer cells.

One or ordinary skill in the art will be able to identify appropriate cell target ligands on each of these types of leukocytes that may be utilized as a means to localize the delivery vehicles described herein by installing an appropriately matching targeting moiety on the delivery vehicle, e.g., an antibody, peptide, protein, oligonucleotide, small molecule, vitamin, or aptamer which is coupled (covalently or non-covalently to the delivery vehicle) such that the delivery vehicle becomes localized to the target cell due to specific, and preferably, selective interaction between the targeting moiety and the cell target ligand.

In various embodiments, the disclosure contemplates delivery vehicles that target and/or localize to leukocytes, and in particular, to a particular lymphocyte, such as a CD4⁺ T cell. In particular embodiments, the delivery vehicles comprise one or more targeting moieties that are capable of targeting the delivery vehicles to T cells, including helper T cells.

One of ordinary skill in the art will appreciate that leukocytes comprise cell surface antigens known as CD antigens which are characteristic of different types of leukocytes and help define various subpopulations of leukocytes.

The cluster of differentiation (CD) is a nomenclature system conceived to identify and classify antigens found on the cell surface of leukocytes. Initially, surface antigens were named after the monoclonal antibodies that bound to them. As there were often multiple monoclonal antibodies raised against each antigen in different labs, the need arose to adopt a consistent nomenclature. The current system was adopted in 1982 through the 1st International Workshop and Conference on Human Leukocyte Differentiation Antigens (HLDA). The Human Cell Differentiation Molecules organization continues to hold HLDA conferences to maintain and develop the list of known CD markers.

Under this naming system, antigens that are well characterized are assigned an arbitrary number (e.g., CD1, CD2, CD3, CD4, CD5, CD8 etc.) whereas molecules that are recognized by just one monoclonal antibody are given the provisional designation "CDw" e.g., CDw50. Lower class letters are also added after the assigned number to indicate larger molecules that share a common chain, for example CD1a or CD1d. Physiologically, CD molecules do not belong in any particular class, with their functions ranging widely from cell surface receptors to adhesion molecules. Although initially used just for human leukocytes, the CD molecule naming convention has now been expanded to cover different species (e.g., mouse) as well as other cell types. As of April 2016, human CD antigens are numbered up to CD371.

The presence or absence of a specific antigen from the surface of a particular cell population is denoted with "+" or "-" respectively. Varying cellular expression levels are also marked as hi or low, for example central memory T-cells are CD62Lhi whereas effector memory T-cells are CD62Llow. Monitoring the expression profiles of different CD antigens has permitted the identification, isolation and phenotyping of cell types according to their function in various immune processes.

The delivery vehicles of the present disclosure may include one or more targeting moieties that bind to or otherwise associate with a CD4 antigen. In various embodiments, the targeting moieties for targeting the delivery vehicles to a target cell (e.g., leukocytes) are antibodies or an antibody binding fragments. Such antibodies or antibody binding fragments may include, but are not limited to, anti-CD4 antibodies or antigen binding fragments.

As used herein, "antibody" refers to a polypeptide of the immunoglobulin family that is capable of binding a corresponding antigen non-covalently, reversibly, and in a specific manner (e.g., a CD4 antigen). For example, a naturally occurring IgG antibody is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

Antibodies of the present disclosure include, but are not limited to, monoclonal antibodies, human antibodies, humanized antibodies, camelid antibodies, chimeric antibodies, and anti-idiotypic (anti-Id) antibodies (including, e.g., anti-Id antibodies to antibodies of the present disclosure). The antibodies can be of any isotype/class (e.g., IgG, IgE, IgM, IgD, IgA and IgY), or subclass (e.g., IgG1, IgG2, IgG3, IgG4, IgA1 and IgA2).

As used herein, "complementarity-determining domains" or "complementary-determining regions" ("CDRs") interchangeably refer to the hypervariable regions of VL and VH. The CDRs are the target protein-binding site of the antibody chains that harbors specificity for such target protein. There are three CDRs (CDR1-3, numbered sequentially from the N-terminus) in each human VL or VH, constituting about 15-20% of the variable domains. CDRs can be referred to by their region and order. For example, "VHCDR1" or "HCDR1" both refer to the first CDR of the heavy chain variable region. The CDRs are structurally complementary to the epitope of the target protein and are thus directly responsible for the binding specificity. The remaining stretches of the VL or VH, the so-called framework regions, exhibit less variation in amino acid sequence (Kuby, Immunology, 4th ed., Chapter 4. W.H. Freeman & Co., New York, 2000).

The positions of the CDRs and framework regions can be determined using various well known definitions in the art, e.g., Kabat, Chothia, and AbM (see, e.g., Johnson et al., Nucleic Acids Res., 29:205-206 (2001): Chothia and Lesk, J. Mol. Biol., 196:901-917 (1987); Chothia et al., Nature, 342:877-883 (1989); Chothia et al., J. Mol. Biol., 227:799-817 (1992); Al-Lazikani et al., J. Mol. Biol., 273:927-748 (1997)). Definitions of antigen combining sites are also described in the following: Ruiz et al., Nucleic Acids Res., 28:219-221 (2000); and Lefranc, M. P., Nucleic Acids Res., 29:207-209 (2001): MacCallum et al., J. Mol. Biol., 262:732-745 (1996); and Martin et al., Proc. Natl. Acad. Sci. USA, 86:9268-9272 (1989); Martin et al., Methods Enzymol., 203:121-153 (1991): and Rees et al., In Sternberg M. J. E. (ed.), Protein Structure Prediction, Oxford University Press, Oxford, 141-172 (1996).). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HC CDR1), 50-65 (HC CDR2), and 95-102 (HC CDR3) in a VH, e.g., a mammalian VH, e.g., a human VH; and amino acid residues 24-34 (LC CDR1), 50-56 (LC CDR2), and 89-97 (LC CDR3) in a VL, e.g., a mammalian VL, e.g., a human VL.

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention, the numbering of the constant region domains increases as they become more distal from the antigen binding site or amino-terminus of the antibody. The N-terminus is a variable region and at the C-terminus is a constant region; the CH3 and CL domains actually comprise the carboxy-terminal domains of the heavy and light chain, respectively.

As used herein, "antigen binding fragment" refers to one or more portions of an antibody that retain the ability to specifically interact with (e.g., by binding, steric hindrance, stabilizing/destabilizing, spatial distribution) an epitope of a CD4 antigen of a leukocyte. Examples of binding fragments include, but are not limited to, single-chain Fvs (scFv), disulfide-linked Fvs (sdFv), Fab fragments, F(ab') fragments, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., Nature 341:544-546, 1989), which consists of a VH domain; and an isolated complementarity determining region (CDR), or other epitope-binding fragments of an antibody.

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv ("scFv"); see, e.g., Bird et al., Science 242:423-426, 1988: and Huston et al., Proc. Natl. Acad. Sci. 85:5879-5883, 1988). Such single chain antibodies are also intended to be encompassed within the term "antigen-binding fragment." These antigen binding fragments are obtained using conventional techniques known to those of skill in the art, and the fragments are screened for utility in the same manner as are intact antibodies.

Antigen binding fragments can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can be grafted into scaffolds based on polypeptides such as fibronectin type III (Fn3) (see U.S. Pat. No. 6,703,199, which describes fibronectin polypeptide monobodies). Accordingly, the antibodies and antigen binding fragments herein (e.g., anti-CD4 antigen binding fragments) can be a variety of structures, including, but not limited to bispecific antibodies, minibodies, domain antibodies, synthetic antibodies, antibody mimetics, chimeric antibodies, antibody fusions (sometimes referred to as "antibody conjugates"), and fragments of each, respectively. Specific antibody fragments (or antigen binding fragments) include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) the dAb fragment, which consists of a single variable region, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (viii) bispecific single chain Fv dimers and (ix) "diabodies" or "triabodies", multivalent or multispecific fragments constructed by gene fusion. The antibody fragments may be modified. For example, the molecules may be stabilized by the incorporation of disulfide bridges linking the VH and VL domains. Examples of antibody formats and architectures are described in Carter, 2006, Nature Reviews Immunology 6:343-357 and references cited therein, all expressly incorporated by reference.

Antigen binding fragments can be incorporated into single chain molecules comprising a pair of tandem Fv segments (VH-CH1-VH-CH1) which, together with complementary light chain polypeptides, form a pair of antigen binding regions (Zapata et al., Protein Eng. 8:1057-1062, 1995; and U.S. Pat. No. 5,641,870).

As used herein, "monoclonal antibody" refers to polypeptides, including antibodies and antigen binding fragments that have substantially identical amino acid sequence or are derived from the same genetic source. This term also includes preparations of antibody molecules of single molecular composition. A monoclonal antibody composition displays a single binding specificity and affinity for a particular epitope.

As used herein, a "human antibody" includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik et al., J. Mol. Biol. 296:57-86, 2000).

In some embodiments, the antibody is a chimeric antibody or antigen-binding fragment thereof. A chimeric antibody is an antibody comprising amino acid sequences from different genetic sources. In some embodiments, the chimeric antibody comprises amino acid sequences from a mouse and amino acid sequences from a human. In some embodiments a chimeric antibody comprises a variable domain derived from a mouse and constant domains derived from a human.

In some embodiments, the antibody is a humanized antibody or antigen-binding fragment thereof. By "humanized" antibody as used herein is meant an antibody comprising a human framework region (FR) and one or more complementarity determining regions (CDRs) from a non-human (usually mouse or rat) antibody. The non-human antibody providing the CDRs is called the "donor" and the human immunoglobulin providing the framework is called the "acceptor". Humanization relies principally on the grafting of donor CDRs onto acceptor (human) VL and VH frameworks (Winter U.S. Pat. No. 5,225,539, incorporated entirely by reference). This strategy is referred to as "CDR grafting." "Backmutation" of selected acceptor framework residues to the corresponding donor residues is often required to regain affinity that is lost in the initial grafted construct (U.S. Pat. No. 5,693,762, incorporated entirely by reference). The humanized antibody optimally also will comprise at least a portion of an immunoglobulin constant region, typically that of a human immunoglobulin, and thus will typically comprise a human Fc region. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein, all incorporated entirely by reference). Humanization or other methods of reducing the immunogenicity of nonhuman antibody variable regions may include resurfacing methods, as described for example in Roguska et al., 1994, Proc. Natl. Acad. Sci. USA 91:969-973, incorporated entirely by reference. In one embodiment, selection-based methods may be employed to humanize and/or affinity mature antibody variable regions, that is, to increase the affinity of the variable region for its target antigen. Other humanization methods may involve the grafting of only parts of the CDRs, including but not limited to methods described in U.S. Ser. No. 09/810,502: Tan et al., 2002, J. Immunol. 169:1119-1125; De Pascalis et al., 2002, J. Immunol. 169:3076-3084, incorporated entirely by reference. Structure-based methods may be employed for humanization and affinity maturation, for example as described in U.S. Ser. No. 10/153,159 and related applications, all incorporated entirely by reference.

In some embodiments, the antibody is a human engineered antibody. A human engineered antibody refers to an antibody derived from a non-human source, such as mouse, in which one or more substitutions have been made to improve a desired characteristic of the antibody, such as to increase stability or reduce immunogenicity when the antibody is administered to a subject. In some embodiments, the substitutions are made at low-risk positions (e.g. exposed to solvent but not contributing to antigen binding or antibody structure). Such substitutions mitigate the risk that a subject will generate an immune response against the antibody following its administration, without affecting the ability of the antibody to bind to a desired epitope or antigen (see, e.g,. Studnicka et al. Protein Eng. 1994. 7(6):805-814).

In some embodiments, the antibody is a single chain antibody or antigen-binding fragment. A single chain antibody, or single chain variable fragment (scFV) is a protein or polypeptide comprising a VH domain and a VL domain joined together, such as by a synthetic linker, to form a single protein or polypeptide (see, e.g., Bird et al., Science. 242:423-426, 1988; and Huston et al., Proc. Natl. Acad. Sci. 85:5879-5883, 1988).

In some embodiments, the antibody is an antibody fragment or antigen-binding fragment. An antibody fragment is protein or polypeptide derived from an antibody. An antigen-binding fragment is a protein or polypeptide derived from an antibody that is capable of binding to the same epitope or antigen as the antibody from which it was derived.

In some embodiments, the antibody has reduced glycosylation, no glycosylation, or is hypofucosylated. Glycosylation refers to the covalent attachment of sugar, monosaccharide, disaccharide, oligosaccharide, polysaccharide, or glycan moieties to a molecule, such as a polypeptide or protein. These sugar or glycan moieties are generally attached to an antibody in a post-translational matter, prior to secretion by a B cell. An antibody with reduced glycosylation has fewer of these attached sugar or glycan moieties than the number that are typically attached to an antibody with a substantially identical amino acid sequence, such as when the antibody is produced by a B cell in vitro or in vivo in a mouse or human. An antibody with no glycosylation has no attached sugar or glycan moieties. An antibody that is hypofucosylated has fewer fucosyl residues than the number that are typically attached to an antibody with a substantially identical amino acid sequence, such as when the antibody is produced by a B cell in vitro or in vivo in a mouse or human.

In still other embodiments, the antibodies and antigen binding fragments discussed herein may be modified in a manner that reduces immunogenicity. Modifications to reduce immunogenicity may include modifications that reduce binding of processed peptides derived from the parent sequence to MHC proteins. For example, amino acid modifications would be engineered such that there are no or a minimal number of immune epitopes that are predicted to bind, with high affinity, to any prevalent MHC alleles. Several methods of identifying MHC-binding epitopes in protein sequences are known in the art and may be used to score epitopes in an antibody of the present invention. See, for example, U.S. Ser. No. 09/903,378, U.S. Ser. No. 10/754,296, U.S. Ser. No. 11/249,692, and references cited therein, all expressly incorporated by reference.

CD4 is a type I transmembrane protein in which four immunoglobulin superfamily domains (designated in order as D 1 to D4 from the N terminal to the cell membrane side) are present on the outside of the cells, and two N-linked sugar chains in total are bound to the domains D3 to D4. CD4 binds to a major histocompatibility complex (MHC) class II molecule through Dl and D2 domains, and then activates the T cells. Further, it is also known that CD4 polymerizes through D3 and D4 domains. The Dl domain of CD4 is known to serve as a receptor for a human immunodeficiency virus (HIV) (Anderson et al, Clinical Immunology and Immunopathology, 84(1):73-84), 1997).

CD4 comprises the following amino acid sequence as per entry No. P01730-1 (UniParc):

MNRGVPFRHLLLVLQLALLPAATQGKKVVLGKKGDTVELTCTASQKKSIQFHWKNSNQ
IKILGNQGSFLTKGPSKLNDRADSRRSLWDQGNFPLIIKNLKIEDSDTYICEVEDQKEEVQ
LLVFGLTANSDTHLLQGQSLTLTLESPPGSSPSVQCRSPRGKNIQGGKTLSVSQLELQDSG
TWTCTVLQNQKKVEFKIDIVVLAFQKASSIVYKKEGEQVEFSFPLAFTVEKLTGSGELW
WQAERASSSKSWITFDLKNKEVSVKRVTQDPKLQMGKKLPLHLTLPQALPQYAGSGNL
TLALEAKTGKLHQEVNLVVMRATQLQKNLTCEVWGPTSPKLMLSLKLENKEAKVSKRE
KAVWVLNPEAGMWQCLLSDSGQVLLESNIKVLPTWSTPVQPMALIVLGGVAGLLLFIGL
GIFFCVRCRHRRRQAERMSQIKRLLSEKKTCQCPHRFQKTCSPI (SEQ ID NO:1).

The anti-CD4 antibodies of the present invention may be any antibody that binds to CD4, e.g., may comprise the variable regions (e.g., the CDRs) of any known or undiscovered anti-CD4 antibody. Antibodies of the invention may display selectivity for CD4. Examples include full-length versus splice variants, cell-surface vs. soluble forms, selectivity for various polymorphic variants, or selectivity for specific conformational forms of a target. An antibody of the present invention may bind any epitope or region on CD4 and may be specific for fragments, mutant forms, splice forms, or aberrant forms of said antigens. Examples of CD4-positive cells include CD4-positive T cells such as a Thl cell, a Th2 cell, a Thl7 cell, a regulatory T cell (Treg), and a γδT cell. Further, CD4-positive cells are associated with diseases including cancer and inflammatory diseases (e.g., autoimmune disease or an allergic disease).

Numerous anti-CD4 antibodies and antigen binding fragments are known in the art and/or are available commercially, all of which may find use in the present invention.

Table 1 provides a list of various commercially-sourced anti-CD4 antibodies that may be used in the present disclosure.

**Table 1: Exemplary anti-CD4 antibodies available commercially**

| **Antibody Name/Description** | **Source** |
|---|---|
| CD4 Monoclonal Antibody (RPA-T4), PE, eBioscience^{™} | ThermoFisher Scientific Cat #12-0049-42 |
| CD4 Monoclonal Antibody (RPA-T4), FITC, eBioscience^{™} | ThermoFisher Scientific Cat #11-0049-80 |
| CD4 Monoclonal Antibody (RPA-T4), PE, eBioscience^{™} | ThermoFisher Scientific Cat #12-0049-80 |
| CD4 Monoclonal Antibody (OKT4 (OKT-4)), FITC, eBioscience^{™} | ThermoFisher Scientific Cat # 11-0048-42 |
| CD4 Monoclonal Antibody (OKT4 (OKT-4)), FITC, eBioscience^{™} | ThermoFisher Scientific Cat # 11-0048-80 |
| CD4 Monoclonal Antibody (OKT4 (OKT-4)), Alexa Fluor 700, eBioscience^{™} | ThermoFisher Scientific Cat # 56-0048-82 |
| CD4 Monoclonal Antibody (OKT4 (OKT-4)), Alexa Fluor 488, eBioscience^{™} | ThermoFisher Scientific Cat # 53-0048-42 |
| CD4 Monoclonal Antibody (OKT4 (OKT-4)), Alexa Fluor 700, eBioscience^{™} | ThermoFisher Scientific Cat # 56-0048-41 |
| CD4 Monoclonal Antibody (RPA-T4), eBioscience^{™} | ThermoFisher Scientific Cat # 14-0049-82 |
| CD4 Monoclonal Antibody (RPA-T4), APC, eBioscience^{™} | ThermoFisher Scientific Cat # 17-0049-42 |
| CD4 Monoclonal Antibody (RPA-T4), PerCP-Cyanine5.5, eBioscience^{™} | ThermoFisher Scientific Cat # 45-0049-42 |
| CD4 Monoclonal Antibody (RPA-T4), PE-Cvanine7, eBioscience^{™} | ThermoFisher Scientific Cat # 25-0049-42 |
| CD4 Monoclonal Antibody (RPA-T4), PE-Cyanine5, eBioscience^{™} | ThermoFisher Scientific Cat # 15-0049-42 |
| CD4 Monoclonal Antibody (RPA-T4), eFluor 450, eBioscience^{™} | ThermoFisher Scientific Cat # 48-0049-42 |
| CD4 Monoclonal Antibody (RPA-T4), Alexa Fluor 700, eBioscience^{™} | ThermoFisher Scientific Cat # 56-0049-42 |
| CD4 Monoclonal Antibody (RPA-T4), Biotin, eBioscience^{™} | ThermoFisher Scientific Cat # 13-0049-82 |
| CD4 Monoclonal Antibody (RPA-T4), Functional Grade, eBioscience^{™} | ThermoFisher Scientific Cat # 16-0049-85 |
| CD4 Monoclonal Antibody (RPA-T4), PE-eFluor 610, eBioscience^{™} | ThermoFisher Scientific Cat # 61-0049-42 |

In addition to commercial source, a large number of monoclonal antibodies against CD4 have been reported in the literature. Various anti-CD4 mAbs are under clinical development for the purpose of treating cancers, immune diseases, and infections. For example, based on the fact that the binding between CD4 and HIV is essential for the infection of HIV, an antibody which recognizes Dl domain of CD4 can inhibit the infection of HIV, under the development as an HIV therapeutic agent. Examples of anti-CD4 mAbs developed as a therapeutic agent for cancers or immune diseases include zanolimumab (6G5), ibalizumab, tregalizumab, and keliximab (CE9.1). These antibodies are antibodies which exert their medicinal efficacy by specifically attacking CD4-expressing cells which are target cells, and it is considered that the mechanism of medicinal efficacy is mainly due to an ADCC activity (Kim er a/., Blood, 109(11):4655-4662, 2007).

In addition, the present disclosure contemplates the use of any of the anti-CD4 antibodies or antibody fragments thereof disclosed in the following references: US7338658B2; US5741488A; US5871732A; US9758581B2; Delmonico et al., "Anti-CD4 monoclonal antibody therapy," Clin Transplant, 1996, Oct 10(5): pp. 397-403; Konig et al., "Tregalizumab - A Monoclonal Antibody to Target Regulatory T Cells," Front Immunol. 2016, Vol.7:11; and JF Bach, "Therapeutic monoclonal antibodies," Ann Pharm Fr., 2006, 64(5): 308-11, each of which are incorporated herein by reference in their entireties.

All of the above-noted commercially-available and anti-CD4 antibodies known in the literature can be used in the instant disclosure.

In certain other embodiments, the targeting moieties for targeting the delivery vehicles to a target cell (e.g., leukocytes) are anti-CD8 antibodies or antigen binding fragments.

CD8 is a surface glycoprotein that functions as a co-receptor for TCR recognition of peptide antigen complexed with MHC Class I molecule (pMHCI). It is expressed either as an αα homodimer or as an αβ heterodimer (Zamoyska, Immunity, 1 :243-6, 1994), both chains expressing a single extracellular Ig superfamily (IgSF) V domain, a membrane proximal hinge region, a transmembrane domain, and a cytoplasmic tail. CD8 interacts with im and the a2 and a3 domains of MHC Class I molecules using its β strands and the complementary determining regions (CDRs) within the extracellular IgSF V domain. This association increases the adhesion/avidity of the T cell receptor with its Class I target.

In addition, an internal signaling cascade mediated by the CD8a chain associated tyrosine protein kinase p561ck4'5 leads to T cell activation. Lck is required for activation and expansion of naive CD8+ T cells; however its expression is not essential for responses of memory CD8+ T cells to secondary antigenic stimulation in vivo or in vitro (Bachman et al, J Exp Med, 189: 1521-30, 1999). As shown by either CD8a or CD8B gene targeted mice, CD8 plays an important role in the maturation and function of MHC Class I-restricted T lymphocytes (Nakayama et al, Science, 263: 1131-3, 1984). One patient suffering from repeated bacterial infections was found to display a CD8 deficiency due to a single mutation in the CD8a gene. The lack of CD8 did not appear to be essential for either CD8+ T cell lineage commitment or peripheral cytolytic function (de la Calle-Martin et al, J Clin Invest, 108: 117- 23, 2001).

The human CD8 molecule is a glycoprotein and cell surface marker expressed on cytotoxic T-cells (CTLs). These are a subset of T-lymphocytes and play an important role in the adaptive immune system of vertebrates. They are responsible for the elimination of virus-infected cells or other abnormal cells such as some tumor cells. These cells are specifically recognized via the T-cell receptor (TCR), which interacts with the certain antigen presented via MHC (major histocompatibility complex) class I on target cells.

An exemplary CD8 amino acid sequence is represented by P01732-1 (UniParc), which is known as the canonical sequence:




 (SEQ
ID NO:2).

Numerous anti-CD8 antibodies and antigen binding fragments are known in the art and/or are available commercially, all of which may find use in the present invention.

Table 2 provides a list of various commercially-sourced anti-CD8 antibodies that may be used in the present disclosure.

**Table 2: Exemplary anti-CD8 antibodies available commercially**

| **Antibody Name/Description** | **Source** |
|---|---|
| CD8a Monoclonal Antibody (RPA-T8), PE, eBioscience^{™} | ThermoFisher Scientific Cat # 12-0088-42 |
| CD8a Monoclonal Antibody (RPA-T8), PE, eBioscience^{™} | ThermoFisher Scientific Cat # 12-0088-80 |
| CD8a Monoclonal Antibody (OKT8 (OKT-8)), Alexa Fluor 488, eBioscience^{™} | ThermoFisher Scientific Cat # 53-0086-42 |
| CD8a Monoclonal Antibody (53-6.7), eBioscience^{™} | ThermoFisher Scientific Cat # 14-0081-82 |
| CD8a Monoclonal Antibody (53-6.7), FITC, eBioscience^{™} | ThermoFisher Scientific Cat # 11-0081-82 |
| CD8a Monoclonal Antibody (53-6.7), PE, eBioscience^{™} | ThermoFisher Scientific Cat # 12-0081-82 |
| CD8a Monoclonal Antibody (53-6.7), eFluor 450, eBioscience^{™} | ThermoFisher Scientific Cat # 48-0081-82 |
| CD8a Monoclonal Antibody (53-6.7), Biotin, eBioscience^{™} | ThermoFisher Scientific Cat # 13-0081-82 |
| CD8a Monoclonal Antibody (53-6.7), Alexa Fluor 700, eBioscience^{™} | ThermoFisher Scientific Cat # 56-0081-82 |
| CD8a Monoclonal Antibody (53-6.7), PE-Cyanine5, eBioscience^{™} | ThermoFisher Scientific Cat # 15-0081-82 |
| CD8a Monoclonal Antibody (53-6.7), Alexa Fluor 488, eBioscience^{™} | ThermoFisher Scientific Cat # 53-0081-82 |
| CD8a Monoclonal Antibody (53-6.7), Functional Grade, eBioscience^{™} | ThermoFisher Scientific Cat # 16-0081-82 |
| CD8a Monoclonal Antibody (53-6.7), eFluor 660, eBioscience^{™} | ThermoFisher Scientific Cat # 50-0081-82 |
| CD8a Monoclonal Antibody (53-6.7), Alexa Fluor 532, eBioscience^{™} | ThermoFisher Scientific Cat # 58-0081-80 |
| CD8a Monoclonal Antibody (RPA-T8), PE, eBioscience^{™} | ThermoFisher Scientific Cat # 12-0088-42 |
| CD8a Monoclonal Antibody (OKT8 (OKT-8)), Alexa Fluor 488, eBioscience^{™} | ThermoFisher Scientific Cat # 12-0088-80 |
| CD8a Monoclonal Antibody (RPA-T8), APC, eBioscience^{™} | ThermoFisher Scientific Cat # 53-0086-42 |
| CD8a Monoclonal Antibody (RPA-T8), PerCP-Cyanine5.5, eBioscience^{™} | ThermoFisher Scientific Cat # 17-0088-42 |
| CD8a Monoclonal Antibody (RPA-T8), Alexa Fluor 532, eBioscience^{™} | ThermoFisher Scientific Cat # 45-0088-42 |
| CD8a Monoclonal Antibody (RPA-T8), APC-eFluor 780, eBioscience^{™} | ThermoFisher Scientific Cat # 58-0088-42 |

There are numerous anti-CD8 antibodies, including monoclonal antibodies, known in the art, including: 2D2: 4D12.1; 7B12 IG1 1 ; 8E-1.7; 8G5; 14; 21Thy; 51.1 ; 66.2; 109-2D4; 138-17; 143-44; 278F24: 302F27; AICD8.1 ; anti-T8; B9.1.1; B9.2.4; B9.3.1 ; B9.4.1 ; B9.7.6; B9.8.6; B9.1 1: B9.1 1.10: BE48: BL15, BL-TS8; BMAC8; BU88; BW135/80; C1-11G3; CIO; C12/D3; CD8-4C9: CLB-T8/1 ; CTAG-CD8, 3B5; F80-1D4D11 ; F101-87 (S-T8a); GIO-I; GlO-1.1; HI208: HI209: HI212: HIT8a: HIT8b; HIT8d; ICO-31 ; ICO-122; IP48; ITI-5C2; ITM8-1; JML-H7: JML-H8: L2: L533: Leu-2a: LT8; LY17.2E7; LY19.3B2; M236; M-T122; M-T415; M-T805: M-T806: M- T807: M-T808: M-T809; M-T1014; MCD8; MEM-31; MEM- 146; NU-Ts/c; OKT8: OKT8f: P218: RPA-T8: SM4: T8; T8 /2T8-19; T8 /2T8-2A1 ; T8 /2T8- 1B5; T8 /2T8-1C1 ; T8 /7Pt3F9: T8 /21thy2D3; T8 /21 thy; T8 /TPE3FP; T8b; T41D8; T811 ; TU68; TU102; UCHT4; VIT8; VIT8b; WuT8-l; X107; YTC141.1; and/or YTC 182.20. In addition to commercial sources, a large number of monoclonal antibodies against CD8 have been reported in the literature. For example, the anti-CD8 antibodies or fragments thereof described in the following publications are contemplated herein: AU2014249243B2: 10,746,726: 9,790,279; 9,758,581; 9,587,022; 8,877,913; 8,685,651; 8,673,304: 8,586,715: 8,440,806: 8,399,621: 7,541,443; 7,482,000; 7,452,981; 7,452,534; 7,338,658; 6,136,310: 6,056,956; 5,871,732; and 5,741,488, each of which are incorporated herein by reference in their entireties.

All of the above-noted commercially-available and anti-CD8 antibodies known in the literature can be used in the instant disclosure.

In certain other embodiments, the targeting moieties for targeting the delivery vehicles to a target cell (e.g., leukocytes) are anti-CD3 antibodies or antigen binding fragments.

CD3 antigen is associated with the T-cell receptor complex on T-cells. Multispecific antigen binding proteins having specificity to CD3 and an antigen of a target cell can trigger the cytotoxic activity of T-cells on target cells. Namely, by multispecific binding of the antigen binding protein to CD3 and to a target cell, e.g. a tumor cell, cell lysis of the target cell may be induced. Antigen binding proteins with a CD3 binding site and their production are known in the art (and described for example in Kipriyanov et al., 1999, Journal of Molecular Biology 293:41-56, Le Gall et al., 2004, Protein Engineering, Design & Selection, 17/4:357-366).

The CD3 antigen is a complex of 5 invariable polypeptide chains: γ, δ, ε, ζ and η, whose molecular weights are respectively 25-28, 21, 20, 16 and 22 kDa. The CD3 chains are clustered in a group of two invariant dimers, γ/ε and δ/ε associated with a variable dimer which consists of ζ homodimers, or ζ /η, or ζ/γ FcR heterodimers (y FcR being the γ chain of the Fc receptors), or γFcR homodimers. The CD3 is part of a bigger complex which includes the T cell receptor (TCR). CD3 complex associated with the TCR is involved in the recognition of peptides bound to the major histocompatibility complex class I and II during the immune response. T cell activation may be induced when a foreign antigen is presented to the TCR through MHC complex. The CD3 antigen is expressed by mature T lymphocytes and by a subset of thymocytes.

Numerous anti-CD3 antibodies and antigen binding fragments are known in the art and/or are available commercially, all of which may find use in the present invention.

Table 3 provides a list of various commercially-sourced anti-CD8 antibodies that may be used in the present disclosure.

**Table 3: Exemplary anti-CD3 antibodies available commercially**

| Antibody Name/Description | Source |
|---|---|
| CD3 Monoclonal Antibody (17A2), eBioscience^{™} | ThermoFisher Scientific Cat # 14-0032-82 |
| CD3 Monoclonal Antibody (17A2), eBioscience^{™} | ThermoFisher Scientific Cat # 48-0037-42 |
| CD3 Monoclonal Antibody (OKT3), Biotin, eBioscience^{™} | ThermoFisher Scientific Cat # 13-0037-82 |
| CD3 Monoclonal Antibody (OKT3), Alexa Fluor 488, eBioscience^{™} | ThermoFisher Scientific Cat # 53-0037-42 |
| CD3 Monoclonal Antibody (OKT3), Alexa Fluor 700, eBioscience^{™} | ThermoFisher Scientific Cat # 56-0037-42 |
| CD3 Monoclonal Antibody (OKT3), eFluor 660, eBioscience^{™} | ThermoFisher Scientific Cat # 50-0037-42 |
| CD3 Monoclonal Antibody (OKT3), Super Bright 600, eBioscience^{™} | ThermoFisher Scientific Cat # 63-0037-42 |
| CD3 Monoclonal Antibody (UCHT1), Alexa Fluor 532, eBioscience^{™} | ThermoFisher Scientific Cat # 58-0038-42 |
| CD3 Monoclonal Antibody (UCHT1), FITC, eBioscience^{™} | ThermoFisher Scientific Cat # 11-0038-42 |
| CD3 Monoclonal Antibody (UCHT1), FITC, eBioscience^{™} | ThermoFisher Scientific Cat # 11-0038-80 |
| CD3 Monoclonal Antibody (UCHT1), eBioscience^{™} | ThermoFisher Scientific Cat # 14-0038-82 |

Anti-CD3 antibodies, including monoclonal antibodies, are known in the art, including: those disclosed in US2018/0057593, US11007267B2, US10865251B2, US10759858B2, US10906978B2, US20210253701A1, US20200123255A1, US20210095027A1, US20210147561A1, US10544220B2, US20190284278A1, US20190263904A1, and US20200048348A1, each of which are incorporated herein by reference in their entireties.

All of the above-noted commercially-available and anti-CD3 antibodies known in the literature can be used in the instant disclosure.

In addition, the antibodies of the present disclosure which may be used as targeting moieties on the delivery vehicle used herein may also be made by any suitable conventional means. In one approach, following immunization with an antigen of interest (e.g., CD4), somatic cells with the potential for producing antibodies, specifically B lymphocytes (B cells), are selected for use in the MAb generating protocol. These cells may be obtained from biopsied spleens or lymph nodes, or from circulating blood. The antibody-producing B lymphocytes from the immunized animal are then fused with cells of an immortal myeloma cell, generally one of the same species as the animal that was immunized or human or human/mouse chimeric cells. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas). Any one of a number of myeloma cells may be used, as are known to those of skill in the art (Goding, pp. 65-66, 1986: Campbell, pp. 75-83, 1984).

### Methods for generating antibodies

Methods for generating hybrids of antibody-producing spleen or lymph node cells and myeloma cells usually comprise mixing somatic cells with myeloma cells in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter et al. (1977). The use of electrically induced fusion methods also is appropriate (Goding, pp. 71-74, 1986).

Culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays dot immunobinding assays, and the like. The selected hybridomas are then serially diluted or single-cell sorted by flow cytometric sorting and cloned into individual antibody-producing cell lines, which clones can then be propagated indefinitely to provide mAbs.

The cell lines may be exploited for MAb production in two basic ways. A sample of the hybridoma can be injected (often into the peritoneal cavity) into an animal (e.g., a mouse). Optionally, the animals are primed with a hydrocarbon, especially oils such as pristane (tetramethylpentadecane) prior to injection. When human hybridomas are used in this way, it is optimal to inject immunocompromised mice, such as SCID mice, to prevent tumor rejection. The injected animal develops tumors secreting the specific monoclonal antibody produced by the fused cell hybrid. The body fluids of the animal, such as serum or ascites fluid, can then be tapped to provide MAbs in high concentration. The individual cell lines could also be cultured in vitro, where the MAbs are naturally secreted into the culture medium from which they can be readily obtained in high concentrations. Alternatively, human hybridoma cells lines can be used in vitro to produce immunoglobulins in cell supematant. The cell lines can be adapted for growth in serum-free medium to optimize the ability to recover human monoclonal immunoglobulins of high purity.

MAbs produced by either means may be further purified, if desired, using filtration, centrifugation and various chromatographic methods such as FPLC or affinity chromatography. Fragments of the monoclonal antibodies of the disclosure can be obtained from the purified monoclonal antibodies by methods which include digestion with enzymes, such as pepsin or papain, and/or by cleavage of disulfide bonds by chemical reduction. Alternatively, monoclonal antibody fragments encompassed by the present disclosure can be synthesized using an automated peptide synthesizer.

It also is contemplated that a molecular cloning approach may be used to generate monoclonals. For this, RNA can be isolated from the hybridoma line and the antibody genes obtained by RT-PCR and cloned into an immunoglobulin expression vector. Alternatively, combinatorial immunoglobulin phagemid libraries are prepared from RNA isolated from the cell lines and phagemids expressing appropriate antibodies are selected by panning using viral antigens. The advantages of this approach over conventional hybridoma techniques are that many more antibodies can be produced and screened in a single round, and that new specificities are generated by H and L chain combination which further increases the chance of finding appropriate antibodies.

U.S. patents, each incorporated herein by reference, that teach the production of antibodies useful in the present disclosure include U.S. Patent 5,565,332, which describes the production of chimeric antibodies using a combinatorial approach; U.S. Patent 4,816,567 which describes recombinant immunoglobulin preparations; and U.S. Patent 4,867,973 which describes antibody-therapeutic agent conjugates.

### Combinations

In one embodiment, the invention provides a combination of T cell targeted delivery vehicles, targeting two or more T cell antigens. In one embodiment, the two or more T cell antigens are selected from CD1, CD2, CD3, CD4, CD5, CD7, CD8, CD16, CD25, CD26, CD27, CD28, CD30, CD38, CD39, CD40L, CD44, CD45, CD62L, CD69, CD73, CD80, CD83, CD86, CD95, CD103, CD119, CD126, CD150, CD153, CD154, CD161, CD183, CD223, CD254, CD275, CD45RA, CXCR3, CXCR5, FasL, IL18R1, CTLA-4, OX40, GITR LAG3, ICOS, PD-1, leu-12, TCR, TLR1, TLR2, TLR3, TLR4, TLR6, NKG2D, CCR, CCR1, CCR2, CCR4, CCR6, or CCR7. In one embodiment, the combination comprises one or more T cell targeted delivery vehicles, targeting a surface antigen of a CD4+ T cell and a surface antigen of a CD8+ T cell. In one embodiment, the combination comprises two or more T cell targeted delivery vehicles, targeting CD4 and CD8.

In one embodiment, the combination of T cell targeted delivery vehicles delivers the same agent to T cells expressing different surface antigens. In one embodiment, the combination of T cell targeted delivery vehicles delivers a first agent to one subset of T cells expressing a first surface antigen and a second, different agent to a second subset of T cells expressing a second surface antigen. Therefore, in various embodiments, the combinations of the invention can be used deliver 1, 2, 3, 4, 5, 6, 7, 8, 9, 10 or more than 10 different agents to T cells.

In one embodiment, the composition of the present invention comprises a combination of agents described herein. In certain embodiments, a composition comprising a combination of agents described herein has an additive effect, wherein the overall effect of the combination is approximately equal to the sum of the effects of each individual agent. In other embodiments, a composition comprising a combination of agents described herein has a synergistic effect, wherein the overall effect of the combination is greater than the sum of the effects of each individual agent.

A composition comprising a combination of agents comprises individual agents in any suitable ratio. For example, in one embodiment, the composition comprises a 1:1 ratio of two individual agents. However, the combination is not limited to any particular ratio. Rather any ratio that is shown to be effective is encompassed.

### Conjugation

In various embodiments of the invention, the delivery vehicle is conjugated to a targeting domain. Exemplary methods of conjugation can include, but are not limited to, covalent bonds, electrostatic interactions, and hydrophobic ("van der Waals") interactions. In one embodiment, the conjugation is a reversible conjugation, such that the delivery vehicle can be disassociated from the targeting domain upon exposure to certain conditions or chemical agents. In another embodiment, the conjugation is an irreversible conjugation, such that under normal conditions the delivery vehicle does not dissociate from the targeting domain.

In some embodiments, the conjugation comprises a covalent bond between an activated polymer conjugated lipid and the targeting domain. The term "activated polymer conjugated lipid" refers to a molecule comprising a lipid portion and a polymer portion that has been activated via functionalization of a polymer conjugated lipid with a first coupling group. In one embodiment, the activated polymer conjugated lipid comprises a first coupling group capable of reacting with a second coupling group. In one embodiment, the activated polymer conjugated lipid is an activated pegylated lipid. In one embodiment, the first coupling group is bound to the lipid portion of the pegylated lipid. In another embodiment, the first coupling group is bound to the polyethylene glycol portion of the pegylated lipid. In one embodiment, the second functional group is covalently attached to the targeting domain.

The first coupling group and second coupling group can be any functional groups known to those of skill in the art to together form a covalent bond, for example under mild reaction conditions or physiological conditions. In some embodiments, the first coupling group or second coupling group are selected from the group consisting of maleimides, N-hydroxysuccinimide (NHS) esters, carbodiimides, hydrazide, pentafluorophenyl (PFP) esters, phosphines, hydroxymethyl phosphines, psoralen, imidoesters, pyridyl disulfide, isocyanates, vinyl sulfones, alpha-haloacetyls, aryl azides, acyl azides, alkyl azides, diazirines, benzophenone, epoxides, carbonates, anhydrides, sulfonyl chlorides, cyclooctyne, aldehydes, and sulfhydryl groups. In some embodiments, the first coupling group or second coupling group is selected from the group consisiting of free amines (-NH₂), free sulfhydryl groups (-SH), free hydroxide groups (-OH), carboxylates, hydrazides, and alkoxyamines. In some embodiments, the first coupling group is a functional group that is reactive toward sulfhydryl groups, such as maleimide, pyridyl disulfide, or a haloacetyl. In one embodiment, the first coupling group is a maleimide.

In one embodiment, the second coupling group is a sulfhydryl group. The sulfhydryl group can be installed on the targeting domain using any method known to those of skill in the art. In one embodiment, the sulfhydryl group is present on a free cysteine residue. In one embodiment, the sulfhydryl group is revealed via reduction of a disulfide on the targeting domain, such as through reaction with 2-mercaptoethylamine. In one embodiment, the sulfhydryl group is installed via a chemical reaction, such as the reaction between a free amine and 2-iminothilane or N-succinimidyl S-acetylthioacetate (SATA).

In some embodiments, the polymer conjugated lipid and targeting domain are functionalized with groups used in "click" chemistry. Bioorthogonal "click" chemistry comprises the reaction between a functional group with a 1,3-dipole, such as an azide, a nitrile oxide, a nitrone, an isocyanide, and the link, with an alkene or an alkyne dipolarophiles. Exemplary dipolarophiles include any strained cycloalkenes and cycloalkynes known to those of skill in the art, including, but not limited to, cyclooctynes, dibenzocyclooctynes, monofluorinated cyclcooctynes, difluorinated cyclooctynes, and biarylazacyclooctynone.

### Peptide targeting moiety

In one embodiment, the targeting domain of the invention comprises a peptide. In certain embodiments, the peptide targeting domain specifically binds to a target of interest.

The peptide of the present invention may be made using chemical methods. For example, peptides can be synthesized by solid phase techniques (Roberge J Y et al (1995) Science 269: 202-204), cleaved from the resin, and purified by preparative high performance liquid chromatography. Automated synthesis may be achieved, for example, using the ABI 431 A Peptide Synthesizer (Perkin Elmer) in accordance with the instructions provided by the manufacturer.

The peptide may alternatively be made by recombinant means or by cleavage from a longer polypeptide. The composition of a peptide may be confirmed by amino acid analysis or sequencing.

The variants of the peptides according to the present invention may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the peptide is an alternative splice variant of the peptide of the present invention, (iv) fragments of the peptides and/or (v) one in which the peptide is fused with another peptide, such as a leader or secretory sequence or a sequence which is employed for purification (for example, His-tag) or for detection (for example, Sv5 epitope tag). The fragments include peptides generated via proteolytic cleavage (including multi-site proteolysis) of an original sequence. Variants may be post-translationally, or chemically modified. Such variants are deemed to be within the scope of those skilled in the art from the teaching herein.

As known in the art the "similarity" between two peptides is determined by comparing the amino acid sequence and its conserved amino acid substitutes of one peptide to a sequence of a second peptide. Variants are defined to include peptide sequences different from the original sequence, preferably different from the original sequence in less than 40% of residues per segment of interest, more preferably different from the original sequence in less than 25% of residues per segment of interest, more preferably different by less than 10% of residues per segment of interest, most preferably different from the original protein sequence in just a few residues per segment of interest and at the same time sufficiently homologous to the original sequence to preserve the functionality of the original sequence. The present invention includes amino acid sequences that are at least 60%, 65%, 70%, 72%, 74%, 76%, 78%, 80%, 90%, or 95% similar or identical to the original amino acid sequence. The degree of identity between two peptides is determined using computer algorithms and methods that are widely known for the persons skilled in the art. The identity between two amino acid sequences is preferably determined by using the BLASTP algorithm [BLAST Manual, Altschul, S., et al., NCBI NLM NIH Bethesda, Md. 20894, Altschul, S., et al., J. Mol. Biol. 215: 403-410 (1990)].

The peptides of the invention can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Some modifications or processing events require introduction of additional biological machinery. For example, processing events, such as signal peptide cleavage and core glycosylation, are examined by adding canine microsomal membranes or Xenopus egg extracts (U.S. Pat. No. 6,103,489) to a standard translation reaction.

The peptides of the invention may include unnatural amino acids formed by post-translational modification or by introducing unnatural amino acids during translation.

### Nucleic acid targeting moiety

In one embodiment, the targeting domain of the invention comprises an isolated nucleic acid, including for example a DNA oligonucleotide and a RNA oligonucleotide. In certain embodiments, the nucleic acid targeting domain specifically binds to a target of interest. For example, in one embodiment, the nucleic acid comprises a nucleotide sequence that specifically binds to a target of interest.

The nucleotide sequences of a nucleic acid targeting domain can alternatively comprise sequence variations with respect to the original nucleotide sequences, for example, substitutions, insertions and/or deletions of one or more nucleotides, with the condition that the resulting nucleic acid functions as the original and specifically binds to the target of interest.

In the sense used in this description, a nucleotide sequence is "substantially homologous" to any of the nucleotide sequences describe herein when its nucleotide sequence has a degree of identity with respect to the nucleotide sequence of at least 60%, advantageously of at least 70%, preferably of at least 85%, and more preferably of at least 95%. Other examples of possible modifications include the insertion of one or more nucleotides in the sequence, the addition of one or more nucleotides in any of the ends of the sequence, or the deletion of one or more nucleotides in any end or inside the sequence. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215: 403-410 (1990)].

### Antibody targeting moiety

In one embodiment, the targeting domain of the invention comprises an antibody, or antibody fragment. In certain embodiments, the antibody targeting domain specifically binds to a target of interest. Such antibodies include polyclonal antibodies, monoclonal antibodies, Fab and single chain Fv (scFv) fragments thereof, bispecific antibodies, heteroconjugates, human and humanized antibodies.

The antibodies may be intact monoclonal or polyclonal antibodies, and immunologically active fragments (e.g., a Fab or (Fab)2 fragment), an antibody heavy chain, an antibody light chain, humanized antibodies, a genetically engineered single chain Fv molecule (Ladner et al, U.S. Pat. No. 4,946,778), or a chimeric antibody, for example, an antibody which contains the binding specificity of a murine antibody, but in which the remaining portions are of human origin. Antibodies including monoclonal and polyclonal antibodies, fragments and chimeras, may be prepared using methods known to those skilled in the art.

Such antibodies may be produced in a variety of ways, including hybridoma cultures, recombinant expression in bacteria or mammalian cell cultures, and recombinant expression in transgenic animals. The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity.

In one embodiment, the targeting domain of the instant invention is an antibody that specifically binds to a surface antigen of a CD4⁺ T cell. In one embodiment, the targeting domain of the instant invention is an antibody that specifically binds to CD4.

### T cells

In various embodiments, the target of the delivery vehicles can be any type of cell in the body (i.e., "target cells"). In preferred embodiments, target cells are immune cells, such as, but not limited to, any class of myeloid cell (e.g., neutrophils, eosinophils, mast cells, basophils, and monocytes) or any class of lymphocyte (e.g., T cells (e.g., cytotoxic T cells, helper T cells, or memory T cells), B cells (e.g., plasma cells and memory B cells), and natural killer cells).

In some embodiments, T cells that can be targeted using the compositions of the invention immunostimulatory cells, i.e., cells that mediate an immune response. In some embodiments, T cells that can be targeted using the compositions of the invention can include, but are not limited to, T helper cells (CD4+) and CD4+ cytotoxic T cells (also referred to as cytotoxic T lymphocytes, CD4+ CTL). In certain embodiments, the target cells are T cells. In some embodiments, T cells that can be targeted using the compositions of the invention can be CD4+ or CD8+ and can include, but are not limited to, T helper cells (CD4+), cytotoxic T cells (also referred to as cytotoxic T lymphocytes, CTL: CD8- T cells), and memory T cells, including central memory T cells (TCM), stem memory T cells (TSCM), stem-cell-like memory T cells (or stem-like memory T cells), and effector memory T cells, for example, TEM cells and TEMRA (CD45RA+) cells, effector T cells, Th1 cells, Th2 cells, Th9 cells, Th17 cells, Th22 cells, Tfh (follicular helper) cells, T regulatory cells, natural killer T cells, mucosal associated invariant T cells (MAIT), and γδ T cells. Major T cell subtypes include TN (naive), TSCM (stem cell memory), TCM (central memory), TTM (Transitional Memory), TEM (Effector memory), and TTE (Terminal Effector), TCR-transgenic T cells, T-cells redirected for universal cytokine-mediated killing (TRUCK), Tumor infiltrating T cells (TIL), CAR-T cells or any T cell that can be used for treating a disease or disorder. In some embodiments, the T cells are CD4+ T cells.

### Therapeutic Methods

The present invention provides methods of delivering at least one agent for diagnosis, treatment or prevention of a disease, or a disease or disorder to a CD4⁺ T cell. In one embodiment, the CD4+ T cell targeted delivery vehicle comprises or encapsulates an agent to be administered to a subject. In some embodiments, the agent is a nucleoside modified mRNA. The present invention therefore provides methods of delivering at least one agent to a CD4⁺ T cell.

In one embodiment, the CD4+ T cell targeted delivery vehicle comprises or encapsulates a therapeutic agent for the treatment of a disease or disorder. In some embodiments, the therapeutic agent is a nucleoside modified mRNA. The present invention therefore provides methods of delivering at least one therapeutic agent to a CD4⁺ T cell. In certain embodiments, the method is used to treat or prevent a disease or disorder in a subject. Exemplary diseases or disorders include, but are not limited to, cancers, infectious diseases, and immunological disorders.

The following are non-limiting examples of cancers that can be treated or prevented by the disclosed methods: acute lymphoblastic leukemia, acute myeloid leukemia, adrenocortical carcinoma, appendix cancer, basal cell carcinoma, bile duct cancer, bladder cancer, bone cancer, brain and spinal cord tumors, brain stem glioma, brain tumor, breast cancer, bronchial tumors, burkitt lymphoma, carcinoid tumor, central nervous system atypical teratoid/rhabdoid tumor, central nervous system embryonal tumors, central nervous system lymphoma, cerebellar astrocytoma, cerebral astrocytoma/malignant glioma, cerebral astrocytotna/malignant glioma, cervical cancer, childhood visual pathway tumor, chordoma, chronic lymphocytic leukemia, chronic myelogenous leukemia, chronic myeloproliferative disorders, colon cancer, colorectal cancer, craniopharyngioma, cutaneous cancer, cutaneous t-cell lymphoma, endometrial cancer, ependymoblastoma, ependymoma, esophageal cancer, ewing family of tumors, extracranial cancer, extragonadal germ cell tumor, extrahepatic bile duct cancer, extrahepatic cancer, eye cancer, fungoides, gallbladder cancer, gastric (stomach) cancer, gastrointestinal cancer, gastrointestinal carcinoid tumor, gastrointestinal stromal tumor (gist), germ cell tumor, gestational cancer, gestational trophoblastic tumor, glioblastoma, glioma, hairy cell leukemia, head and neck cancer, hepatocellular (liver) cancer, histiocytosis, hodgkin lymphoma, hypopharyngeal cancer, hypothalamic and visual pathway glioma, hypothalamic tumor, intraocular (eye) cancer, intraocular melanoma, islet cell tumors, kaposi sarcoma, kidney (renal cell) cancer, langerhans cell cancer, langerhans cell histiocytosis, laryngeal cancer, leukemia, lip and oral cavity cancer, liver cancer, lung cancer, lymphoma, macroglobulinemia, malignant fibrous histiocytoma of bone and osteosarcoma, medulloblastoma, medulloepithelioma, melanoma, merkel cell carcinoma, mesothelioma, metastatic squamous neck cancer with occult primary, mouth cancer, multiple endocrine neoplasia syndrome, multiple myeloma, mycosis, myelodysplastic syndromes, myelodysplastic/myeloproliferative diseases, myelogenous leukemia, myeloid leukemia, myeloma, myeloproliferative disorders, nasal cavity and paranasal sinus cancer, nasopharyngeal cancer, neuroblastoma, non-hodgkin lymphoma, non-small cell lung cancer, oral cancer, oral cavity cancer, oropharyngeal cancer, osteosarcoma and malignant fibrous histiocytoma, osteosarcoma and malignant fibrous histiocytoma of bone, ovarian, ovarian cancer, ovarian epithelial cancer, ovarian germ cell tumor, ovarian low malignant potential tumor, pancreatic cancer, papillomatosis, paraganglioma, parathyroid cancer, penile cancer, pharyngeal cancer, pheochromocytoma, pineal parenchymal tumors of intermediate differentiation, pineoblastoma and supratentorial primitive neuroectodermal tumors, pituitary tumor, plasma cell neoplasm, plasma cell neoplasm/multiple myeloma, pleuropulmonary blastoma, primary central nervous system cancer, primary central nervous system lymphoma, prostate cancer, rectal cancer, renal cell (kidney) cancer, renal pelvis and ureter cancer, respiratory tract carcinoma involving the nut gene on chromosome 15, retinoblastoma, rhabdomyosarcoma, salivary gland cancer, sarcoma, sezary syndrome, skin cancer (melanoma), skin cancer (nonmelanoma), skin carcinoma, small cell lung cancer, small intestine cancer, soft tissue cancer, soft tissue sarcoma, squamous cell carcinoma, squamous neck cancer , stomach (gastric) cancer, supratentorial primitive neuroectodermal tumors, supratentorial primitive neuroectodermal tumors and pineoblastoma, T-cell lymphoma, testicular cancer, throat cancer, thymoma and thymic carcinoma, thyroid cancer, transitional cell cancer, transitional cell cancer of the renal pelvis and ureter, trophoblastic tumor, urethral cancer, uterine cancer, uterine sarcoma, vaginal cancer, visual pathway and hypothalamic glioma, vulvar cancer, waldenstrom macroglobulinemia, and wilms tumor.

In some embodiments, the present invention features methods for treating or preventing autoimmune diseases, including, but not limited to, rheumatoid arthritis/seronegative arthropathies, osteoarthritis, inflammatory bowel disease, systemic lupus erythematosis, iridoeyelitis/uveitistoptic neuritis, idiopathic pulmonary fibrosis, systemic vasculitis/Wegener's gramilornatosis, sarcoidosis, including, but not limited to, rheumatoid arthritis/seronegative arthropathies, osteoarthritis, inflammatory bowel disease, systemic lupus erythematosis, iridoeyelitis/uveitistoptic neuritis, idiopathic pulmonary fibrosis, systemic vasculitis/Wegener's gramilornatosis, sarcoidosis, myocarditis, postmyocardial infarction syndrome, postpericardiotomy syndrome, subacute bacterial endocarditis (SBE), anti-glomerular basement membrane nephritis, interstitial cystitis, lupus nephritis, autoimmune hepatitis, primary biliary cholangitis(PBC), primary sclerosing cholangitis, antisynthetase syndrome, alopecia areata, autoimmune angioedema, autoimmune progesterone dermatitis, autoimmune urticaria, bullous pemphigoid, cicatricial pemphigoid, dermatitis herpetiformis, discoid lupus erythematosus, epidermolysis bullosa acquisita, erythema nodosum, gestational pemphigoid, hidradenitis suppurativa, lichen planus, lichen sclerosus, linear IgA disease (LAD), morphea, pemphigus vulgaris, pityriasis lichenoides et varioliformis acuta, Mucha-Habermann disease, psoriasis, systemic sclerodenna, vitiligo, Addison's disease, autoimmune polyendocrine syndrome (APS) type 1, autoimmune polyendocrine syndrome (APS) type 2, autoimmune polyendocrine syndrome (APS) type 3, autoimmune pancreatitis (AIP), diabetes mellitus type 1, autoimmune thyroiditis, Ord's thyroiditis, Graves' disease, autoimmune oophoritis, endometriosis, autoimmune orchitis, Sjogren's syndrome, autoimmune enteropathy, Coeliac disease, Crohn's disease, microscopic colitis, ulcerative colitis, antiphospholipid syndrome(APS, APLS), aplastic anemia, autoimmune hemolytic anemia, autoimmune lymphoproliferative syndrome, autoimmune neutropenia, autoimmune thrombocytopenic purpura, cold agglutinin disease, essential mixed cryoglobulinemia, Evans syndrome, pernicious anemia, pure red cell aplasia, thrombocytopenia, adiposis dolorosa, adult-onset Still's disease, ankylosing spondylitis, CREST syndrome, drug-induced lupus, enthesitis-related arthritis, eosinophilic fasciitis Felty syndrome, IgG4-related disease, juvenile arthritis, Lyme disease (chronic), mixed connective tissue disease (MCTD), palindromic rheumatism, Parry Romberg syndrome, Parsonage-Turner syndrome, psoriatic arthritis, reactive arthritis, relapsing polychondritis, retroperitoneal fibrosis, rheumatic fever, Schnitzler syndrome, undifferentiated connective tissue disease (UCTD), dermatomyositis, fibromyalgia, inclusion body myositis, myositis, myasthenia gravis, neuromyotonia, paraneoplastic cerebellar degeneration, polymyositis, acute disseminated encephalomyelitis (ADEM), acute motor axonal neuropathy, anti-N-methyl-D-aspartate (Anti-NMDA) receptor encephalitis, balo concentric sclerosis, Bickerstaff's encephalitis, chronic inflammatory demyelinating polyneuropathy, Guillain-Barré syndrome, Hashimoto's encephalopathy, idiopathic inflammatory demyelinating diseases, Lambert-Eaton myasthenic syndrome, multiple sclerosis, pattern II, Oshtoran Syndrome, pediatric autoimmune neuropsychiatric disorder associated with streptococcus (PANDAS), progressive inflammatory neuropathy, restless leg syndrome, stiff person syndrome, sydenham chorea, transverse myelitis, autoimmune retinopathy, autoimmune uveitis, Cogan syndrome, Graves ophthalmopathy, intermediate uveitis, ligneous conjunctivitis, Mooren's ulcer, neuromyelitis optica, opsoclonus myoclonus syndrome, optic neuritis, scleritis, Susac's syndrome, sympathetic ophthalmia, Tolosa-Hunt syndrome, autoimmune inner ear disease(AIED), Ménière's disease, Behçet's disease, eosinophilic granulomatosis with polyangiitis (EGPA), giant cell arteritis, granulomatosis with polyangiitis (GPA), IgA vasculitis (IgAV), Kawasaki's disease, leukocytoclastic vasculitis, lupus vasculitis, rheumatoid vasculitis, microscopic polyangiitis (MPA), polyarteritis nodosa (PAN), polymyalgia rheumatic, urticarial vasculitis, vasculitis, and primary immune deficiency.

In some embodiments, the therapeutic agent is an agent for the treatment or prevention of an infection or an infectious disease. In one embodiment, the therapeutic agent is an agent for the treatment or prevention of a bacterial infection or a disease or disorder associated therewith. The bacterium can be from any one of the following phyla: Acidobacteria, Actinobacteria, Aquificae, Bacteroidetes, Caldiserica, Chlamydiae, Chlorobi, Chloroflexi, Chrysiogenetes, Cyanobacteria, Deferribacteres, Deinococcus-Thermus, Dictyoglomi, Elusimicrobia, Fibrobacteres, Firmicutes, Fusobacteria, Gemmatimonadetes, Lentisphaerae, Nitrospira, Planctomycetes, Proteobacteria, Spirochaetes, Synergistetes, Tenericutes, Thermodesulfobacteria, Thermotogae, and Verrucomicrobia.

The bacterium can be a gram-positive bacterium or a gram-negative bacterium. The bacterium can be an aerobic bacterium or an anerobic bacterium. The bacterium can be an autotrophic bacterium or a heterotrophic bacterium. The bacterium can be a mesophile, a neutrophile, an extremophile, an acidophile, an alkaliphile, a thermophile, a psychrophile, a halophile, or an osmophile.

The bacterium can be an anthrax bacterium, an antibiotic resistant bacterium, a disease-causing bacterium, a food poisoning bacterium, an infectious bacterium, Salmonella bacterium, Staphylococcus bacterium, Streptococcus bacterium, or tetanus bacterium. The bacterium can be a mycobacteria, Clostridium tetani, Yersinia pestis, Bacillus anthracis, methicillin-resistant Staphylococcus aureus (MRSA), or Clostridium difficile.

In one embodiment, the therapeutic agent is an agent for the treatment or prevention of a viral infection, or a disease or disorder associated therewith. In some embodiments, the virus is from one of the following families: Adenoviridae, Arenaviridae, Bunyaviridae, Caliciviridae, Coronaviridae (including SARS and SARS-CoV-2), Filoviridae, Hepadnaviridae, Herpesviridae, Orthomyxoviridae, Papovaviridae, Paramyxoviridae, Parvoviridae, Picornaviridae, Poxviridae, Reoviridae, Retroviridae, Rhabdoviridae, or Togaviridae. The viral antigen can be from human immunodeficiency virus (HIV), Chikungunya virus (CHIKV), dengue fever virus, papilloma viruses, for example, human papillomoa virus (HPV), polio virus, hepatitis viruses, for example, hepatitis A virus (HAV), hepatitis B virus (HBV), hepatitis C virus (HCV), hepatitis D virus (HDV), and hepatitis E virus (HEV), smallpox virus (Variola major and minor), vaccinia virus, influenza virus, rhinoviruses, equine encephalitis viruses, rubella virus, yellow fever virus, Norwalk virus, hepatitis A virus, human T-cell leukemia virus (HTLV-I), hairy cell leukemia virus (HTLV-II), California encephalitis virus, Hanta virus (hemorrhagic fever), rabies virus, Ebola fever virus, Marburg virus, measles virus, mumps virus, respiratory syncytial virus (RSV), herpes simplex 1 (oral herpes), herpes simplex 2 (genital herpes), herpes zoster (varicella-zoster, a.k.a., chickenpox), cytomegalovirus (CMV), for example human CMV, Epstein-Barr virus (EBV), flavivirus, foot and mouth disease virus, lassa virus, arenavirus, or a cancer causing virus.

In one embodiment, the therapeutic agent is an agent for the treatment or prevention of a parasitic infection, or a disease or disorder associated therewith. In some embodiments, the parasite is a protozoa, helminth, or ectoparasite. The helminth (i.e., worm) can be a flatworm (e.g., flukes and tapeworms), a thorny-headed worm, or a round worm (e.g., pinworms). The ectoparasite can be lice, fleas, ticks, and mites.

The parasite can be any parasite causing any one of the following diseases: Acanthamoeba keratitis, Amoebiasis, Ascariasis, Babesiosis, Balantidiasis, Baylisascariasis, Chagas disease, Clonorchiasis, Cochliomyia, Cryptosporidiosis, Diphyllobothriasis, Dracunculiasis, Echinococcosis, Elephantiasis, Enterobiasis, Fascioliasis, Fasciolopsiasis, Filariasis, Giardiasis, Gnathostomiasis, Hymenolepiasis, Isosporiasis, Katayama fever, Leishmaniasis, Lyme disease, Malaria, Metagonimiasis, Myiasis, Onchocerciasis, Pediculosis, Scabies, Schistosomiasis, Sleeping sickness, Strongyloidiasis, Taeniasis, Toxocariasis, Toxoplasmosis, Trichinosis, and Trichuriasis.

The parasite can be Acanthamoeba, Anisakis, Ascaris lumbricoides, Botfly, Balantidium coli, Bedbug, Cestoda (tapeworm), Chiggers, Cochliomyia hominivorax, Entamoeba histolytica, Fasciola hepatica, Giardia lamblia, Hookworm, Leishmania, Linguatula serrata, Liver fluke, Loa loa, Paragonimus - lung fluke, Pinworm, Plasmodium falciparum, Schistosoma, Strongyloides stercoralis, Mite, Tapeworm, Toxoplasma gondii, Trypanosoma, Whipworm, or Wuchereria bancrofti.

In one embodiment, the therapeutic agent is an agent for the treatment or prevention of a fungal infection, or a disease or disorder associated therewith. In some embodiments, the fungus is Aspergillus species, Blastomyces dermatitidis, Candida yeasts (e.g., Candida albicans), Coccidioides, Cryptococcus neoformans, Cryptococcus gattii, dermatophyte, Fusarium species, Histoplasma capsulatum, Mucoromycotina, Pneumocystis jirovecii, Sporothrix schenckii, Exserohilum, or Cladosporium.

By way of a non-limiting example, in one embodiment, the invention provides a CD4+ T cell-targeted delivery vehicle comprising or encapsulating a nucleoside-modified 1086C Env mRNA, encoding the clade C transmitted/founder human immunodeficiency virus (HIV)-1 envelope (Env) 1086C, for the treatment or prevention of HIV infection or a disease or disorder associated therewith.

It will be appreciated by one of skill in the art, when armed with the present disclosure including the methods detailed herein, that the invention is not limited to treatment of diseases or disorders that are already established. Particularly, the disease or disorder need not have manifested to the point of detriment to the subject; indeed, the disease or disorder need not be detected in a subject before treatment is administered. That is, significant signs or symptoms of diseases or disorders do not have to occur before the present invention may provide benefit. Therefore, the present invention includes a method for preventing diseases or disorders, in that a composition, as discussed previously elsewhere herein, can be administered to a subject prior to the onset of diseases or disorders, thereby preventing diseases or disorders.

One of skill in the art, when armed with the disclosure herein, would appreciate that the prevention of a disease or disorder, encompasses administering to a subject a composition as a preventative measure against the development of, or progression of, a disease or disorder. As more fully discussed elsewhere herein, methods of modulating the level or activity of a gene, or gene product, encompass a wide plethora of techniques for modulating not only the level and activity of polypeptide gene products, but also for modulating expression of a nucleic acid, including either transcription, translation, or both.

The invention encompasses delivery of a delivery vehicle, comprising at least one agent, conjugated to a targeting domain. To practice the methods of the invention: the skilled artisan would understand, based on the disclosure provided herein, how to formulate and administer the appropriate composition to a subject. The present invention is not limited to any particular method of administration or treatment regimen.

One of skill in the art will appreciate that the compositions of the invention can be administered singly or in any combination. Further, the compositions of the invention can be administered singly or in any combination in a temporal sense, in that they may be administered concurrently, or before, and/or after each other. One of ordinary skill in the art will appreciate, based on the disclosure provided herein, that the compositions of the invention can be used to prevent or to treat a disease or disorder, and that a composition can be used alone or in any combination with another composition to affect a therapeutic result. In various embodiments, any of the compositions of the invention described herein can be administered alone or in combination with other modulators of other molecules associated with diseases or disorders.

Administration of the compositions of the invention (e.g., the delivery vehicles) to a human patient can be by any route, including but not limited to intravenous, intranodal, intradermal, transdermal, subcutaneous, intramuscular, inhalation (e.g., via an aerosol), buccal (e.g., sub-lingual), topical (i.e., both skin and mucosal surfaces, including airway surfaces), intrathecal, intraarticular, intraplural, intracerebral, intra-arterial, intraperitoneal, oral, intralymphatic, intranasal, rectal or vaginal administration, by perfusion through a regional catheter, or by direct intralesional injection. In one embodiment, the compositions of the invention (e.g. the delivery vehicles) are administered by intravenous push or intravenous infusion given over defined period (e.g., 0.5 to 2 hours). The compositions of the invention can be delivered by peristaltic means or in the form of a depot, although the most suitable route in any given case will depend, as is well known in the art, on such factors as the species, age, gender and overall condition of the subject, the nature and severity of the condition being treated and/or on the nature of the particular composition (i.e., dosage, formulation) that is being administered. In particular embodiments, the route of administration is via bolus or continuous infusion over a period of time, once or twice a week. In other particular embodiments, the route of administration is by subcutaneous injection given in one or more sites (e.g. thigh, waist, buttocks, arm), optionally once or twice weekly. In one embodiment, the compositions, and/or methods of the invention are administered on an outpatient basis.

In one embodiment, the invention includes a method comprising administering a combination of compositions described herein. In certain embodiments, the method has an additive effect, wherein the overall effect of the administering a combination of compositions is approximately equal to the sum of the effects of administering each individual inhibitor. In other embodiments, the method has a synergistic effect, wherein the overall effect of administering a combination of compositions is greater than the sum of the effects of administering each individual composition.

The method comprises administering a combination of composition in any suitable ratio. For example, in one embodiment, the method comprises administering two individual compositions at a 1:1 ratio. However, the method is not limited to any particular ratio. Rather any ratio that is shown to be effective is encompassed.

### Pharmaceutical Compositions

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the description of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, mammals including commercially relevant mammals such as non-human primates, cattle, pigs, horses, sheep, cats, and dogs.

Pharmaceutical compositions that are useful in the methods of the invention may be prepared, packaged, or sold in formulations suitable for ophthalmic, oral, rectal, vaginal, parenteral, topical, pulmonary, intranasal, buccal, intravenous, intracerebroventricular, intradermal, intramuscular, or another route of administration. Other contemplated formulations include projected nanoparticles, liposomal preparations, resealed erythrocytes containing the active ingredient, and immunogenic-based formulations.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient.

In addition to the active ingredient, a pharmaceutical composition of the invention may further comprise one or more additional pharmaceutically active agents.

Controlled- or sustained-release formulations of a pharmaceutical composition of the invention may be made using conventional technology.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, intraocular, intravitreal, subcutaneous, intraperitoneal, intramuscular, intradermal, intrasternal injection, intratumoral, intravenous, intracerebroventricular and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (i.e., powder or granular) form for reconstitution with a suitable vehicle (e.g., sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt.

A pharmaceutical composition of the invention may be prepared, packaged, or sold in a formulation suitable for pulmonary administration via the buccal cavity. Such a formulation may comprise dry particles which comprise the active ingredient and which have a diameter in the range from about 0.5 to about 7 nanometers, and preferably from about 1 to about 6 nanometers. Such compositions are conveniently in the form of dry powders for administration using a device comprising a dry powder reservoir to which a stream of propellant may be directed to disperse the powder or using a self-propelling solvent/powder-dispensing container such as a device comprising the active ingredient dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than 1 nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic or solid anionic surfactant or a solid diluent (preferably having a particle size of the same order as particles comprising the active ingredient).

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (i.e., powder or granular) form for reconstitution with a suitable vehicle (e.g., sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other parentally-administrable formulations that are useful include those that comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

As used herein, "additional ingredients" include, but are not limited to, one or more of the following: excipients; surface active agents; dispersing agents: inert diluents: granulating and disintegrating agents; binding agents; lubricating agents: sweetening agents: flavoring agents: coloring agents; preservatives; physiologically degradable compositions such as gelatin: aqueous vehicles and solvents; oily vehicles and solvents: suspending agents: dispersing or wetting agents; emulsifying agents, demulcents; buffers; salts; thickening agents: fillers: emulsifying agents; antioxidants; antibiotics; antifungal agents; stabilizing agents: and pharmaceutically acceptable polymeric or hydrophobic materials. Other "additional ingredients" which may be included in the pharmaceutical compositions of the invention are known in the art and described, for example in Remington's Pharmaceutical Sciences (1985, Genaro, ed., Mack Publishing Co., Easton, PA), which is incorporated herein by reference.

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the present invention and practice the claimed methods. The following working examples therefore are not to be construed as limiting in any way the remainder of the disclosure.

### Example 1: Highly efficient CD4+ T cell targeting and genetic recombination using engineered CD4+ T cell-homing mRNA-LNPs

mRNA-based therapeutics offer numerous advantages that address challenges with the current protein- or viral-based immunotherapy approaches, such as difficult manufacturing, instability, lack of control over amount and duration of expression, high toxicity, and in some cases, genomic integration or off-site effects (Goswami et al., 2019, Front. Oncol, 9:297; Das et al., 2015, J. Cell. Physiol, 230:259-271: Chames et al., 2009, Br. J. Pharmacol, 157:220-233). Efficient *in vivo* delivery has been the key obstacle in development of mRNA-based immunodierapeutics. To date, T cell modification for clinical application has required extraction of autologous T cells, expansion, and genomic editing *ex vivo,* which is expensive and time-consuming, and precludes widespread use for more common diseases, such as HIV or sickle cell anemia. T cells are known as hard-to-transfect cells (Peer et al., 2010, Journal of Controlled Release, 148:63-68; Gust et al., 2008, Cell Commun Signal, 6:3: Goffinet et al., 2006, The FASEB Journal, 20:500-502). Here, it is demonstrated that targeting human T cells with an anti-CD4 antibody-conjugated Luc mRNA containing LNP, but not with control IgG-conjugated LNP, resulted in strong binding and luciferase expression in human CD4+ T cells in a dose-dependent manner (Figure 1A through Figure 1C). Similarly, when injected systemically into C57BL/6 mice, anti-mouse CD4/mRNA-LNP specifically accumulated and the mRNA was translated in T cell-enriched tissues, such as spleen and lymph nodes (Figure 3 and Figure 4A through Figure 4C). Furthermore, the potential of the T cell-targeted mRNA-LNP system to mediate genome editing using a Cre/loxP reporter system was functionally evaluated. Cre-mediated genetic recombination was induced in CD4+ T cells *in vivo.* Interestingly, the signal from nontargeted mRNA-LNP in both splenic and lymphatic tissues at high dose (30 µg per mouse) was not zero, as observed in untreated mice (28% ZsGreen1+ cells among CD3+CD8- cells). This observation is likely due to expression of an ApoE receptor by some T cells (Sundqvist, 2018, Front. Immunol, 9:974; Panezai et al., 2017, Immunology 152:308-327), as LNP bind ApoE and typically target the liver 31. The percentage of gene edited cells was further increased by multiple injections of anti-CD4/mRNA-LNP. This high level of *in vivo* T cell targeted genetic recombination has not been reported elsewhere. An important finding for potential gene editing therapies was that similar levels of gene recombination were observed in resting and activated CD4+ T cells. Evaluation of the presence of successfully targeted T cells over time showed a gradual decrease of ZsGreen1 signal in spleen during one-week post-treatment with anti-CD4/mRNA-LNP. This trend was expected considering the lifespan of circulatory T cells, which are a predominant population of T cells in spleen (Langeveld et al., 2006, Journal of Clinical Investigation, 36:250-256: Bronte et al., 2013, Immunity, 39:806-818). However, ZsGreen1 expression in lymph nodes remained minimally changed over the 7-day experiment time after treatment with anti- CD4/mRNA-LNP. In a report analyzing the migration of 51Cr-labeled thoracic duct lymphocytes (TDLs) in major lymphoid and non-lymphoid tissues of rats revealed that lymphocytes have longer residence time in the lymph nodes than the spleen 24. Comparable residence times were reported for mice as well (Mandl et al., 2012, Proc Natl Acad Sci U S A, 109: 18036-18041). Finally, whether various T cell subtypes differed in being targeted was evaluated. There was no significant difference in uptake and recombination among naive, memory, and effector memory subtypes when treated with anti-CD4/Cre mRNA-LNP. The anti-CD4 mRNA-LNP system allows for CD4+ T cell targeting in the tissues, such as spleen and lymph nodes, which is critical for T cell therapies. The current T cell targeting platform has great potential for many *in vivo* T cell manipulation-based applications by making T cell targeted therapeutic mRNA delivery possible. *In vivo* delivery to specific cell types (e.g. T lymphocytes, among others) is an intensely developing field, evidenced by many recent studies (Veiga et al., 2020, Adv Drug Deliv Rev, 159:364-376; Mizrahy et al., 2017, Mol Ther, 25:1491-1500; Fenton et al., 2017, Adv Mater, 29; Ramishetti et al., 2016, J Drug Target, 24:780-786: Veiga et al., 2018, Nat Commun, 9:4493). LNP modified with antibodies have been used for delivery of siRNA to lymphocytes for gene silencing purposes (Ramishetti et al., 2015, ACS Nano, 9:6706-6716). Ramishetti et al. surface modified siRNA-loaded LNP with anti- CD4 monoclonal antibodies for targeting CD4+ T lymphocytes. They observed gene silencing in approximately 30% of CD4+ T cells isolated from spleen, which is only half of the targeted functional activity that was observed with the CD4-targeted Cre mRNA-LNP (~60% of CD4+ T cells in spleen). It is of note that because of their use of siRNA-LNP and the non-binary readout of their experiments, direct comparison of targeting efficiencies of the two platforms is not straightforward. Other attempts have been made for lymphocyte targeting with other lipid- and polymer-based carriers. McKinlay et al. (McKinlay et al., 2018, Proceedings of the National Academy of Sciences 115, E5859-E5866) reported on a combinatorial chemical approach of mRNA delivery using hybrid lipid-based amphiphilic charge-altering releasable transporters (CARTs), achieving approximately 1.5% T lymphocyte transfection efficiency in mice. Similarly, Fenton et al. (Fenton et al., 2017, Advanced Materials, 29:1606944) described specific LNP design for delivering mRNA to B lymphocytes without using active targeting ligand. They showed an enhanced luminescence signal from the B cell targeted-Luc mRNA-LNP formulation in spleen compared to other non-selective formulations of their LNP formulation library. Veiga et al. (Veiga et al., 2018, Nature communications, 9:4493-4493) delivered mRNA in surface modified LNP to inflammatory Ly6C+ leukocytes using their ASSET platform, which also employs monoclonal antibody targeting. Delivery and expression of mRNA encoding IL-10 showed significant therapeutic effect in a colitis model. While some T cells also express Ly6c, as do monocytes, macrophages and neutrophils, it was not determined which populations of leukocytes take up and express Ly6c-targeted LNP, and to what extent. The targeted mRNA-LNP platform is the first report of an LNP-based mRNA delivery system for selective and functional CD4+ targeting. Overall, the T cell-targeted mRNA-LNP platform presented here offers tremendous opportunity for a wide range of *in vivo* T cell manipulations. The great potential of this system to reach all T cell subtypes in difficult-to-access-tissues such as lymph nodes, will make the targeting platform available for many types of T cell manipulation *in vivo.* The application potentials include delivering mRNA therapeutics to T cells for potential HIV cure. In particular, targeted delivery of engineered genomic editing enzymes have the potential to cure HIV, by excising the HIV integrated provirus from the genome of infected cells (Karpinski et al., 2016, Nat Biotechnol, 34:401-409). Additionally, targeted modification of lymphocytes have numerous applications for development of fast-acting and cost-effective immunotherapeutics for a range of cancers, infectious diseases, and immunological disorders.

The materials and methods used for the experiments are now described.

### Mice

C57BL/6J mice. Equal numbers of male and female C57BL/6J mice were purchased from Jackson laboratories. Ai6(RCL-ZsGreen) mice. Ai6 (RCL-ZsGreen) mice on C57BL/6J background were purchased from Jackson Laboratory (stock no: 007906) and bred homozygous in-house. Ai6 is a Cre reporter allele with a loxP-flanked STOP cassette preventing transcription of a CAG promoterdriven enhanced green fluorescent protein variant (ZsGreen1) - all inserted into the Gt(ROSA)26Sor locus. Upon Cre-mediated recombination, Ai6 mice express robust ZsGreen1 fluorescence.

### mRNA production and LNP preparation

Coding sequences of Cre recombinase or firefly luciferase were codon-optimized, synthesized and cloned into the mRNA production plasmid (pUC-ccTEV-Cre-A101 and pUC-ccTEV-Luc2-A101, respectively). mRNAs were produced using T7 RNA polymerase (Megascript, Ambion) on linearized plasmids. mRNAs were transcribed to contain 101 nucleotide-long poly(A) tails. m1Ψ-5'-triphosphate (TriLink) instead of UTP was used to generate modified nucleoside-containing mRNA. Capping of the *in vitro* transcribed mRNAs was performed co-transcriptionally using the trinucleotide cap1 analog, CleanCap (TriLink). mRNA was purified by cellulose purification, as described (Baiersdorfer et al., 2019, Mol Ther Nucleic Acids, 15:26-35). All mRNAs were analyzed by native agarose gel electrophoresis and were stored frozen at -20°C.

m1ψ-containing mRNAs were encapsulated in LNP using a self-assembly process in which an aqueous solution of mRNA at pH = 4.0 is rapidly mixed with a solution of lipids dissolved in ethanol (Maier et al., 2013, Mol Ther, 21:1570-1578). LNP used in this study were similar in composition to those described previously (Maier et al., 2013, Mol Ther, 21:1570-1578: Jayaraman et al., 2012, Angew Chem Int Ed Engl, 51:8529-8533), which contain an ionizable cationic lipid (Acuitas)/ phosphatidylcholine/cholesterol/PEG-lipid (50:10:38.5:1.5 mol/mol) and were encapsulated at an RNA to total lipid ratio of ~0.04 (wt/wt). The diameter of the nanoparticles was ~80 nm as measured by dynamic light scattering using a Zetasizer Nano ZS (Malvern Instruments Ltd., Malvern, UK) instrument. mRNA-LNP formulations were stored at -80 °C at a concentration of mRNA of ~1 µg/µL.

### Monoclonal antibody-conjugated lipid nanoparticles

LNP were conjugated with mAbs specific for CD4. Purified NA/LE Rat anti-mouse CD4 (BD PharmingenTM), purified rat anti-human CD4 antibody, clone A161A1 (BioLegend), and control isotype-matched IgG were coupled to LNP via SATA-maleimide conjugation chemistry, as described earlier 18. Briefly, LNP were modified with DSPE-PEG-maleimide by a post-insertion technique. The antibody was modified with SATA (N-succinimidyl S-acetylthioacetate) (Sigma-Aldrich) to introduce sulfhydryl groups allowing conjugation to maleimide. SATA was deprotected using 0.5 M hydroxylamine followed by removal of the unreacted components by G-25 Sephadex Quick Spin Protein columns (Roche Applied Science, Indianapolis, IN). The reactive sulfhydryl group on the antibody was then conjugated to maleimide moieties using thioether conjugation chemistry. Purification was performed using Sepharose CL-4B gel filtration columns (Sigma-Aldrich). mRNA content was calculated by performing a modified Quant-iT RiboGreen RNA assay (Invitrogen). Size and surface charge of the targeted lipid nanoparticles were determined using dynamic light scattering (DLS) and laser doppler velocimetry (LDV), respectively on a Malvern Zetasizer Nano ZS (Malvern Instruments, Worcestershire, UK). Both size and zeta potential measurements were carried out in PBS pH 7.4 at 25°C in relevant disposable capillary cells. A non-invasive back scatter system (NIBS) with a scattering angle of 173° was used for size measurements. Diameters of unconjugated and antibody-modified mRNA-LNP were interpreted as normalized intensity size distribution as well as z-average values for particle preparations.

### In vitro cell binding studies

For cell binding studies using radioactivity measurements, LNP were first radiolabeled with Na125I using Iodination Beads (Pierce) as described earlier (Khoshnejad et al., 2016, Bioconjug Chem, 27:628-637). Human CD4+ T cells were then incubated with increasing quantities of either Anti-CD4/ or Control IgG/mRNA-LNP for one hour at room temperature. Incubation medium was then removed and cells were washed with PBS buffer three times to remove the unbound nanoparticles from the cell surface. The cells were lysed with 1% Triton X100 in 1 N NaOH and the cell-associated radioactivity was measured by a Wallac 1470 Wizard gamma counter (Gaithersburg, MD) and compared to total added activity.

For cell binding studies using flow cytometry, human CD4+ T cells were seeded at 150,000 cells per well in 24-well plates. LNP carrying Poly(C) RNA were added to the media at increasing quantities of mRNA per well, and cells were incubated for one hour at room temperature. Incubation medium was then removed and cells were washed with PBS buffer three times to remove the unbound nanoparticles from the cell surface. FITC-tagged anti-rat IgG (Abcam, Cambridge, UK) was used to monitor binding of antibody-conjugated LNP on a BD LSR II flow cytometer.

### In vitro cell transfection studies

For cell transfection studies using firefly luciferase mRNA, human CD4+ T cells were plated in 48-well plates. After 18 hours, LNP carrying reporter luciferase mRNA were added at increasing concentrations to the cells, and incubated for 1.5 hours. Plates were then washed three times with PBS and complete medium was added to the cells. After culturing for 24 hours in complete media, cells were washed with PBS, lysed in luciferase cell culture lysis reagent (Promega, Madison, WI) and the luciferase enzymatic activity as luminescence (Luciferase assay system, Promega) was measured 18. Transfections were performed in triplicate. For cell transfection studies using Cre recombinase mRNA, spleens from two Ai6 mice were harvested, and a pooled single cell suspension was produced. 2 million splenocytes were then plated in each well of 6-well plates. Cells were incubated with 1, 3, 6 or 9 µg of CD4-targeted or non-targeted (unconjugated or control IgG-conjugated) Cre mRNA-LNP overnight. Cells were then collected and stained with Live/Dead Aqua (Thermo Fisher Sci, L34966) and antibodies against CD3 and CD8 (and CD4, which was omitted from later experiments), and the percentage of ZsGreen1-expressing CD3+CD8- cells was determined using flow cytometry.

### Biodistribution of anti-CD4/mRNA-LNP in C57BL/6J mice; tissue uptake

¹²⁵I-radiolabeled mRNA-LNP were administered by IV (retro-orbital) injection into C57BL/6 mice (The Jackson Laboratory, Bar Harbor, ME). Blood was collected at 0.5, 1, and 24 hours post-injection from the inferior vena cava. Specific organs (liver, spleen, lung, kidney and heart) were also harvested at the same time-points, rinsed with saline, blotted dry, and weighed. The amount of radioactivity in each organ as well as in 100-µL samples of blood was measured in a gamma counter (Wallac 1470 Wizard gamma counter, Gaithersburg, MD). Tissue uptake as percent of injected dose per gram tissue (%ID/g), and localization ratio (LR) as organ-to-blood ratio were calculated using radioactivity values and weight of the samples. Immunospecificity index (ISI) was also calculated as the ratio of the LR of CD4-targeted mRNA-LNP to that of Control IgG-modified ones.

### Biodistribution of anti-CD4/mRNA-LNP in C57BL/6J mice: luciferase mRNA translation at tissue and cellular level

C57BL/6J mice were IV (retro-orbital) injected with anti-CD4/mRNA-LNP or control IgG/mRNA-LNP formulations. At 5 hours after injection, animals were euthanized and selected organs (liver, spleen, lung, kidney and heart) were harvested, rinsed with PBS, and stored at -80 °C until analysis. When thawed, tissue samples were homogenized in appropriate volumes of cell lysis buffer (1X) (Promega Corp, Madison, WI) containing protease inhibitor cocktail (1X) and mixed gently at 4 °C for one hour. The homogenates were then subjected to cycles of freeze/thaw in dry ice/37 °C and centrifuged for 10 minutes at 16,000 g at 4 °C. Luciferase activity was then measured in the supernatant using a Victor 3 1420 Multilabel Plate Counter (Perkin Elmer, Wellesley, MA). Further, the mRNA expression in the CD3+ cell population was evaluated. CD3+ cells were isolated from the spleens or lymph nodes of injected mice using the MagniSort^{™} Mouse CD3+ Selection Kit (ThermoFisher Scientific, Waltham, MA) based on manufacturer's instructions. Briefly, a biotinylated Anti-Mouse CD3 antibody and streptavidincoated magnetic beads were utilized for CD3+ cell isolation. CD3+ cells were bound to the antibody and then magnetic beads. When placed in a magnetic field, the undesired cells were separated from CD3+ cells by decanting. Luciferase activity measurements were performed on the cell lysate of the CD3+-enriched cell population.

### Bioluminescence imaging

C57BL/6J mice were IV (retro-orbital) injected with anti- CD4/mRNA-LNP or control IgG/mRNA-LNP formulations. At 5 hours after injection, bioluminescence imaging was carried out as described previously 18 using an IVIS Spectrum imaging system (Caliper Life Sciences, Waltham, MA). D-luciferin was administered to mice intraperitoneally at a dose of 150 mg/kg. After 5 minutes, the mice were euthanized; desired tissues were harvested, and immediately placed on the imaging platform. Tissue luminescence was measured on the IVIS imaging system using an exposure time of 5 seconds or longer to ensure that the signal obtained was within operative detection range. Bioluminescence values were also quantified by measuring photon flux (photons/second) in the region of interest using LivingImage software provided by Caliper.

### Determination of targeting efficiency of anti-CD4/mRNA-LNP using a Cre/loxP reporter system

To analyze delivery efficiency to targeted cell populations within the spleen and lymph nodes, mRNA translation was tracked with single-cell resolution. The targeted and nontargeted LNP containing Cre recombinase mRNA were IV (retro-orbital) injected into Ai6 mice carrying a Cre reporter allele with a loxP-flanked STOP cassette preventing transcription of a green fluorescent protein variant (ZsGreen1). Cre recombinase excises the loxP-flanked STOP cassette, therefore allowing the transcription of ZsGreen1. At desired time points after injection, animals were euthanized and spleens and lymph nodes were harvested. The number of CD3⁺CD8⁻ cells emitting green fluorescent signal in organ single cell suspensions was evaluated using flow cytometry.

### Single cell suspension preparation and flow cytometry

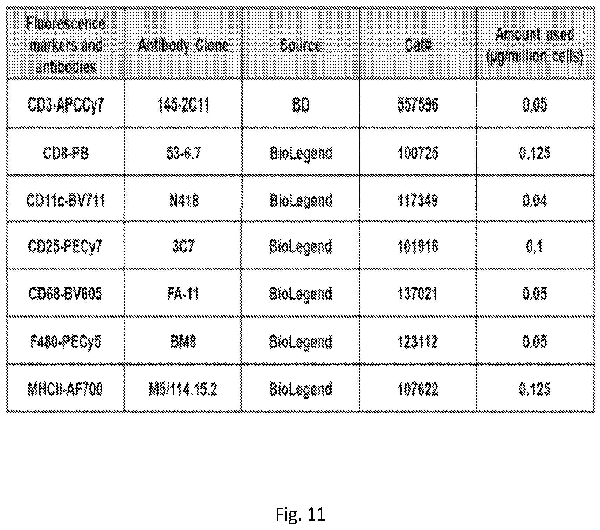

Single cell suspensions were prepared from spleens and lymph nodes. Briefly, the tissues were crushed using the frosted end of glass microscope slides and then passed through a 70-µm filter. Following centrifugation and removal of supernatant, cells were resuspended in RPMI + 10% FBS medium. and were first stained with Live/Dead Aqua cell stain (Thermo Fisher Sci., Cat# L34957), then a mixture of anti-mouse antibodies (Figure 11). The stained single cell populations were characterized on a BD LSR II flow cytometer (BD Biosciences). 500,000 events were collected per sample. Compensation of multicolor flow was carried out using ArC^{™} Amine Reactive beads (Thermo Fisher Sci.) for Live/Dead Aqua, Compbead anti Rat and anti-Hamster Ig κ/Negative Control Compensation Particles set (BD Biosciences) for all antibodies, and GFP BrightComp eBeads (Thermo Fisher Sci., Cat# A10514) for ZsGreen1. Data were analyzed with FlowJo software (Ashland, OR). The materials and methods used in these experiments are now described.

The results of the experiments are now described.

### mRNA-LNP targeting CD4+ T cells in vitro

Considering that T cells do not naturally endocytose nanoparticles, initially CD4+ T cell surface antigens were sought that endocytose after mAb binding. CD4-targeted receptor mediated endocytosis was selected to achieve CD4+ T cell targeted delivery 19. CD4 receptor targeting has also been shown to be capable of uptake and internalization upon nanoparticle binding 20, 21. The binding capacity of the targeted mRNA-LNP was first evaluated on human CD4+ T cells obtained from healthy donors. Anti-CD4 IgG antibody (anti-CD4/mRNA-LNP) or non-specific isotype control IgG (control IgG/mRNA-LNP) was conjugated to mRNA-LNP. As shown in Figure 1A, radiolabeled anti-CD4/mRNA-LNP selectively bound to human CD4+ T cells, while control IgG counterparts did not. Selective targeting to CD4+ T cells was also confirmed using flow cytometry (Figure 1B - Figure 1C). Human CD4+ T cells were incubated with either anti-CD4/Poly(C) RNA-LNP or control IgG/Poly(C) RNA-LNP, and FITC-tagged anti-rat IgG was used to monitor binding of antibody-conjugated LNP to cells. Dose-responsive binding of anti-CD4/Poly(C) RNA-LNP was observed. In order to determine internalization and functional activity (mRNA translation) of the targeted mRNA-LNP, anti-CD4 antibody- or control IgG-conjugated LNP carrying firefly luciferase (Luc)-encoding mRNA were incubated with human CD4+ T cells. Efficient translation of the mRNA in anti-CD4/mRNA-LNP was demonstrated compared to control IgG/mRNA-LNP. Incubation of CD4+ T cells with higher doses of Luc mRNA-LNP yielded higher Luc activity, demonstrating a dose-response correlation (Figure 1C).

In order to directly assess targeting efficiency on the single cell level, and to test the targeting platform for gene editing purposes, splenocytes were harvested from mice harboring Ai6 (a Cre reporter allele with a loxP-flanked STOP cassette, which upon Cre-mediated recombination, expresses robust ZsGreen1 fluorescence), and treated with different amounts of targeted or non-targeted (unconjugated or control IgG-conjugated) Cre mRNA-LNP. The cells were then collected and stained with antibodies against CD3 and CD8 (antibodies are listed in Figure 11) and analyzed by flow cytometry. Gating strategy to identify ZsGreen1 positive cells among CD3+CD8- population is presented on Figure 2B., with the corresponding ZsGreen1 positive cells shown on Figure 2A. CD3+CD8- staining was used instead of direct CD4 staining to identify CD4+ T cells because of the transient disappearance of CD4 upon administration of the anti-CD4 antibody-conjugated nanoparticles (Figure 3). A very low percentage of CD3+CD8-cells exhibited positive ZsGreen1 signal when non-targeted LNP were used, while approximately 80% of this cell population took up and translated Cre mRNA delivered in anti-CD4 antibody-conjugated LNP, even at the lowest amount of mRNA-LNP administered. The same experiment was performed in splenocytes harvested from Ai9 mice, which express robust tdTomato fluorescence following Cre-mediated recombination, and similar results were obtained. This shows the potential of the targeted anti-CD4/mRNA-LNP to efficiently transfect CD4+ T cells *in vitro.*

### Anti-CD4/mRNA-LNP target CD4+ T cells in vivo

Next, the biodistribution of anti-CD4/mRNA-LNP in mice was analyzed after retro-orbital intravenous (IV) administration. LNP were directly labeled with ¹²⁵I prior to conjugation with anti-mouse CD4 or control IgG, therefore, measured radioactivity only showed distribution of particles without any detached targeting antibodies affecting the outcome. To measure tissue uptake, the amount of radioactivity in various tissues (percent of injected dose per gram of tissue-%ID/g) was calculated. As expected, a substantial amount of control IgG/mRNA-LNP particles were still circulating in the blood (19.35±2.2 %ID/g) 0.5 hour (h) post injection, representing a significant change in the biodistribution with a reduction in liver targeting of control IgG LNP (Figure 4A). For anti- CD4/mRNA-LNP, lower amounts of particles were circulating (10.84±0.42 %ID/g). The majority of the anti-CD4/mRNA-LNP uptake occurred in the spleen (131.59±9.71 % ID/g), representing a 3.5-fold increase in splenic uptake compared to the control IgG/mRNA-LNP (37.6±8.67 %ID/g). The localization ratio (LR), defined as the ratio of %ID/g of a given organ to that in the blood, was also calculated for both CD4-targeted and control IgG/mRNA-LNP. The spleen being part of the reticuloendothelial system contributes to non-specific splenic uptake that is observed with the untargeted mRNA-LNP. Anti-CD4/mRNA-LNP were localized in spleen at 6-fold higher level than their control IgG counterparts (Figure 4B, Figure 5A). To further explore and quantitate the kinetics of *in vivo* tissue uptake of anti-CD4/mRNALNP, targeted and non-targeted 125I-labeled poly(C) RNA-LNP were injected IV into mice. Groups of animals were sacrificed at 0.5, 1, and 24 hours after injection, and selected tissues (blood, spleen, liver, lung, and kidneys) were harvested. The highest circulating amount of targeted mRNA-LNP was 10.84±0.42 %ID/g in blood at the earliest time point tested (Figure 4C). At later time points, the concentration of targeted particles in blood quickly dropped to a %ID/g of 6.86±1.34 and 0.51±0.07 at 1 and 24 hours, respectively. Specific splenic uptake of targeted particles peaked at 0.5 h post injection (131.59±9.71 % ID/g) (Figure 4C) and the localization ratio increased over time reaching to 61.15 at the last time point tested, 24 hours (Figure 4D).

### mRNA in targeted LNP is efficiently delivered to CD4+ T cells in vivo

mRNA translation after IV administration of Luc mRNA-LNP was then analyzed. Control IgG/Luc mRNA-LNP and anti-CD4/Luc mRNA-LNP were first administered at a dose of 8 µg (0.32 mg/kg) mRNA. Five hours after injection, various organs were harvested, and luciferase activity was either measured from tissue lysates, or was detected by direct luminescent imaging of whole organs (Figure 6A-Figure 6C). The Luc expression pattern showed a marked difference between anti-CD4 and control IgG/Luc mRNA-LNP-treated mice, as the luminescence signal decreased significantly in liver with CD4- targeting. Most importantly, Luc activity for anti-CD4/Luc-mRNA-LNP was ~7 fold higher compared to the control IgG-modified mRNA-LNP in the spleen (Figure 6A and Figure 6B). After removal of the spleen, kidneys, lungs, heart, and liver - which exhibits high uptake of both unconjugated and antibody-conjugated LNP - lymph node luciferase expression was observed in the anti- CD4/Luc mRNA-LNP-treated mice (Figure 6C). This shows the capacity of targeted LNP to traverse endothelial membranes and functionally access cells in tissues, such as lymph nodes. To demonstrate that mRNA was delivered specifically to the T cell population, CD3+ T cells were isolated (as CD4 selection could not be performed) from the spleen of mice treated as above. Luc activity of the CD3+ population after anti-CD4/Luc mRNA-LNP administration was 33-fold higher than in control IgG/Luc mRNA-LNP-treated samples. It was concluded that with Luc activity concentrated in T cells (Figure 6D), CD4+ T cells are being specifically and efficiently targeted after IV delivery of targeted nanoparticles.

To confirm the targeting efficiency of the CD4-targeted mRNALNP platform with LNP formulations other than ALC-0307 LNPs, the same antibody conjugation strategy was applied on the Acuitas LNP formulation containing the ionizable lipid 0315 (ALC-0315 LNP), which is the LNP formulation in the recently US Food and Drug Administration (FDA)-approved Pfizer/BioNTech COVID vaccine (Thran et al., 2017, EMBO Mol. Med. 9, 1434-1447; WO2017075531A1). The list of ingredients in this LNP formulation includes (4-hydroxybutyl)azanediyl)bis(hexane-6,1-diyl)bis(2-hexyldecanoate) or ALC-0315 ionizable lipid, 2[(polyethylene glycol-2000]-N,N-ditetradecylacetamide, 1,2-distearoyl-sn-glycero-3-phosphocholine and cholesterol. Five hours after injection of anti-CD4-targeted Luc mRNA-ALC-0315 LNPs, a very similar Luc expression pattern to CD4-targeted ALC-0307 LNPs (i.e., a higher luminescence signal in the spleen and a lower signal in liver) was observed when compared to control IgG counterparts. These data prove that similar targeting efficiency is achieved with CD4 targeting of other LNP formulations, such as ALC-0315 LNP.

### CD4-targeted Cre-mRNA-LNP effect genetic recombination in CD4+ T cells in vivo

One use for targeted mRNA therapy is gene editing and insertion. To evaluate the efficiency of delivery of mRNA using CD4-targeted LNP at the cellular level for *in vivo* genetic modification, Cre mRNA-LNP was administered to Ai6 mice (Figure 7A). In these mice, the Cre/loxPmediated expression of a reporter gene encoding the fluorescent protein ZsGreen1 allows for easy readout of successfully transfected and LoxP recombined target cells using flow cytometry (Figure 7B and Figure 7C). A wide range of doses (3, 10, 30, and 90 µg) were tested. Mice were injected IV, then spleens and lymph nodes were harvested the next day, and single cell suspensions were prepared from each tissue. Cells were stained for flow cytometry using antibodies against CD3 and CD8 to identify CD4+ T cells (Figure 11). No signal was observed in non-treated animals, indicating no leakage of the reporter construct. Administration of control IgG/Cre mRNA-LNP led to low efficiency of transfection, similar in level over the range of mRNA doses used, in both tissues tested, i.e. spleens (Figure 7B) and lymph nodes (Figure 7C). As expected, a significant increase in the number of ZsGreen1-expressing cells was observed with anti-CD4/Cre mRNALNP treatment at all tested mRNA doses when compared to control IgG- and unconjugated mRNA-LNP counterparts (Figure 7B and Figure 7C). In mice treated with unconjugated mRNA-LNP, a substantial increase in mRNA delivery and subsequent Cre/loxP recombination was observed (up to approximately 20% of ZsGreen1+ cells in the CD3+CD8- cell population) when the dose was increased to 30 µg. This is still well below the strong response observed with targeted mRNA-LNP at all tested doses, and is likely due to expression of an ApoE receptor by some T cells (Sundqvist, 2018, Front. Immunol, 9:974; Panezai et al., 2017, Immunology 152, 308-327). While administration of 90 µg of anti-CD4/Cre mRNA-LNP resulted in an even higher percentage of ZsGreen1+ CD4+ T cells, this amount of LNP proved to be toxic in all groups (both unconjugated and control IgG-conjugated, and CD4-targeted LNP treatments), thus that dose was eliminated from further experiments. Selective CD4 targeting versus control of untargeted LNP did not increase the uptake of nanoparticles in macrophages and dendritic cells (Figure 8), likely due to their extensive natural phagocytic uptake of nanoparticles, whereas with CD4+ T cells, there is significant increase in targeted mRNA-LNP uptake compared to untargeted control mRNA-LNP. The number of ZsGreen1-expressing cells in non-T cell splenocytes, such as dendritic cells and macrophages, did not differ among the range of doses in this study (Figure 8).

A similar experiment was performed using CD4-targeted ALC-0315 LNPs. When Ai6 mice were i.v. injected with these targeted LNPs carrying Cre mRNA, targeting efficiency comparable to CD4-targeted ALC-0307 LNP-Cre mRNA was observed (increase in the number of ZsGreen1-expressing cells in mice treated with anti-CD4/ALC-0315 LNP-Cre mRNA treatment compared to control IgG counterparts).

### CD4+ T cell-targeting with anti-CD4/mRNA-LNP is not T cell subtype specific

Next, whether the uptake of the targeted LNP were favored by certain T cell subtypes was investigated. One day after the administration of a dose 10 µg of Cre mRNA-LNP, spleens were harvested, and single cell suspensions were stained with antibodies against CD3, CD8, CD44 and CD62L to identify naive, memory, and effector memory T cell subpopulations. No significant preference was found for the CD4-targeted mRNA-LNP to be taken up and expressed by any specific CD4+ T cell subpopulation examined: CD4+ naive T cells (CD44-CD62L-), central memory T cells (CD44+CD62L+), and effector memory T cells (CD44+CD62L-) (Figure 9A). The majority of T cells *in vivo* are not activated. The expression of the T cell activation marker (CD25) was analyzed on the CD4+ T cells receiving Cre mRNA-LNP. Notably, CD4-targeted mRNA-LNP induced Cre recombination in ~57% of resting (CD25-), compared to ~40% of activated (CD25+) CD4+ T cells (Figure 9B), thus demonstrating efficient targeting, transfection, and gene recombination in resting CD4+ T cells.

### Recombined cells decrease over time after CD4-targeted delivery in the spleen

ZsGreen1 expression was tracked for seven days after a single IV administration of 10 µg of Cre mRNALNP. Spleens and lymph nodes were harvested one, four or seven days post-injection, and single cell preparations were stained for flow cytometric analysis as above. Four days after administration of 10 µg of anti-CD4/Cre mRNA-LNP, the number of ZsGreen1-expressing splenic CD4+ T cells dropped significantly (from ~ 50% at day 1 to ~ 32% at day 4). However, it held at a similar level of around 26% at the last time point tested (day 7), still significantly above the values observed with IgG and unconjugated counterparts (Figure 7D). Blood and spleen are sites of transient-recirculating T cells, which this data reflects (Ganusov et al., 2014, PLoS Comput Biol, 10:e1003586; Mandl et al. 2012, Proc Natl Acad Sci U S A, 109:18036-18041). The ZsGreen1 expression for all treatments did not significantly change over 7 days in T cells extracted from lymph nodes (Figure 7E). This reflects the longer residence time of T cells in lymph nodes (Ganusov et al., 2014, PLoS Comput Biol, 10:e1003586; Mandl et al. 2012, Proc Natl Acad Sci U S A, 109:18036-18041).

### In vivo targeted mRNA-LNP-induced specific genetic recombination shows an additive effect

The potential additive effect of targeted mRNA delivery was tested by serial administrations of mRNA-LNP (Figure 10A and Figure 10B). Mice received three or five IV injections of 10 µg doses of Cre mRNA-LNP, one injection every 24 hours. Spleens and lymph nodes were harvested the day after the last injection. Five injections resulted in a significantly higher number of ZsGreen1+ cells when compared to three injections. Interestingly, a steady increase in ZsGreen1- expressing CD4+ T cell numbers was observed for both control IgG/ and unconjugated mRNALNP. However, the expression increased to 28% in the unconjugated group, still relatively lower than the anti-CD4/mRNA-LNP at any number of injections tested. Overall, the sequential administrations of the targeted mRNA-LNP resulted in increasing Cre-induced genetic recombination with increased number of injections in both the spleen and lymph nodes.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

### CLAUSES

Aspects and features of the present disclosure are set out in the following numbered clauses which contain the subject matter of the claims of the parent application as filed:
1. A composition comprising a delivery vehicle conjugated to a targeting domain, wherein the delivery vehicle comprises at least one agent, and wherein the targeting domain specifically binds to a cell surface antigen of a CD4⁺ T cell.
2. The composition of clause 1, wherein the delivery vehicle is selected from the group consisting of a liposome, a lipid nanoparticle, and a micelle.
3. The composition of clause 1, wherein the delivery vehicle is a lipid nanoparticle.
4. The composition of clause 3, wherein the lipid nanoparticle comprises a PEG-lipid conjugated to the targeting domain.
5. The composition of clause 3, wherein the at least one agent is encapsulated in the lipid nanoparticle.
6. The composition of clause 1, wherein the at least one agent is selected from the group consisting of a therapeutic agent, an imaging agent, diagnostic agent, a contrast agent, a labeling agent, a detection agent, and a disinfectant.
7. The composition of clause 1, wherein the at least one agent is a therapeutic agent.
8. The composition of clause 7, wherein the therapeutic agent comprises a nucleic acid molecule.
9. The composition of clause 7, wherein the therapeutic agent comprises a nucleoside modified nucleic acid molecule.
10. The composition of clause 8, wherein the nucleic acid molecule comprises an mRNA molecule.
11. The composition of clause 1, wherein the targeting domain is selected from the group consisting of a nucleic acid molecule, a peptide, an antibody, and a small molecule.
12. The composition of clause 1, wherein the targeting domain is an antibody.
13. The composition of clause 1, wherein the targeting domain is an anti-CD4 antibody.
14. A method of treating or preventing a disease or disorder in a subject in need thereof, the method comprising administering to the subject the composition of clause 7.
15. The method of clause 14, wherein the disease or disorder is selected from the group consisting of cancer, an infectious disease, and an immunological disorder.

## Claims

1. A composition comprising a delivery vehicle conjugated to a targeting domain, wherein the delivery vehicle comprises at least one agent, and wherein the targeting domain specifically binds to a cell surface antigen of a CD4⁺ T cell.

2. The composition of claim 1, wherein the delivery vehicle is selected from the group consisting of a liposome, a lipid nanoparticle, and a micelle.

3. The composition of claim 1, wherein the delivery vehicle is a lipid nanoparticle.

4. The composition of claim 3, wherein the lipid nanoparticle comprises a PEG-lipid conjugated to the targeting domain.

5. The composition of claim 3, wherein the at least one agent is encapsulated in the lipid nanoparticle.

6. The composition of claim 1, wherein the at least one agent is selected from the group consisting of a therapeutic agent, an imaging agent, diagnostic agent, a contrast agent, a labeling agent, a detection agent, and a disinfectant.

7. The composition of claim 1, wherein the at least one agent is a therapeutic agent.

8. The composition of claim 7, wherein the therapeutic agent comprises a nucleic acid molecule.

9. The composition of claim 7, wherein the therapeutic agent comprises a nucleoside modified nucleic acid molecule.

10. The composition of claim 8, wherein the nucleic acid molecule comprises an mRNA molecule.

11. The composition of claim 1, wherein the targeting domain is selected from the group consisting of a nucleic acid molecule, a peptide, an antibody, and a small molecule.

12. The composition of claim 1, wherein the targeting domain is an antibody.

13. The composition of claim 1, wherein the targeting domain is an anti-CD4 antibody.

14. The composition according to claim 7 for use in a method of treating or preventing a disease or disorder in a subject in need thereof.

15. The composition for use according to claim 14, wherein the disease or disorder is selected from the group consisting of cancer, an infectious disease, and an immunological disorder.
